(19) Europäisches Patentamt
European Patent Office
Office européen des brevets

(11) EP 1 386 915 A1

(12) **EUROPEAN PATENT APPLICATION**
published in accordance with Art. 158(3) EPC

(43) Date of publication:
**04.02.2004 Bulletin 2004/06**

(21) Application number: **02724713.9**

(22) Date of filing: **08.05.2002**

(51) Int Cl.[7]: **C07D 231/12**, C07D 233/64, C07D 249/08, C07D 401/06, C07D 401/12, C07D 401/14, C07D 403/12, C07D 417/12, A01N 43/653, A01N 43/824

(86) International application number:
**PCT/JP2002/004452**

(87) International publication number:
**WO 2002/090335 (14.11.2002 Gazette 2002/46)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **09.05.2001 JP 2001138507**

(71) Applicant: **Sumitomo Chemical Takeda Agro Company, Limited**
**Tokyo 103-0027 (JP)**

(72) Inventors:
- **ITOH, Shigeyuki**
  **Tsukuba-shi, Ibaraki 305-0821 (JP)**
- **NORITAKE, Chiemi**
  **Tsukuba-shi, Ibaraki 300-3257 (JP)**
- **IWATA, Atsushi**
  **311 Takedamatsushirorejidensu**
  **Tsukuba-shi, Ibaraki 305-0035 (JP)**

(74) Representative:
**von Kreisler, Alek, Dipl.-Chem. et al**
**Patentanwälte**
**von Kreisler-Selting-Werner**
**Postfach 10 22 41**
**50462 Köln (DE)**

(54) **AZOLE COMPOUNDS, PROCESS FOR PREPARATION OF THE SAME AND USE THEREOF**

(57) The invention provides novel azole compounds useful as pest controllers and novel pest controllers containing the compounds as the active ingredient. The invention discloses azole compounds represented by the following general formula, a production process for preparation of the compounds, and use thereof as insecticides or acaricides:

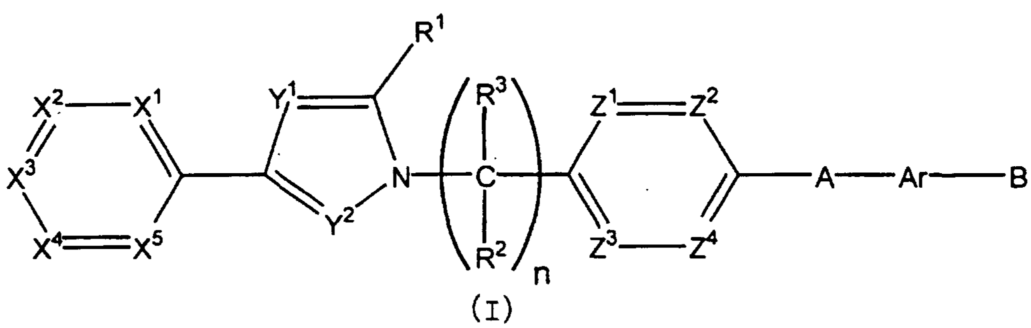

(I)

wherein $X^1$ to $X^5$ and $Z^1$ to $Z^4$ are each N, C-H, C-halogen, C-alkyl, or the like; $Y^1$ and $Y^2$ are each N or CH, with the proviso that both $Y^1$ and $Y^2$ must not be CH; A is a single bond, O, or the like; Ar is an optionally substituted aromatic residue; B is halogeno, alkyl, or the like; $R^1$ is H, halogeno, alkyl, or the like; $R^2$ and $R^3$ are each H, alkyl, or the like; m is 0 to 2; and n is 0 or 1.

EP 1 386 915 A1

## Description

Technical Field

The present invention relates to azole compounds useful as a pest controller, in particular, an insecticide or an acaricide, a process for producing the same and their use.

Background Art

Numerous azole compounds useful as an agricultural chemical have been developed. For example, JP-A 6-234751 (USP 5,482,951, EP 0572142B) discloses a triazole compound represented by the formula:

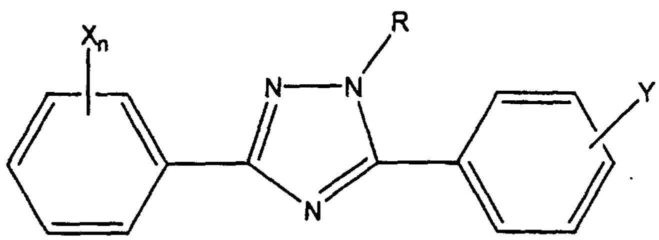

wherein R represents alkyl, X represents hydrogen, halogen etc.; n represents an integer of 1 to 5; Y represents an optionally substituted benzene ring or pyridine ring which binds via oxygen, sulfur, nitrogen, lower alkylene and the like, which is useful as an insecticide or an acaricide.

JP-A 8-92224 discloses a triazole compound represented by the formula:

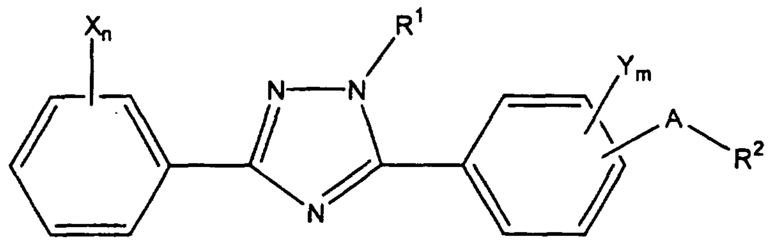

wherein $R^1$ represents alkyl, X represents halogen, alkyl, alkoxy, alkylthio, nitro, cyano or haloalkyl, Y represents halogen, nitro, cyano, alkyl, alkoxy, alkylthio or haloalkyl, n represents 0 to 5, and m represents 0 to 4, which is useful as an insecticide.

JP-A 8-283261 discloses a triazole compound represented by the formula:

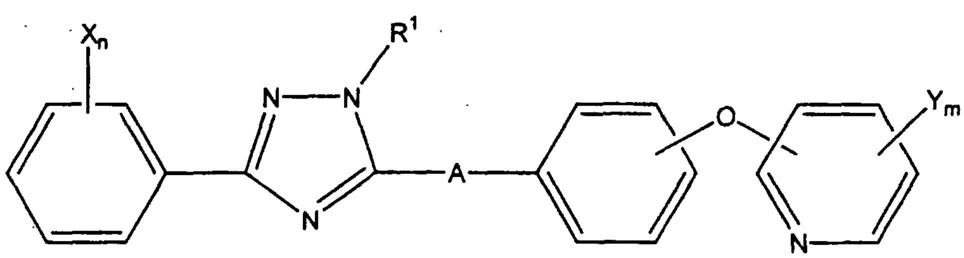

wherein $R^1$ represents alkyl, X represents halogen, Y represents halogen or haloalkyl, and A represents a group represented by the formula :

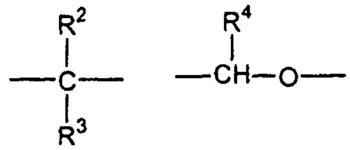

wherein $R^2$ and $R^3$ are the same or different, and represent hydrogen or alkyl, or may constitute a ring, $R^4$ represents hydrogen or alkyl, m represents 0 to 4, and n represents 0 to 5,
which is useful as an insecticide or an acaricide.

DE 3631511 discloses a triazole compound represented by the formula:

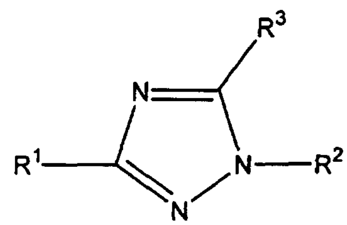

wherein $R^1$ and $R^2$ represent aryl which may be substituted with halogen, phenylalkoxy, phenyl, phenoxy, halophenoxy, arylN(alkyl)amino, pyridyloxy, haloalkylpyridyloxy, morpholino, piperidino, pyrrolidino, pyrrolyl etc., and $R^3$ represents halogen,
which is useful as an insecticide.

JP-A 8-245315 discloses a triazole compound represented by the formula:

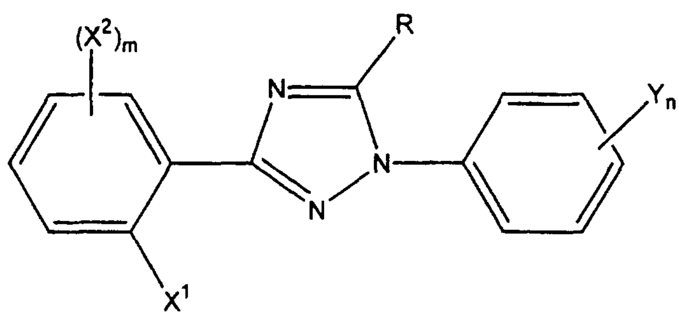

wherein $X^1$, $X^2$ and Y represent independently halogen, R represents $C_2$-$C_6$alkyl, $C_2$-$C_6$alkenyl, $C_3$-$C_6$cycloalkyl, $C_1$-$C_6$alkoxy$C_1$-$C_6$alkyl or $C_1$-$C_6$alkoxycarbonyl$C_1$-$C_6$alkyl, m represents 0 to 4, and n represents 0 to 5,
which is useful as an insecticide or an acaricide.

JP-A 62-19574 discloses a triazole compound represented by the formula:

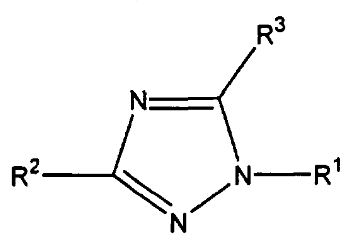

wherein $R^1$ represents phenyl which may be substituted with halogen, alkyl and the like, $R^2$ represents phenyl which may be substituted with halogen, alkyl and the like, and wherein at least one of substituents is at an ortho position,

and $R^3$ represents halogen, methyl or halomethyl, which is useful as a pest controller.

WO 00/24735 discloses a triazole compound represented by the formula:

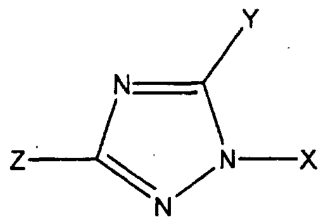

wherein Z represents pyridyl which is substituted with 1 to 4 substituents selected from the group consisting of Cl, F, methyl, halomethyl, methoxy, halomethoxy and methylthio, one of X and Y represents hydrogen, lower alkyl, haloalkyl, lower alkenyl, lower alkynyl, alkoxyalkyl, phenyl or substituted phenyl, the other represents a group represented by the formula:

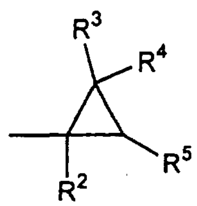 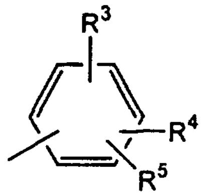 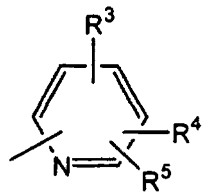 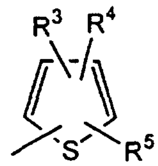 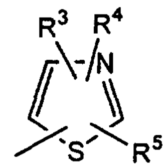

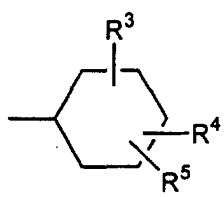 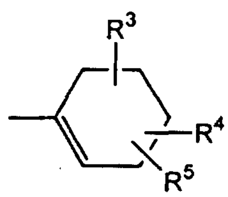 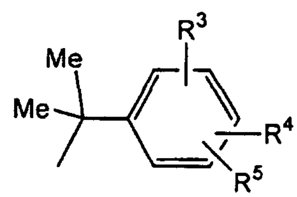

wherein $R^3$ represents pyridyl, pyridyloxy, substituted pyridyl, substituted pyridyloxy, phenoxy, substituted phenoxy, isoxazolyl, substituted isoxazolyl, isoxazolyl, substituted isoxazolyl, phenyl, substituted phenyl, $(CH_2)_nR^6$, $CH_2OR^6$, $CH_2SR^6$, $CH_2NR^6R^6$, $OCH_2R^6$, $SCH_2R^6$, $NR^6R^6CH_2$ and the like, $R^6$ represents hydrogen, lower alkyl, haloalkyl, lower alkenyl, lower alkynyl, phenyl or substituted phenyl, and n represents 1 or 2,
which is useful as an insecticide.

JP-A 11-171877 discloses an azole compound represented by the formula:

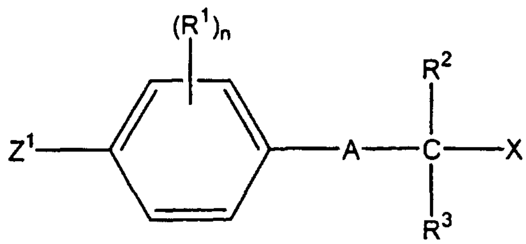

wherein $Z^1$ represents a heterocyclic group containing at least one hetero-atom selected from oxygen atom, sulfur atom and nitrogen atom and having the number of ring atom of 3 to 7, which may be fused with a benzene ring and may be substituted with alkyl, alkenyl or alkynyl each of which may be substituted with halogen or alkoxy, or optionally substituted phenyl; A represents oxygen or sulfur, $R^2$ and $R^3$ represent hydrogen or alkyl, and X represents a group represented by the formula:

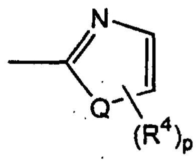 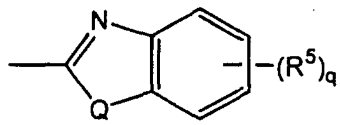

wherein Q represents oxygen or sulfur, which is useful as a herbicide.

JP-A 10-251255 discloses an azole compound represented by the formula:

$$Q^a\text{-}(CH_2)_n\text{-}X\text{-}Q^b\text{-}Q^c$$

wherein $Q^a$ represents a group represented by the formula:

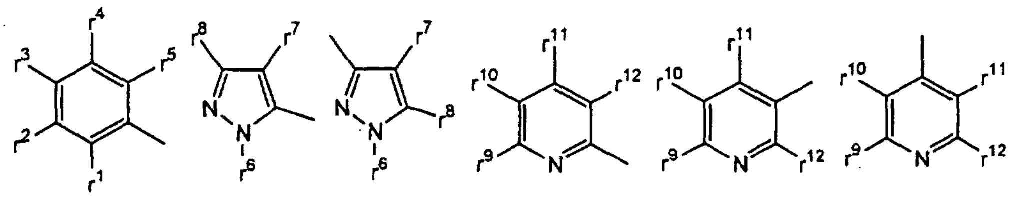

wherein $Q^b$ represents optionally substituted pyridinylene, pyrimidinylene, pyrazinylene, pyridazinylene, triazinylene or tetrazinylene, and $Q^c$ represents a group represented by the formula:

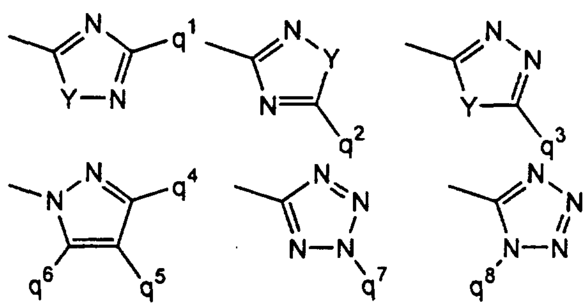

wherein $q^1$ to $q^6$ represent hydrogen, alkyl, haloalkyl, cycloalkyl, alkoxy, cyano, nitro, halogen, phenyl, benzyl, 3-pyridyl and the like, and X and Y represent oxygen, sulfur or N-hydrogen or alkyl, respectively, and n represents 0 to 2, which is useful as a herbicide.

Further, for example, JP-A 6-506953, JP-A 11-228410, Japanese Patent No. 3098772, WO 99/54314, WO 99/32454 and J. Chem. Soc. Perkin Trans. 1 207, 1976 disclose various azole compounds useful as a drug.

Disclosure of Invention

Object of the invention

An object of the present invention is to provide a novel pest controller, in particular, an insecticide or an acaricide containing an azole compound as an active ingredient.

Summary of the invention

The present inventors variously studied an azole compound useful as a pest controller, in particular, an agricultural chemical, succeeded in synthesizing a novel azole compound represented by the following formula (I) (hereinafter, referred to as Compound (I)), and found out that an azole compound represented by the following formula (XXV) (hereinafter, referred to as Compound (XXV)) which includes the compound of the formula (I) and which has not previously been used in utilities as an agricultural chemical, is useful as a pest controller, in particular, an agricultural chemical, inter alia, an insecticide and an acaricide, which resulted in completion of the present invention.

That is, the present invention provides:

1. A compound represented by the formula :

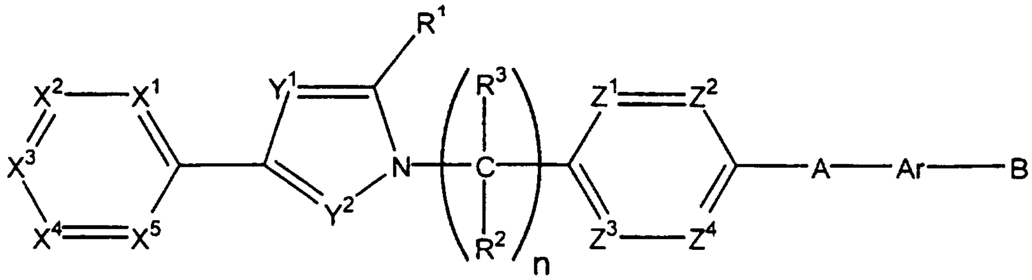

(I)

wherein $X^1$ represents N or C-$R^a$, $X^2$ represents N or C-$R^b$, $X^3$ represents N or C-$R^c$, $X^4$ represents N or C-$R^d$, $X^5$ represents N or C-$R^e$;

$Y^1$ and $Y^2$ are the same or different, and represent N or CH, respectively, provided that they are not CH at the same time;

$Z^1$ represents N or C-$R^f$, $Z^2$ represents N or C-$R^g$, $Z^3$ represents N or C-$R^h$, $Z^4$ represents N or C-$R^i$;

A represents a single bond, O, $CR^jR^kO$, $OCR^jR^k$, $SO_m$, $CR^jR^kSO_m$, $SO_mCR^jR^k$, $NR^l$, $CR^jR^kNR^l$ or $NR^lCR^jR^k$;

Ar represents an aromatic residue optionally having a substituent;

B represents a halogen, an optionally branched alkyl, a cycloalkyl, a haloalkyl, an alkenyl, a haloalkenyl, an alkoxy, a haloalkoxy, an alkyl$SO_m$, a haloalkyl$SO_m$, an amino optionally substituted with 1 or 2 substituents, an alkyl$SO_m$alkyl, a cyano, a nitro or an optionally substituted phenyl;

$R^1$ represents H, a halogen, an optionally substituted and optionally branched alkyl, an optionally substituted cycloalkyl, an optionally substituted alkenyl, an optionally substituted alkynyl, an optionally substituted alkoxy, an optionally substituted alkyl$SO_m$, an amino optionally substituted with 1 or 2 substituents, a cyano, a nitro, a formyl, a hydroxy, an optionally substituted acyl or an optionally substituted alkoxycarbonyl;

$R^2$ and $R^3$ are the same or different, and represent H or an alkyl, respectively, or may form a ring together with the carbon atom which they bind to;

$R^a$ and $R^e$ are the same or different, and represent H, a halogen, an optionally substituted and optionally branched alkyl, an optionally substituted cycloalkyl, an optionally substituted alkoxy, an optionally substituted alkyl$SO_m$ or amino optionally substituted with 1 or 2 substituents, respectively, provided that they are not H at the same time;

$R^b$ and $R^d$ are the same or different, and represent H, a halogen, an optionally substituted and optionally branched alkyl, an optionally substituted cycloalkyl, an optionally substituted alkenyl, an optionally substituted alkynyl, an optionally substituted alkoxy, an optionally substituted alkyl$SO_m$, an amino optionally substituted with 1 or 2 substituents, an optionally substituted acyl, an optionally substituted alkoxycarbonyl, a cyano, a nitro, a formyl or a hydroxy, respectively;

$R^c$ represents H, a halogen, an optionally substituted and optionally branched alkyl, an optionally substituted cycloalkyl, an optionally substituted alkenyl, an optionally substituted alkynyl, an optionally substituted alkoxy, an optionally substituted alkyl$SO_m$, an amino optionally substituted with 1 or 2 substituents, an optionally substituted acyl, an optionally substituted alkoxycarbonyl, a nitro, a formyl or a hydroxy;

$R^f$ and $R^h$ are the same or different, and represent H, a halogen, an optionally substituted and optionally branched alkyl, an optionally substituted cycloalkyl, an optionally substituted alkenyl, an optionally substituted alkynyl, an optionally substituted acyl, an optionally substituted alkoxycarbonyl, a cyano, a nitro, a formyl or a hydroxyl, respectively;

$R^g$ and $R^i$ are the same or different, and represent H, a halogen, an optionally substituted and optionally branched alkyl, an optionally substituted cycloalkyl, an optionally substituted alkenyl, an optionally substituted alkynyl, an optionally substituted alkoxy, an optionally substituted alkyl$SO_m$, an amino optionally substituted with 1 or 2 substituents, an optionally substituted acyl, an optionally substituted alkoxycarbonyl, a cyano, a nitro, a formyl or a hydroxyl, respectively;

$R^j$ and $R^k$ are the same or different, and represent H, a cyano or an alkyl, respectively, or may form a ring together with the carbon atom which they bind to;

$R^l$ represents H or an alkyl;

m represents 0, 1 or 2; n represents 0 or 1;

provided that when $Y^1$ and $Y^2$ are N at the same time, and n is 0, and one of $X^1$ to $X^5$ is N, and $R^1$ is a hydrogen, a lower alkyl, a lower alkenyl, a lower alkynyl, a haloalkyl, an alkoxyalkyl or an optionally substituted phenyl group, then Ar represents an aromatic heterocyclic residue optionally having a substituent,

when $Y^1$ and $Y^2$ are N at the same time, and n is 0, and $X^1$ to $X^5$ are not N, and A is O, S or $NR^l$, then $R^1$ represents a group other than halogen,

when $Y^1$ and $Y^2$ are N at the same time, and n is 0, and $X^1$ to $X^5$ are not N, and A is $OCR^jR^k$ or $SCR^jR^k$, then Ar represents an aromatic hydrocarbon residue optionally having a substituent or an aromatic heterocyclic residue other than an oxyazole ring and an thiazole ring which may be substituted and may be fused with other ring,

when $Y^1$ and $Y^2$ are N at the same time and n is 1, then A represents O, $CR^jR^kO$, $OCR^jR^k$, and $R^1$ represents a group other than $alkylSO_m$,

when $Y^1$ and $Y^2$ are N at the same time, n is 0, and A is a single bond, then $X^1$ to $X^5$ are not N,

or a salt thereof;

2. The compound according to the above-mentioned 1,

wherein 0 to 2 of $X^1$ to $X^5$ are N,

0 to 3 of $Z^1$ to $Z^4$ are N,

Ar is a phenyl group optionally having a substituent or an aromatic 5- or 6-membered heterocyclic group optionally having a substituent,

$R^1$ is a hydrogen, a halogen, an amino optionally substituted with 1 or 2 substituents, an optionally substituted $alkylSO_m$, a cyano, an optionally substituted and optionally branched alkyl, an optionally substituted cycloalkyl, an optionally substituted alkenyl, an optionally substituted alkynyl, an optionally substituted alkoxy, an optionally substituted alkoxycarbonyl, a formyl,

$R^a$, $R^b$, $R^c$, $R^d$ and $R^e$ are the same or different, and are a hydrogen, a halogen, an optionally substituted and optionally branched alkyl, an optionally substituted cycloalkyl, an optionally substituted alkoxy or an optionally substituted $alkylSO_m$, wherein $R^a$ and $R^e$ are not a hydrogen at the same time,

$R^f$ and $R^h$ are a hydrogen, a halogen, an optionally substituted and optionally branched alkyl or an optionally substituted cycloalkyl,

$R^g$ and $R^i$ are a hydrogen, a halogen, an optionally substituted and optionally branched alkyl, an optionally substituted cycloalkyl or an optionally substituted alkoxy;

3. The compound according to the above-mentioned 1, wherein 0 to 1 of $X^1$ to $X^5$ is N, 0 to 2 of $Z^1$ to $Z^4$ are N, Ar is a phenyl group optionally having a substituent or an aromatic 5- or 6-membered heterocyclic group optionally having a substituent, $R^1$ is a hydrogen, a halogen, an amino, a monoalkylamino, a dialkylamino, an $alkylSO_m$, a cyano; an alkyl, a branched alkyl or a cycloalkyl, each of which may be substituted with a halogen, a hydroxy, a cyano, an $alkylSO_m$, an alkoxy or mono- or di-alkylamino; an alkenyl, an alkynyl, an alkoxy, an alkoxycarbonyl or a formyl, $R^a$, $R^b$, $R^c$, $R^d$ and $R^e$ are the same or different and are a hydrogen, a halogen; an alkyl, branched alkyl or a cycloalkyl, each of which may be substituted with halogen; an alkoxy, a haloalkoxy, an alkylthio or a haloalkylthio, respectively, $R^a$ and $R^e$ are not a hydrogen at the same time, $R^f$ and $R^h$ are a hydrogen, a halogen, an alkyl, a branched alkyl or a cycloalkyl, each of which may be substituted with halogen, and $R^g$ and $R^i$ are a hydrogen, a halogen; an alkyl, a branched alkyl, a cycloalkyl or an alkoxy, each of which may be substituted with halogen;

4. The compound according to the above-mentioned 1, wherein A is a single bond, O, $CH_2O$, $OCH_2$, $CH(CH_3)O$, $CH(CN)O$, $OCH(CH_3)$, $CH_2S$, NH or $CH_2NH$, Ar is an optionally halogenated phenyl or pyridyl, pyridazinyl, pyrimidinyl or thiadiazolyl, each of which may be substituted with a halogen or an alkylthio, B is a halogen, an alkyl, a haloalkyl, a haloalkenyl, an alkoxy, a haloalkoxy, a cyano, a nitro or a halophenyl, $R^1$ is H, a halogen, an alkyl, a branched alkyl, a cycloalkyl, a haloalkyl, an alkenyl, an alkynyl, a hydroxyalkyl, a cyanoalkyl, an alkoxy, an $alkylSO_m$, an amino, a dialkylamino, an alkoxyalkyl, an $alkylSO_m$alkyl, a dialkylaminoalkyl, a formyl, an alkoxycarbonyl or a cyano, $R^a$ and $R^e$ are the same or different, and H, a halogen, an alkyl, a haloalkyl, an alkoxy, a haloalkoxy or an alkylthio, respectively (provided that they are not a hydrogen at the same time), $R^b$, $R^d$ and $R^c$ are H or a halogen, $R^f$ and $R^h$ are the same or different and H or a halogen, respectively, $R^g$ and $R^i$ are the same or different and are a hydrogen, a halogen or an alkoxy, respectively, 0 to 1 of $X^1$ to $X^5$ is N, and 0 to 2 of $Z^1$ to $Z^4$ are N;

5. The compound according to the above-mentioned 1, which is 3-(2-chloro-6-fluorophenyl)-1-[4-(3,5-dichloropyridin-2-yloxymethyl)phenyl]-5-methyl-1*H*-1,2,4-triazole, 3-(2-chloro-6-fluorophenyl)-1-[4-(3-chloro-5-trifluoromethylpyridin-2-yloxymethyl)phenyl]-5-methyl-1*H*-1,2,4-triazole or 3-(3-chloropyridin-2-yl)-1-[4-(3-chloro-5-trifluoromethylpyridin-2-yloxymethyl)phenyl]-5-methyl-1*H*-1,2,4-triazole;

6. A process for producing the compound according to the above-mentioned 1, which comprises reacting a compound represented by the formula:

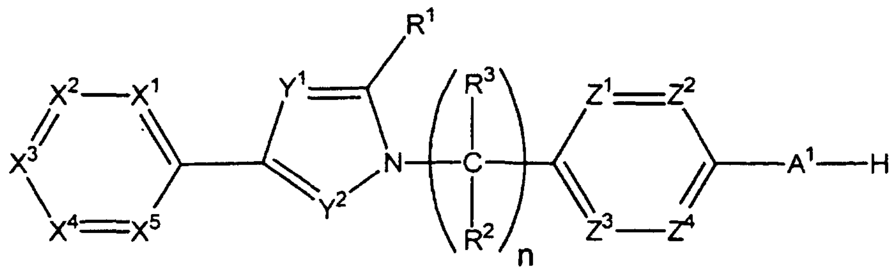

(II)

wherein $A^1$ represents O, $CR^jR^kO$, $SO_m$, $CR^jR^kSO_m$, $NR^l$, or $CR^jR^kNR^l$, and other symbols are as defined in the above 1, with a compound represented by the formula:

$$L^1\text{-Ar-B} \quad \text{(III)}$$

wherein $L^1$ represents a leaving group, and other symbols are as defined in above 1;

7. A process for producing the compound according to the above-mentioned 1, which comprises reacting a compound represented by the formula:

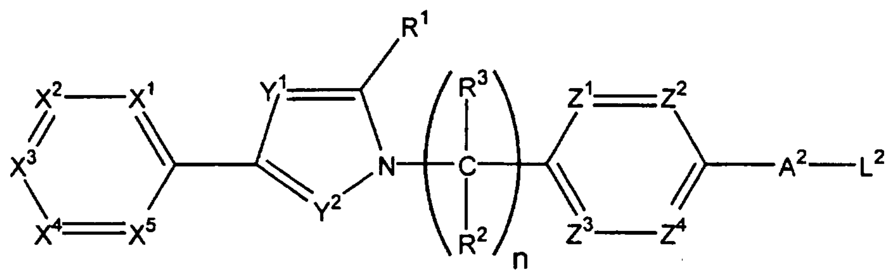

(IV)

wherein $A^2$ represents a single bond or $CR^jR^k$, $L^2$ represents a leaving group, and other symbols are as defined in the above 1,
with a compound represented by the formula:

$$H\text{-}A^3\text{-Ar-B} \quad \text{(V)}$$

wherein $A^3$ represents O, $SO_m$ or $NR^l$, and other symbols are as defined in the above 1;

8. A process for producing the compound according to the above-mentioned 1, which comprises reacting a compound represented by the formula:

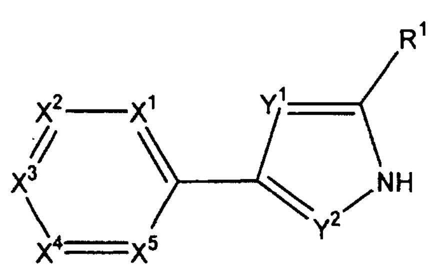

(VI)

wherein respective symbols are as defined in the above 1, with a compound represented by the formula:

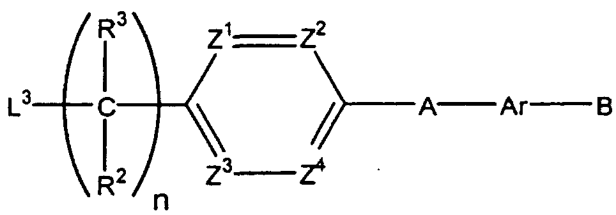

(VII)

wherein $L^3$ represents a leaving group, and other symbols are as defined in the above 1;

9. A process for producing the compound according to the above-mentioned 1 wherein both $Y^1$ and $Y^2$ are N, which comprises:

(1) reacting a compound represented by the formula:

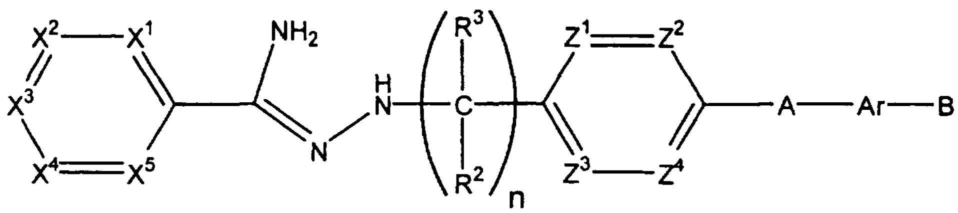

(VIII)

$$R^1\text{-}C(=O)\text{-}L^4 \quad (X)$$

or

$$R^1C(L^{4'})_3 \quad (XI)$$

wherein $L^4$ and $L^{4'}$ represent a leaving group, and $R^1$ is as defined in the above 1, or

(2) reacting the compound represented by the above-mentioned formula (VIII) with a compound represented by the formula :

$$L^5\text{-}C(=O)\text{-}L^6 \quad (XII)$$

wherein $L^5$ and $L^6$ represent a leaving group,
to obtain a compound represented by the formula:

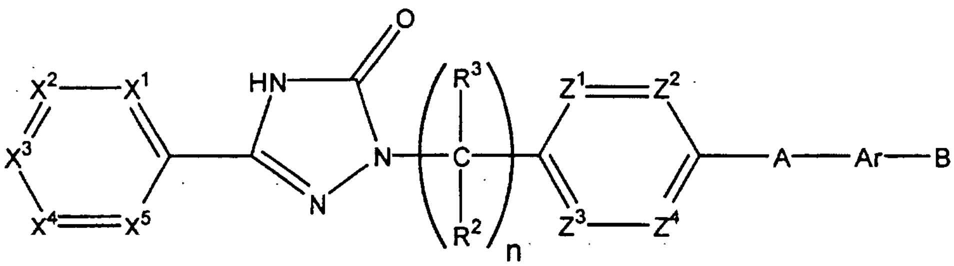

(XIII)

wherein respective symbols are as defined in the above 1, and reacting the resulting compound with a halogenating agent;

10. A process for producing the compound according to the above-mentioned 1 wherein $Y^1$ is CH and $Y^2$ is N, which comprises:

(1) reacting a compound represented by the formula :

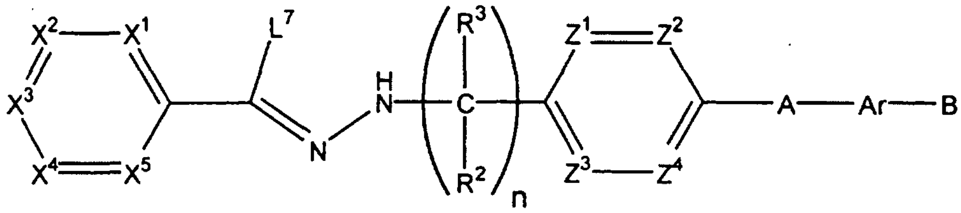

(XIV)

wherein $L^7$ represents a leaving group,
with a compound represented by the formula:

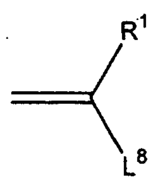

(XV)

wherein $L^8$ represents a leaving group, or
(2) reacting a compound represented by the above-mentioned formula (XIV) with a compound represented by the formula:

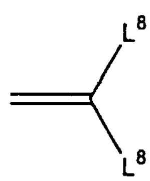

(XVI)

wherein $L^8$ is as defined above,
to obtain a compound represented by the formula:

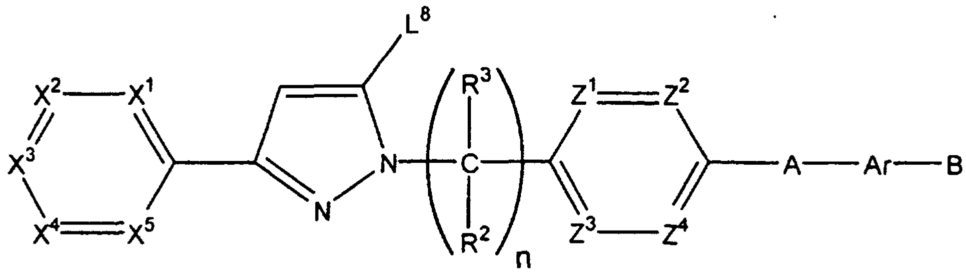

(XVII)

wherein $L^8$ is as defined above, and other symbols are as defined in the above 1, and reacting the resulting compound with a compound represented by the formula:

$$R^1\text{-H} \qquad \text{(XVIII)}$$

wherein $R^1$ is as defined in the above 1;

11. A process for producing the compound according to the above-mentioned 1 wherein $Y^1$ is N and $Y^2$ is N or CH, which comprises reacting a compound represented by the formula:

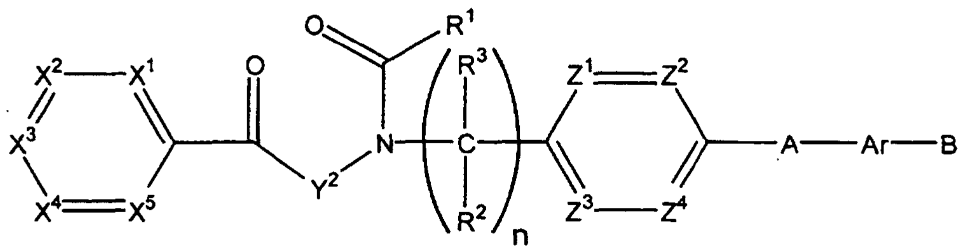

(XIX)

wherein respective symbols are as defined in the above 1, with a compound represented by the formula:

$$\text{Acy-OHNH}_3 \qquad \text{(XX)}$$

wherein Acy represents an acyl group;

12. A process for producing the compound according to the above-mentioned 1 wherein $Y^1$ is N and $Y^2$ is CH or N, which comprises reacting a compound represented by the formula:

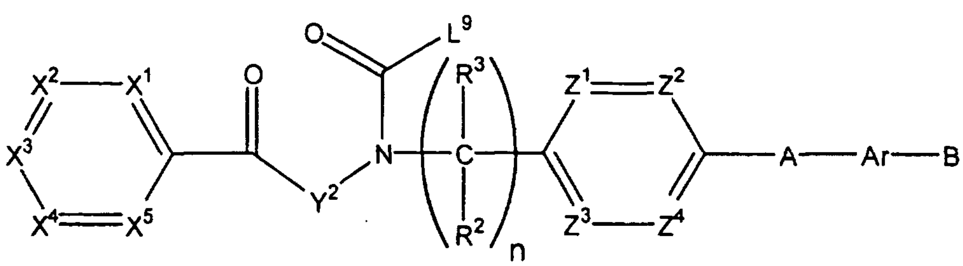

(XXI)

wherein $L^9$ represents a leaving group, with a compound represented by the formula:

$$Acy\text{-}OHNH_3 \qquad (XXII)$$

wherein Acy represents an acyl group, to obtain a compound represented by the formula:

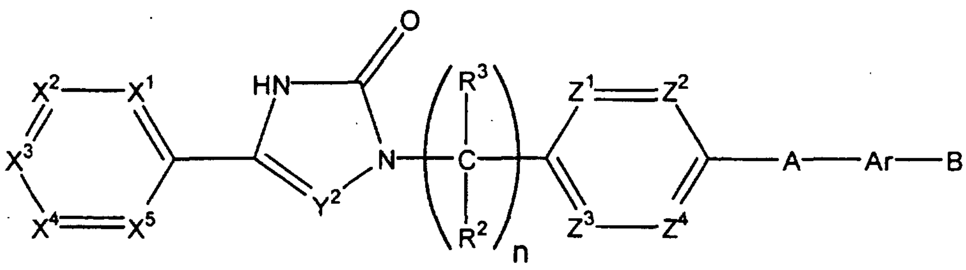

(XXIII)

wherein respective symbols are as defined in the above 1, and reacting the resulting compound with a halogenating agent;

13. A process for producing the compound according to the above-mentioned 1, which comprises reacting a compound represented by the formula:

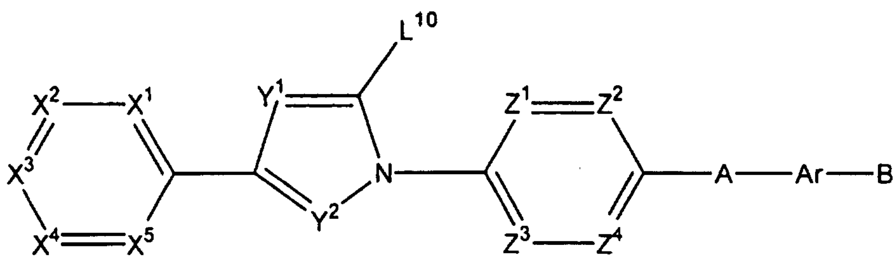

(XXIV)

wherein $L^{10}$ represents a leaving group, and other symbols are as defined in the above 1, with a compound represented by the formula:

$$R^1\text{-}H \qquad (XVIII)$$

wherein $R^1$ is as defined in the above 1;

14. A pest controller which comprises as an active ingredient a compound represented by the formula:

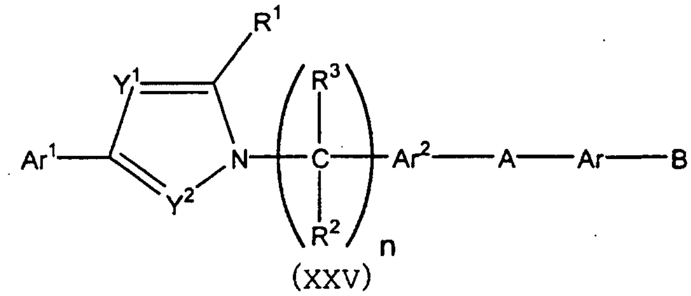

(XXV)

wherein $Ar^1$ represents a 6-membered aromatic hydrocarbon group or a 6-membered nitrogen-containing aromatic heterocyclic group, each of which has a substituent at an ortho position and may be further substituted;
$Ar^2$ represents an optionally substituted 6-membered aromatic hydrocarbon group or an optionally substituted 6-membered nitrogen-containing aromatic heterocyclic group;
$Y^1$ and $Y^2$ are the same or different, and represent N or CH, respectively, provided that they are not CH at the same time;
$R^1$ represents H, a halogen, an optionally substituted and optionally branched alkyl, an optionally substituted cycloalkyl, an optionally substituted alkenyl, an optionally substituted alkynyl, an optionally substituted alkoxy, an optionally substituted alkyl$SO_m$, an amino optionally substituted with 1 or 2 substituents, a cyano, a nitro, a formyl, a hydroxy, an optionally substituted acyl or an optionally substituted alkoxycarbonyl;
$R^2$ and $R^3$ are the same or different, and represent H or an alkyl, respectively, or may form a ring together with a carbon atom which they bind to;
A represents a single bond, O, $CR^jR^kO$, $OCR^jR^k$, $SO_m$, $CR^jR^kSO_m$, $SO_mCR^jR^k$, $NR^l$, $CR^jR^kNR^l$ or $NR^lCR^jR^k$;
Ar represents an aromatic residue optionally having a substituent;
B represents a halogen, an optionally branched alkyl, a cycloalkyl, a haloalkyl, an alkenyl, a haloalkenyl, an alkoxy, a haloalkoxy, an alkyl$SO_m$, a haloalkyl$SO_m$, an amino optionally substituted with 1 or 2 substituents, an alkyl$SO_m$alkyl, a cyano, a nitro or an optionally substituted phenyl;
$R^j$ and $R^k$ are the same or different, and represent H, a cyano or an alkyl, respectively, or may form a ring together with a carbon atom which they bind to; $R^l$ represents H or an alkyl;
m represents 0, 1 or 2; n represents 0 or 1;
provided that, when $Y^1$ and $Y^2$ are N at the same time, and n is 0, and $Ar^1$ is pyridyl, and $R^1$ is hydrogen, a lower alkyl, a lower alkenyl, a lower alkynyl, a haloalkyl, an alkoxyalkyl or an optionally substituted phenyl group, then Ar represents an aromatic heterocyclic residue optionally having a substituent,
when $Y^1$ and $Y^2$ are N at the same time, and n is 0 and $Ar^1$ is a 6-membered aromatic hydrocarbon group, and A is O, S, or $NR^l$, then $R^1$ represents a group other than a halogen,
when $Y^1$ and $Y^2$ are N at the same time, n is 0, and $Ar^1$ is a 6-membered aromatic hydrocarbon group, and A is $OCR^jR^k$ or $SCR^jR^k$, then Ar represents an aromatic hydrocarbon residue optionally having a substituent or an aromatic heterocyclic residue other than an oxazole ring and a thiazole ring which may be substituted and may be fused with other ring,
when $Y^1$ and $Y^2$ are N at the same time, and n is 1, then A represents O, $CR^jR^kO$, or $OCR^j R^k$ and $R^1$ represents a group other than alkyl$SO_m$,
when $Y^1$ and $Y^2$ are N at the same time, n is 0, and A is a single bond, then $Ar^1$ is a 6-membered aromatic hydrocarbon group, or a salt thereof,
15. The pest controller according to the above-mentioned 14, wherein the compound of the formula (XXV) is a compound represented by the formula:

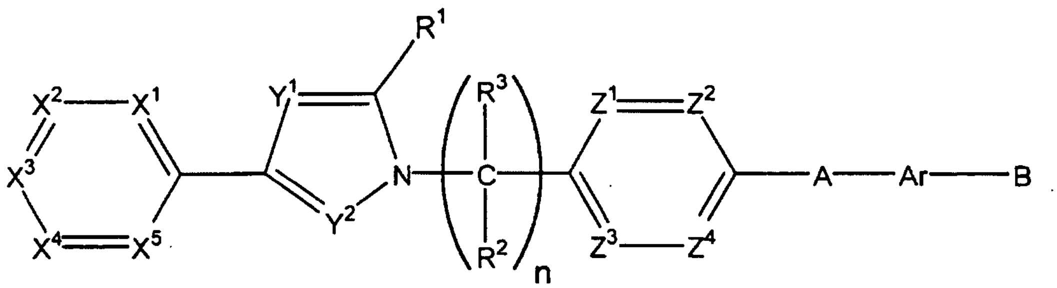

(I)

wherein $X^1$ represents N or C-$R^a$, $X^2$ represents N or C-$R^b$, $X^3$ represents N or C-$R^c$, $X^4$ represents N or C-$R^d$ and $X^5$ represents N or C-$R^e$;

$Y^1$ and $Y^2$ are the same or different, and represent N or CH, respectively, provided that they are not CH at the same time;

$Z^1$ represents N or C-$R^f$, $Z^2$ represents N or C-$R^g$, $Z^3$ represents N or C-$R^h$ and $Z^4$ represents N or C-$R^i$;

A represents a single bond, O, $CR^jR^kO$, $OCR^jR^k$, $SO_m$, $CR^jR^kSO_m$, $SO_mCR^jR^k$, $NR^l$, $R^jR^kNR^l$ or $NR^lR^jR^k$;

Ar represents an aromatic residue optionally having a substituent;

B represents a halogen, an optionally branched alkyl, a cycloalkyl, a haloalkyl, an alkenyl, a haloalkenyl, an alkoxy, a haloaokoxy, an alkyl$SO_m$, a haloalkyl$SO_m$, an amino optionally substituted with 1 or 2 substituents, an alkyl$SO_m$alkyl, a cyano, a nitro or an optionally substituted phenyl;

$R^1$ represents H, a halogen, an optionally substituted and optionally branched alkyl, an optionally substituted cycloalkyl, an optionally substituted alkenyl, an optionally substituted alkynyl, an optionally substituted alkoxy, an optionally substituted alkyl$SO_m$, an amino optionally substituted with 1 or 2 substituents, a cyano, a nitro, a formyl, a hydroxy, an optionally substituted acyl or an optionally substituted alkoxycarbonyl;

$R^2$ and $R^3$ are the same or different and represent H or an alkyl, respectively, or may form a ring together with a carbon atom which they bind to;

$R^a$ and $R^e$ are the same or different and represent H, a halogen, an optionally substituted and optionally branched alkyl, an optionally substituted cycloalkyl, an optionally substituted alkoxy or an optionally substituted alkyl$SO_m$, or an amino optionally substituted with 1 or 2 substituents, respectively, provided that they are not H at the same time;

$R^b$ and $R^d$ are the same or different and represent H, a halogen, an optionally substituted and optionally branched alkyl, an optionally substituted cycloalkyl, an optionally substituted alkenyl, an optionally substituted alkynyl, an optionally substituted alkoxy, an optionally substituted alkyl$SO_m$, an amino optionally substituted with 1 or 2 substituents, an optionally substituted acyl, an optionally substituted alkoxycarbonyl, a cyano, a nitro, a formyl or a hydroxy, respectively;

$R^c$ represents H, a halogen, an optionally substituted and optionally branched alkyl, an optionally substituted cycloalkyl, an optionally substituted alkenyl, an optionally substituted alkynyl, an optionally substituted alkoxy, an optionally substituted alkyl$SO_m$, an amino optionally substituted with 1 or 2 substituents, an optionally substituted acyl, an optionally substituted alkoxycarbonyl, a nitro, a formyl or a hydroxy;

$R^f$ and $R^h$ are the same or different and represent H, a halogen, an optionally substituted and optionally branched alkyl, an optionally substituted cycloalkyl; an optionally substituted alkenyl, an optionally substituted alkynyl, an optionally substituted acyl, an optionally substituted alkoxycarbonyl, a cyano, a nitro, a formyl or a hydroxy, respectively;

$R^g$ and $R^i$ are the same or different and represent H, a halogen, an optionally substituted and optionally branched alkyl, an optionally substituted cycloalkyl, an optionally substituted alkenyl, an optionally substituted alkynyl, an optionally substituted alkoxy, an optionally substituted alkyl$SO_m$, an amino optionally substituted with 1 or 2 substituents, an optionally substituted acyl, an optionally substituted alkoxycarbonyl, a cyano, a nitro, a formyl or a hydroxy, respectively;

$R^j$ and $R^k$ are the same or different and represent H, a cyano or an alkyl, respectively, or may form a ring together with a carbon atom which they bind to; $R^l$ represents H or an alkyl;

m represents 0, 1 or 2; n represents 0 or 1; provided that, when $Y^1$ and $Y^2$ are N at the same time, and n is 0, and one of $X^1$ to $X^5$ is N, and $R^1$ is hydrogen, a lower alkyl, a lower alkenyl, a lower alkynyl, a haloalkyl, an alkoxyalkyl or an optionally substituted phenyl group, then Ar represents an aromatic heterocyclic residue optionally having a substituent,

when $Y^1$ and $Y^2$ are N at the same time, and n is 0, and $X^1$ to $X^5$ are not N, and A is O, S or $NR^l$, then $R^1$ represents a group other than halogen,

when $Y^1$ and $Y^2$ are N at the same time, and n is 0, and $X^1$ to $X^5$ are not N, and A is $OCR^jR^k$ or $SCR^jR^k$, then Ar represents an aromatic hydrocarbon group optionally having a substituent or an aromatic heterocyclic residue other than an oxazole ring and a thiazole ring which may be substituted and may be fused with other ring,
when $Y^1$ and $Y^2$ are N at the same time, and n is 1, then A represents O, $CR^jR^kO$ or $OCR^jR^k$ and $R^1$ represents a group other than an $alkylSO_m$,
when $Y^1$ and $Y^2$ are N at the same time, n is 0, and A is a single bond, then $X^1$ to $X^5$ are not N,
or a salt thereof;
16. The pest controller according to the above-mentioned 15, wherein A is a single bond, O, $CH_2O$, $OCH_2$, $CH(CH_3)O$, CH(CN)O, $OCH(CH_3)$, $CH_2S$, NH or $CH_2NH$, Ar is an optionally halogenated phenyl, or pyridyl, pyridazinyl, pyrimidinyl or thiadiazolyl, each of which may be substituted with a halogen or an alkylthio, B is a halogen, an alkyl, a haloalkyl, a haloalkenyl, an alkoxy, a haloalkoxy, a cyano, a nitro or a halophenyl, $R^1$ is H, a halogen, an alkyl, a branched alkyl, a cycloalkyl, a haloalkyl, an alkenyl, an alkynyl, a hydroxyalkyl, a cyanoalkyl, an alkoxy, an $alkylSO_m$, an amino, a dialkylamino, an alkoxyalkyl, an $alkylSO_m$alkyl, a dialkylaminoalkyl, a formyl, an alkoxycarbonyl or a cyano, $R^a$ and $R^e$ are the same or different and are H, a halogen, an alkyl, a haloalkyl, an alkoxy, a haloalkoxy or an alkylthio, respectively (provided that, they are not hydrogen at the same time), $R^b$, $R^d$ and $R^c$ are H or a halogen, $R^f$ and $R^h$ are the same or different and are a hydrogen or a halogen, respectively, $R^g$ and $R^i$ are the same or different and are a hydrogen, a halogen or an alkoxy, respectively, 0 to 1 of $X^1$ to $X^5$ is N, and 0 to 2 of $Z^1$ to $Z^4$ are N;
17. The pest controller according to the above-mentioned 14, which comprises 3-(2-chloro-6-fluorophenyl)-1-[4-(3,5-dichloropyridin-2-yloxymethyl)phenyl]-5-methyl-1*H*-1,2,4-triazole, 3-(2-chloro-6-fluorophenyl)-1-[4-(3-chloro-5-trifluoromethylpyridin-2-yloxymethyl)phenyl]-5-methyl-1*H*-1,2,4-triazole or 3-(3-chloropyridin-2-yl)-1-[4-(3-chloro-5-trifluoromethylpyridin-2-yloxymethyl)phenyl]-5-methyl-1*H*-1,2,4-triazole; and
18. The pest controller according to any one of the above-mentioned 14 to 17, which is an insecticide or an acaricide.

## Detailed Description of the Invention

In Compound (I) or (XXV), there may be geometrical isomers and/or steric isomers, and the present invention includes those respective isomers and mixtures of those isomers.

As used herein, the term "lower" regarding a substituent denotes a substituent having a carbon number of 1 to 6, preferably 1 to 4.

In Compound (I), $X^1$ represents N or C-$R^a$, $X^2$ represents N or C-$R^b$, $X^3$ represents N or C-$R^c$, $X^4$ represents N or C-$R^d$, and $X^5$ represents N or C-$R^e$.

$R^a$ and $R^e$ are the same or different, and represent H, a halogen, an optionally substituted and optionally branched alkyl, an optionally substituted cycloalkyl, an optionally substituted alkoxy or an optionally substituted $alkylSO_m$ (m is 0 to 2), or an amino optionally substituted with 1 or 2 substituents, provided that they are not H at the same time. That is, a 6-membered aromatic ring constituted with $X^1$ to $X^5$ has a substituent at an ortho position.

Examples of halogen include fluorine, chlorine, bromine, iodine and the like.

Examples of the optionally substituted and optionally branched alkyl include an optionally substituted $C_{1-6}$ alkyl group (e.g. methyl, ethyl, n-propyl, isopropyl, n-butyl, isobutyl, sec-butyl, tert-butyl etc.) and the like. Examples of a substituent for the alkyl include a hydroxyl group, an amino group, a mono- or di-$C_{1-6}$ alkylamino (e.g. methylamino, ethylamino, propylamino, dimethylamino, diethylamino etc.), a $C_{1-6}$ alkoxy group (e.g. methoxy, ethoxy, propoxy, isopropoxy, butoxy etc.), a $C_{1-4}$ alkylthio group (e.g. methylthio, ethylthio, n-propylthio, isopropylthio, butylthio etc.), a halogen (e.g. fluorine, chlorine, bromine, iodine), a carboxyl group, a nitro group, a cyano group and the like.

Examples of the optionally substituted cycloalkyl include an optionally substituted $C_{3-9}$ cycloalkyl group (e.g. cyclopropyl, cyclobutyl, cyclopentyl, cyclohexyl etc.) and the like and the substituent is exemplified by those for the aforementioned alkyl.

Examples of the optionally substituted alkoxy include an optionally substituted $C_{1-6}$ alkoxy group (e.g. methoxy, ethoxy, propoxy, isopropoxy, butoxy etc.) and the like and the substituent is exemplified by those for the aforementioned alkyl.

Examples of the optionally substituted $alkylSO_m$. include an optionally substituted $C_{1-6}$alkylthio group (e.g. methylthio, ethylthio, n-propylthio, isopropylthio, n-butylthio etc.), an optionally substituted $C_{1-6}$ alkylsulfinyl group (e.g. methylsulfinyl, ethylsulfinyl, n-propylsulfinyl, isopropylsulfinyl, n-butylsulfinyl), an optionally substituted $C_{1-6}$ alkylsulfonyl group (e.g. methylsulfonyl, ethylsulfonyl, n-propylsulfonyl, isopropylsulfonyl, n-butylsulfonyl etc.) and the like and the substituent is exemplified by those for the aforementioned alkyl.

The number of substitution is 1 to 6, preferably 1 to 3, within a replaceable range.

Examples of the amino substituted with 1 or 2 substituents include a mono- or di-$C_{1-6}$ alkylamino group (e.g. methylamino, ethylamino, propylamino, isopropylamino, dimethylamino, diethylamino, methylethylamino etc.) and the like.

$R^b$ and $R^d$ are the same or different and represent H, a halogen, an optionally substituted and optionally branched alkyl, an optionally substituted cycloalkyl, an optionally substituted alkenyl, an optionally substituted alkynyl, an optionally substituted alkoxy, an optionally substituted alkyl$SO_m$, an amino optionally substituted with 1 or 2 substituents, an optionally substituted acyl, an optionally substituted alkoxycarbonyl, a cyano, a nitro, a formyl or a hydroxy, respectively.

As a halogen, an optionally substituted and optionally branched alkyl, an optionally substituted cycloalkyl, an optionally substituted alkenyl, an optionally substituted alkynyl, an optionally substituted alkoxy, an optionally substituted alkyl$SO_m$ and an amino optionally substituted with 1 or 2 substituents, the same groups as those described above are used.

Examples of the acyl group of the optionally substituted acyl group include an acyl group having 1 to 20 carbon atoms which is derived from carboxylic acid and, for example, (1) formyl, (2) an alkanoyl group, preferably, an alkanoyl having 2 to 10 carbon atoms (e.g. $C_{1-9}$ alkyl-carbonyl group such as acetyl, propionyl, butyryl, isobutyl, pentanoyl, hexanoyl, heptanoyl, pivaloyl etc.), (3) a cycloalkanoyl group, preferably a cycloalkanoyl group having 4 to 10 carbon atoms (e.g. cyclopropylcarbonyl, cyclobutylcarbonyl, cyclopentylcarbonyl, cyclohexylcarbonyl etc.), (4) an alkenylcarbonyl group, preferably an alkenylcarbonyl group having 3 to 10 carbon atoms (e.g. acryloyl, allylcarbonyl, isopropenylcarbonyl, isobutenylcarbonyl, 1-methylallylcarbonyl, cinnamoyl etc.), (5) an alkynylcarbonyl group, preferably an alkynylcarbonyl group having 3 to 7 carbon atoms (e.g. propyncarbonyl, propargylcarbonyl, 2-butynylcarbonyl, 3-butynylcarbonyl, 3-pentynylcarbonyl etc.), (6) an arylcarbonyl group, preferably an arylcarbonyl group having 7 to 14 carbon atoms (e.g. benzoyl, 1-naphthoyl, 2-naphthoyl etc.), (7) an alkoxycarbonyl group, preferably an alkoxycarbonyl group having 2 to 6 carbon atoms (e.g. methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl etc.), (8) an aryloxycarbonyl group, preferably an aryloxycarbonyl group having 7 to 14 carbon atoms (e.g. phenoxycarbonyl group), (9) an aralkylcarbonyl group, preferably an aralkylcarbonyl group having 8 to 19 carbon atoms (e.g. phenyl-$C_{1-4}$ alkylcarbonyl such as benzylcarbonyl, phenethylcarbonyl, phenylpropylcarbonyl etc., benzhydrylcarbonyl, naphthyl-$C_{1-4}$ alkylcarbonyl such as 1-naphthylethylcarbonyl etc.), (10) an aralkyloxycarbonyl group, preferably an aralkyloxycarbonyl group having 8 to 19 carbon atoms (e.g. phenyl-$C_{1-4}$ alkyloxycarbonyl such as benzyloxycarbonyl, phenethyloxycarbonyl, phenylpropyloxycarbonyl), (11) a carbamoyl group, and (12) a cyclic aminocarbonyl group (e.g. 1-pyrrolidinocarbonyl, piperidinocarbonyl, morpholinocarbonyl, thiomorpholinocarbonyl, 1-perhydroazepinylcarbonyl etc.) are used.

When the acyl group is an alkanoyl group, an alkenylcarbonyl group or an alkynylcarbonyl group, the acyl group may have 1 to 6 (preferably 1 to 3) substituents selected from the group consisting of a hydroxyl group, an amino group, a mono- or di-$C_{1-6}$ alkylamino group (e.g. methylamino, ethylamino, propylamino, dimethylamino, diethylamino etc.), a $C_{1-6}$ alkoxy group (e.g. methoxy, ethoxy, propoxy, isopropoxy, butoxy etc.), a $C_{1-6}$ alkylthio group (e.g. methylthio, ethylthio, n-propylthio, isopropylthio, n-butylthio etc.), a halogen atom (e.g. fluorine, chlorine, bromine, iodine), a carboxyl group, a nitro group, a cyano group, and a phenyl group.

When the acyl group is a cycloalkanoyl group, an arylcarbonyl group, an alkoxycarbonyl group, an aryloxycarbonyl group, an aralkylcarbonyl group or an aralkyloxycarbonyl group, the acyl group may have 1 to 5 (preferably 1 to 3) substituents selected from the group consisting of a hydroxyl group, an amino group, a mono-or di-$C_{1-6}$ alkylamino group (e.g. methylamino, ethylamino, propylamino, dimethylamino, diethylamino etc.), a $C_{1-6}$ alkoxy group (e.g. methoxy, ethoxy, propoxy, isopropoxy, butoxy etc.), a $C_{1-6}$ alkylthio group (e.g. methylthio, ethylthio, n-propylthio, isopropylthio, n-butylthio etc.), a halogen atom (e.g. fluorine, chlorine, bromine, iodine), a carboxyl group, a nitro group, a cyano group, a phenyl group, a $C_{1-6}$ alkyl group (e.g. methyl, ethyl, n-propyl, isopropyl, n-butyl, isobutyl, sec-butyl, tert-butyl etc.), a $C_{2-6}$ alkenyl group (e.g. vinyl, allyl, 1-propenyl, 1-butenyl, 2-butenyl etc.), and a $C_{2-6}$ alkynyl group (e.g. ethynyl, 1-propynyl, propargyl, 1-butynyl etc.).

When the acyl group is a carbamoyl group, the acyl group may have 1 or 2 substituents selected from the group consisting of (1) a $C_{1-6}$ alkyl group (e.g. methyl, ethyl, n-propyl, isopropyl, n-butyl, isobutyl, sec-butyl, tert-butyl etc.), (2) a $C_{3-9}$ cycloalkyl group (e.g. cyclopropyl, cyclobutyl, cyclopentyl, cyclohexyl etc.), (3) a $C_{2-6}$ alkenyl group (e.g. vinyl, allyl, 1-propenyl, 1-butenyl, 2-butenyl etc.), (4) a $C_{2-6}$ alkynyl group (e.g. ethynyl, 1-propynyl, propargyl, 1-butynyl etc.), (5) a hydroxyl group, (6) a $C_{1-6}$ alkoxy group (e.g. methoxy, ethoxy, propoxy, isopropoxy, butoxy etc.), (7) an amino group, (8) a mono- or di-$C_{1-6}$ alkylamino group (e.g. methylamino, ethylamino, propylamino, dimethylamino, diethylamino etc.), (9) a cyclic amino group (e.g. 1-pyrrolidino, piperidino, morpholino, 4-methyl-1-piperazino etc.) and (10) a phenyl group, and the substituent group may form a cyclic amino group together with a nitrogen atom which it binds to (e.g. 1-pyrrolidino, piperidino, morpholino, thiomorpholino, 4-methyl-1-piperazino etc.). Further, the substituent may be substituted with 1 to 6 (preferably 1 to 3) substituents selected from the group consisting of a hydroxyl group, an amino group, a mono- or di-$C_{1-6}$ alkylamino group (e.g. methylamino, ethylamino, propylamino, dimethylamino, diethylamino etc.), a $C_{1-6}$ alkoxy group (e.g. methoxy, ethoxy, propoxy, isopropoxy, butoxy etc.), a $C_{1-6}$ alkylthio group (e.g. methylthio, ethylthio, n-propylthio, isopropylthio, n-butylthio etc.), a halogen atom (e.g. fluorine, chlorine, bromine, iodine), a phenyl group, a carboxyl group, a nitro group and a cyano group.

Examples of the optionally substituted alkoxycarbonyl include an optionally substituted $C_{1-6}$ alkoxycarbonyl

group (e.g. methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl etc.) and the like, and the substituent is exemplified by those for the aforementioned alkyl.

$R^c$ represents H, a halogen, an optionally substituted and optionally branched alkyl, an optionally substituted cycloalkyl, an optionally substituted alkenyl, an optionally substituted alkynyl, an optionally substituted alkoxy, an optionally substituted alkyl$SO_m$, an amino optionally substituted with 1 or 2 substituents, an optionally substituted acyl, an optionally substituted alkoxycarbonyl, a nitro, a formyl or a hydroxy and, for example, the same groups as those described above can be used.

Preferably, $R^a$ and $R^e$ are the same or different, and are H, a halogen, a haloalkyl, an alkoxy, a haloalkoxy, an alkylthio or an alkyl, respectively (provided that they are not H at the same time), and $R^b$, $R^d$ and $R^c$ are H or a halogen.

$Y^1$ and $Y^2$ are the same or different, and represent N or CH, respectively, provided that they are not CH at the same time. That is, Compound (I) is an imidazole, pyrazole or triazole compound.

$R^1$ represents H, a halogen, an optionally substituted and optionally branched alkyl, an optionally substituted cycloalkyl, an optionally substituted alkenyl, an optionally substituted alkynyl, an optionally substituted alkoxy, an optionally substituted alkyl$SO_m$, an amino optionally substituted with 1 or 2 substituents, a cyano, a nitro, a formyl, a hydroxy, an optionally substituted acyl or an optionally substituted alkoxycarbonyl and, for example, the same groups as those described above are used. Preferably, $R^1$ is H, a halogen, an optionally branched alkyl, a cycloalkyl, a haloalkyl, a hydroxyalkyl, a cyanoalkyl, an alkoxy, an alkyl$SO_m$, an amino, a dialkylamino, an alkoxyalkyl, an alkyl$SO_m$alkyl, a dialkylaminoalkyl, an alkenyl, an alkynyl, a formyl, an alkoxycarbonyl or a cyano.

$R^2$ and $R^3$ are the same or different, and represent H or an alkyl, respectively, or may form a ring together with a carbon atom which they bind to. Examples of alkyl include the aforementioned $C_{1-6}$alkyl and the like, and examples of the ring formed by $R^2$ and $R^3$ include a 3- to 8-membered homocyclic or heterocyclic ring (e.g. cyclopropane, cyclobutane, cyclopentane, cyclohexane, aziridine, azetidine, morpholine, thiomorpholine, piperazine, piperidine, pyrrolidine, hexahydropyrimidine etc.) and the like. Preferred is H.

n is 0 or 1.

$Z^1$ represents N or C-$R^f$, $Z^2$ represents N or C-$R^g$, $Z^3$ represents N or C-$R^h$, and $Z^4$ represents N or C-$R^i$.

$R^f$ and $R^h$ are the same or different, and represent H, or the aforementioned halogen, optionally substituted and optionally branched alkyl, optionally substituted cycloalkyl, optionally substituted alkenyl, optionally substituted alkynyl, optionally substituted acyl, optionally substituted alkoxycarbonyl, cyano, nitro, formyl or hydroxy, respectively.

$R^g$ and $R^i$ are the same or different, and represent H, or the aforementioned halogen, optionally substituted and optionally branched alkyl, optionally substituted cycloalkyl, optionally substituted alkenyl, optionally substituted alkynyl, optionally substituted alkoxy, optionally substituted alkyl$SO_m$, amino optionally substituted with 1 or 2 substituents, optionally substituted acyl, optionally substituted alkoxycarbonyl, cyano, nitro, formyl or hydroxy.

Preferably, $R^f$, $R^g$, $R^h$ and $R^i$ are the same or different, and are H, a halogen or an alkoxy, respectively.

A represents a single bond, O, $CR^jR^kO$, $OCR^jR^k$, $SO_m$, $CR^jR^kSO_m$, $SO_mCR^jR^k$, $NR^l$, $CR^jR^kNR^l$ or $NR^lCR^jR^k$.

$R^j$ and $R^k$ are the same or different, and represent H, cyano or the aforementioned alkyl, or may form the aforementioned ring together with a carbon atom which they bind to.

$R^l$ represents H or the aforementioned alkyl.

Preferably, A is a single bond, O, $CH_2O$, $OCH_2$, $CH(CH_3)O$, CH(CN)O, $OCH(CH_3)$, $CH_2S$, NH or $CH_2NH$.

Ar represents an aromatic residue optionally having a substituent, for example, an aromatic hydrocarbon residue optionally having a substituent or an aromatic heterocyclic residue optionally having a substituent, and further, these groups may be fused with 1 or more carbocycles or heterocycles. As examples thereof, examples of the aromatic hydrocarbon residue include a $C_{6-14}$ aryl (e.g. phenyl, 1-naphthyl, 2-naphthyl, 2-anthryl etc.) and the like, and examples of an aromatic heterocyclic ring include a 5- to 10-membered aromatic haeterocycle (e.g. 2- or 3-thienyl, thiadiazolyl, 2-, 3-or 4-pyridyl, 3- or 4-pyridazinyl, 2-, 4-or 5-pyrimidinyl, 2-, 3-, 4-, 5- or 8-quinolyl, 1-, 3-, 4- or 5-isoquinolyl, 1-, 2- or 3-indolyl, 2-benzothiazolyl, 2-benzo[b]thienyl, benzo[*b*]indolyl, 2-or 3-imidazo[1,2-*a*]pyridinyl, 2-, 3-or 6-imidazo [1,2-*b*]pyridazinyl) and the like.

Examples of the substituent for these groups include a halogen (e.g. fluorine, chlorine, bromine, iodine etc.), $C_{1-3}$ alkylenedioxy (e.g. methylenedioxy, ethylenedioxy etc.), nitro, cyano, an optionally halogenated $C_{1-6}$ alkyl (e.g. methyl, ethyl, propyl, isopropyl, butyl, isobutyl, sec-butyl, tert-butyl, pentyl, hexyl etc.), an optionally halogenated $C_{2-6}$ alkenyl (e.g. vinyl, allyl, isopropenyl, butenyl, isobutenyl, sec-butenyl etc.), an optionally halogenated $C_{2-6}$alkynyl (e.g. ethynyl, propargyl, butynyl, 1-hexynyl etc.), an optionally halogenated $C_{3-6}$ cycloalkyl (e.g. cyclopropyl, cyclobutyl, cyclopentyl, cyclohexyl etc.), a $C_{6-14}$ aryl (e.g. phenyl, 1-naphthyl, 2-naphthyl, biphenylyl, 2-anthryl etc.), an optionally halogenated $C_{1-6}$ alkoxy (e.g. methoxy, ethoxy, propoxy, isopropoxy, butoxy, isobutoxy, sec-butoxy, pentyloxy, hexyloxy etc.), an optionally halogenated aforementioned $C_{1-6}$ alkylthio or mercapto, hydroxy, an aforementioned mono- or di-$C_{1-6}$ alkylamino, a mono- or di-$C_{6-14}$arylamino (e.g. phenylamino, 1-naphthylamino, diphenylamino etc.), an aforementioned acyl, acylamino, acyloxy, a 5- to 7-membered cyclic amino optionally having a substituent (e.g. morpholino, thiomorpholino, pyperazino-1-yl, piperidino, pyrrolidin-1-yl etc.), an aforementioned 5- to 10-membered aromatic het-

erocyclic group, sulfo, a $C_{6-14}$ aryloxy (e.g. phenyloxy, naphthyloxy etc.) and the like. The number of substitution is 1 to 6, preferably 1 to 3 within a replaceable range.

Preferable Ar is an optionally halogenated phenyl, or pyridyl, pyridazinyl, pyrimidinyl or thiadiazolyl, each of which may be substituted with a halogen.

B represents a halogen, an optionally branched alkyl, a cycloalkyl, a haloalkyl, an alkenyl, a haloalkenyl, an alkoxy, a haloalkoxy, an alkyl$SO_m$, a haloalkyl$SO_m$, an amino, an alkylamino, a dialkylamino, an alkyl$SO_m$alkyl, a cyano, a nitro or an optionally substituted phenyl, each of which is exemplified above. Preferably, B is halogen, alkyl, haloalkyl, haloalkenyl, alkoxy, cyano, nitro or halophenyl.

n is 0 or 1.

In Compound (XXV), $Ar^l$ represents a 6-membered aromatic hydrocarbon group (e.g. phenyl etc.) or 6-membered nitrogen-containing aromatic heterocyclic group (e.g. pyridyl etc.), which has a substituent at an ortho position and may be further substituted, and $Ar^2$ represents a 6-membered aromatic hydrocarbon group or 6-membered nitrogen-containing aromatic heterocyclic group. As a substituent for them, the same substituents as those exemplified for the aforementioned Ar are used.

Other symbols are as defined above.

Examples of a particularly preferable compound of the present invention include:

3-(2-chloro-6-fluorophenyl)-1-[4-(3,5-dichloropyridin-2-yloxymethyl)phenyl]-5-methyl-1H-1,2,4-triazole,
3-(2-chloro-6-fluorophenyl)-1-[4-(3-chloro-5-trifluoromethylpyridin-2-yloxymethyl)phenyl]-5-methyl-1H-1,2,4-triazole, and
3-(3-chloropyridin-2-yl)-1-[4-(3-chloro-5-trifluoromethylpyridin-2-yloxymethyl)phenyl]-5-methyl-1H-1,2,4-triazole.

Compounds (I) and (XXIV) of the present invention may form a salt in some cases, which are included in the present invention. Preferably, salts are agriculturally acceptable salts. That is, when the compound has an acidic group such as a carboxyl group and a sulfo group in a molecule, the compound may form a salt with a base and, as the base, for example, inorganic bases such as alkali metals such as sodium, potassium, lithium and the like, alkaline earth metals such as calcium, magnesium and the like and ammonia, and organic bases such as pyridine, collidine, triethylamine and triethanolamine are used. In addition, when the compound has a basic group such as an amino group in a molecule, the compound may form a salt with an acid and, as the acid, for example, inorganic acids such as hydrochloric acid, hydrobromic acid, hydroiodic acid, phosphoric acid, sulfuric acid, perchloric acid and the like, or salts with organic acids such as formic acid, acetic acid, tartaric acid, malic acid, citric acid, oxalic acid, succinic acid, benzoic acid, picric acid, methanesulfonic acid, *p*-toluenesulfonic acid and the like are used. In addition, Compounds (I) and (XXIV) may form an intramolecular salt in some cases, which are included in the present invention.

The process for producing Compound (I) will be illustrated below.

Compound (I) can be produced, for example, by the production processes shown in the following A to H:

Production Process A

Compound (I) can be produced by reacting a compound represented by the formula:

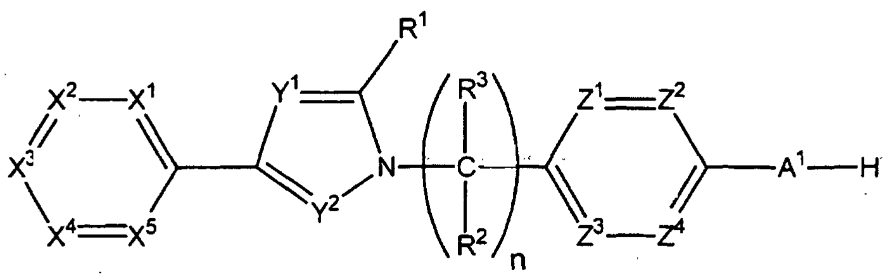

(II)

wherein $A^1$ represents O, $CR^jR^kO$, $SO_m$, $CR^jR^kSO_m$, $NR^l$ or $CR^jR^kNR^l$, and other symbols are as defined above, with a compound represented by the formula:

$$L^1\text{-Ar-B} \quad (III)$$

wherein $L^1$ represents a leaving group, and other symbols are as defined above.

As a leaving group represented by $L^1$, for example, a halogen (e.g. fluorine, chlorine, bromine, iodine), an acyloxy group ($C_{1-10}$acyloxy group such as formyloxy group; $C_{1-6}$ alkylcarbonyloxy group optionally substituted with 1 to 3 halogens such as acetoxy group, propionyloxy group and trifluoroacetoxy group; $C_{1-6}$ alkoxycarbonyloxy group such as methoxycarbonyloxy and t-butoxycarbonyloxy), a group represented by the formula: $RSO_m$ (wherein R represents alkyl or phenyl, and m is 0, 1 or 2), a group represented by the formula: $R'SO_3$ (wherein R' represents alkyl or alkylphenyl) and the like can be used.

In the present reaction, the amount of the aforementioned compound of the formula (III) is not particularly limited, and the compound may be used as a solvent in a large excessive amount, but preferably the amount is about 0.8 to 5 equivalent.

Favorable results can occasionally be obtained by the presence of a base or action of a base before or after the reaction for the purpose of promoting the reaction and reducing side products. As these bases, for example, alkali metal alcoholate such as sodium ethylate, sodium methylate, potassium tert-butoxide and the like, organic bases such as triethylamine, diisopropylethylamine, pyridine, 4-dimethylaminopyridine, *N,N*-dimethylaniline and the like, inorganic bases such as potassium carbonate, sodium carbonate, sodium hydroxide, potassium hydroxide, sodium bicarbonate, potassium bicarbonate and the like, metal hydrides such as lithium hydride, sodium hydride, potassium hydride and the like, and organic lithium reagents such as butyllithium, lithiumdiisopropylamide and the like can be used. The amount of a base to be used is not particularly limited as far as it does not adversely affect the reaction, and a base can be used in a large excessive amount also doubling as a solvent.

Further, favorable results can be obtained in some cases by the presence of a catalyst for the purpose of promoting the reaction and reducing side products. As such catalyst, for example, quaternary ammonium salts such as trioctylmethylammonium chloride, triethylbenzylammonium chloride, tetrabutylammonium chloride and the like, and crown ethers such as 18-crown-6, 15-crown-5 and the like can be used. An amount of the catalyst to be used is not particularly limited as far as it does not adversely affect the reaction, and the amount is preferably about 0.001 to 0.1 equivalent.

The present reaction can be carried out using a suitable solvent. The solvent is not particularly limited as far as it does not react with a reaction substrate, a reaction reagent and a product to give side products, and a solvent which dissolves both reaction substrate and reaction regent is desirable. As such solvent, aliphatic hydrocarbons such as pentane, hexane, heptane, petroleum ether and the like, aromatic hydrocarbons such as benzene, toluene, xylene and the like, esters such as methyl acetate, ethyl acetate, ethyl formate, ethyl propionate and the like, ketones such as acetone, methyl ethyl ketone and the like, ethers such as diethyl ether, dipropyl ether, diisopropyl ether, dibutyl ether, tetrahydrofuran, dioxane and the like, nitriles such as acetonitrile, propionitrile and the like, acid amides such as dimethylformamide, dimethylacetamide and the like, sulfoxides such as dimethyl sulfoxide and the like, sulfones such as sulfolane and the like, phosphoric acid amides such as hexamethylphosphoramide and the like, halogenated hydrocarbons such as dichloromethane, chloroform, 1,2-dichloroethane, carbon tetrachloride and the like, aromatic amines such as pyridine, picoline, lutidine, quinoline and the like, and mixed solvents thereof, water, and mixed solvents thereof with water are used.

The reaction temperature is usually about -50 to 200°C, preferably about -30 to 150°C. The reaction time is generally about 0.1 to 96 hours, preferably 0.1 to 72 hours, more preferably about 0.1 to 24 hours.

The resulting compound may be supplied for the following reaction as a raw material after isolated or purified by known means such as concentration, concentration under reduced pressure, liquid nature conversion, transference dissolution, solvent extraction, distillation, crystallization, recrystallization, chromatography or the like, or the reaction mixture may also be supplied as a raw material as it is.

Production Process B

Compound (I) can be produced by reacting the compound represented by the formula:

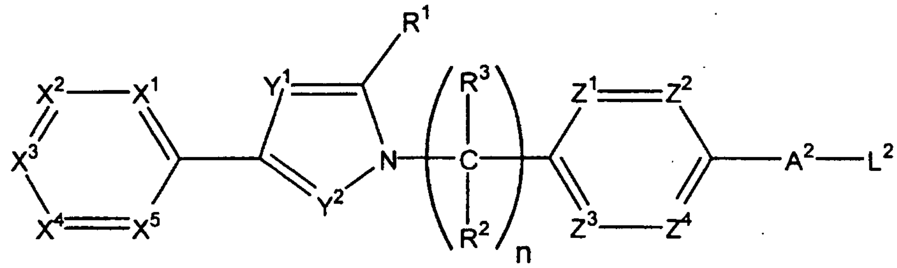

(IV)

wherein $A^2$ represents a single bond or $CR^jR^k$, $L^2$ represents a leaving group, and other symbols are as defined above, with a compound represented by the formula:

$$H\text{-}A^3\text{-}Ar\text{-}B \quad (V)$$

wherein $A^3$ represents O, $SO_m$ or $NR^l$, and other symbols are as defined above.

Examples of the leaving group represented by $L^2$ include the same leaving groups as those for $L^1$.

In the present reaction, the amount of the compound of the aforementioned formula (V) is not particularly limited, and the compound may be used as a solvent in a large excessive amount, but preferably the amount is about 0.8 to 5 equivalent.

Favorable results can occasionally be obtained by the presence of a base or action of a base before or after the reaction for the purpose of promoting the reaction and reducing side products. As such base, for example, alkali metal alcoholate such as sodium ethylate, sodium methylate, potassium tert-butoxide and the like, organic bases such as triethylamine, diisopropylethylamine, pyridine, 4-dimethylaminopyridine, *N,N*-dimethylaniline and the like, inorganic bases such as potassium carbonate, sodium carbonate, sodium hydroxide, potassium hydroxide, sodium bicarbonate, potassium bicarbonate and the like, metal hydrides such as lithium hydride, sodium hydride, potassium hydride and the like, and organic lithium reagents such as butyllithium, lithiumdiisopropylamide and the like can be used. The amount of a base to be used is not particularly limited as far as it does not adversely affect the reaction, and the base can be used in a large excessive amount also doubling as a solvent.

Favorable results can occasionally be obtained by the presence of a catalyst for the purpose of promoting the reaction and reducing side products. As such catalyst, for example, quaternary ammonium salts such as trioctylmethylammonium chloride, triethylbenzylammonium chloride, tetrabutylammonium chloride and the like, and crown ethers such as 18-crown-6, 15-crown-5 and the like can be used. The amount of a catalyst to be used is not particularly limited as far as it does not adversely affect the reaction, and the amount is preferably about 0.001 to 0.1 equivalent.

The present reaction can be carried out using a suitable solvent. The solvent is not particularly limited as far as it does not react with a reaction substrate, a reaction regent and a product to give side products, and the solvent which dissolves both reaction substrate and reaction regent is desirable. As such solvent, aliphatic hydrocarbons such as pentane, hexane, heptane, petroleum ether and the like, aromatic hydrocarbons such as benzene, toluene, xylene and the like, esters such as methyl acetate, ethyl acetate, ethyl formate, ethyl propionate and the like, ketones such as acetone, methyl ethyl ketone and the like, ethers such as diethyl ether, dipropyl ether, diisopropyl ether, dibutyl ether, tetrahydrofuran, dioxane and the like, nitriles such as acetonitrile, propionitrile and the like, acid amides such as dimethylformamide, dimethylacetamide and the like, sulfoxides such as dimethyl sulfoxide and the like, sulfones such as sulfolane and the like, phosphoric acid amides such as hexamethylphosphoramide and the like, halogenated hydrocarbons such as dichloromethane, chloroform, 1,2-dichloroethane, carbon tetrachloride and the like, aromatic amines such as pyridine, picoline, lutidine, quinoline and the like, and mixed solvents thereof, water, and mixed solvents thereof with water are used.

The reaction temperature is usually about -50 to 200°C, preferably about -30 to 150°C. The reaction time is generally about 0.1 to 96 hours, preferably 0.1 to 72 hours, more preferably about 0.1 to 24 hours.

The resulting compound may be supplied for the following reaction as a raw material after isolated or purified by the known means per se such as concentration, concentration under reduced pressure, liquid nature conversion, transference dissolution, solvent extraction, distillation, crystallization, recrystallization, chromatography or the like, or the reaction mixture may also be supplied as a raw material as it is.

Production Process C

Compound (I) can be produced by reacting the compound represented by the formula:

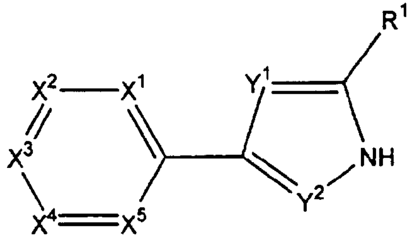

(VI)

wherein respective symbols are as defined above,
with the compound represented by the formula:

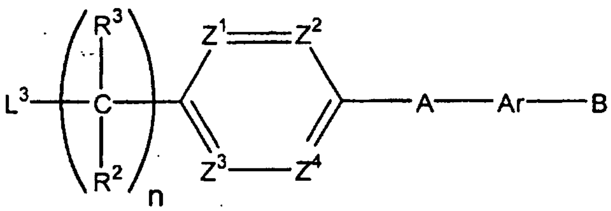

(VII)

wherein $L^3$ represents a leaving group, and other symbols are as defined above.

As a leaving group, the same groups as the leaving groups represented by $L^1$ are used.

In the present reaction, the amount of the compound of the aforementioned formula (VII) is not particularly limited, and the compound may be used as a solvent in a large excessive amount, but preferably the amount is about 0.8 to 5 equivalent.

Favorable results can occasionally be obtained by the presence of a base or action of a base before or after the reaction for the purpose of promoting the reaction and reducing side products. As such base, for example, alkali metal alcoholate such as sodium ethylate, sodium methylate, potassium tert-butoxide and the like, organic bases such as triethylamine, diisopropylethylamine, pyridine, 4-dimethylaminopyridine, *N,N*-dimethylaniline and the like, inorganic bases such as potassium carbonate, sodium carbonate, sodium hydroxide, potassium hydroxide, sodium bicarbonate, potassium bicarbonate and the like, metal hydrides such as lithium hydride, sodium hydride, potassium hydride and the like, and organic lithium reagents such as butyllithium, lithiumdiisopropylamide and the like can be used. The amount of a base to be used is not particularly limited as far as it does not adversely affect the reaction, and a base can be used in a large excessive amount also doubling as a solvent.

Further favorable results can occasionally be obtained by the presence of a catalyst for the purpose of promoting the reaction and reducing side products. As such catalyst, for example, quaternary ammonium salts such as trioctylmethylammonium chloride, triethylbenzylammonium chloride, tetrabutylammonium chloride and the like, and crown ethers such as 18-crown-6, 15-crown-5 and the like can be used. The amount of a catalyst to be used is not particularly limited as far as it does not adversely affect the reaction, and the amount is preferably about 0.001 to 0.1 equivalent.

The present reaction can be carried out using a suitable solvent. The solvent is not particularly limited as far as it does not react with a reaction substrate, a reaction regent and a product to give side products, and the solvent which dissolves both reaction substrate and reaction regent is desirable. As such solvent, aliphatic hydrocarbons such as pentane, hexane, heptane, petroleum ether and the like, aromatic hydrocarbons such as benzene, toluene, xylene and the like, esters such as methyl acetate, ethyl acetate, ethyl formate, ethyl propionate and the like, ketones such as acetone, methyl ethyl ketone and the like, ethers such as diethyl ether, dipropyl ether, diisopropyl ether, dibutyl ether, tetrahydrofuran, dioxane and the like, nitriles such as acetonitrile, propionitrile and the like, acid amides such as dimethylformamide, dimethylacetamide and the like, sulfoxides such as dimethyl sulfoxide and the like, sulfones such as sulfolane and the like, phosphoric acid amides such as hexamethylphosphoramide and the like, halogenated hydrocarbons such as dichloromethane, chloroform, 1,2-dichloroethane, carbon tetrachloride and the like, aromatic amines such as pyridine, picoline, lutidine, quinoline and the like, and mixed solvents thereof, water, and mixed solvents thereof with water are used.

The reaction temperature is usually about -50 to 200°C, preferably about -30 to 150°C. The reaction time is generally about 0.1 to 96 hours, preferably 0.1 to 72 hours, more preferably about 0.1 to 24 hours.

The resulting compound may be supplied for the following reaction as a raw material after isolated or purified by the known means per se such as concentration, concentration under reduced pressure, liquid nature conversion, transference dissolution, solvent extraction, distillation, crystallization, recrystallization, chromatography or the like, or the reaction mixture may also be supplied as a raw material as it is.

Production Process D

Compound (I) wherein $Y^1$ and $Y^2$ are both N can be produced by:

(1) reacting a compound represented by the formula:

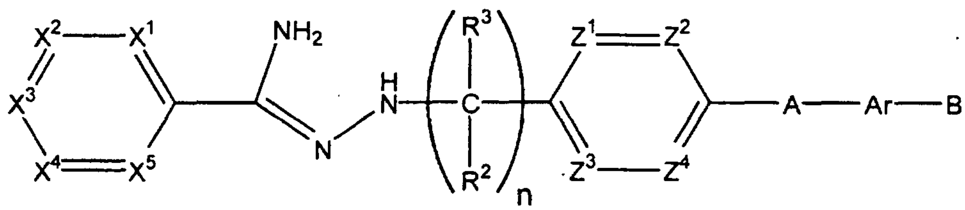

(VIII)

wherein the respective symbols as defined in above 1, with a compound represented by the formula:

$$R^1\text{-C(=O)-O-C(=O)-}R^1 \qquad \text{(IX)}$$

or the formula:

$$R^1\text{-C(=O)-}L^4 \qquad \text{(X)}$$

or

$$R^1C(L^{4'})_3 \qquad \text{(XI)}$$

wherein $L^4$ and $L^{4'}$ represent a leaving group, and $R^1$ is as defined above, or

(2) reacting the compound represented by the aforementioned formula (VIII) with a compound represented by the formula:

$$L^5\text{-C(=O)-}L^6 \qquad \text{(XII)}$$

wherein $L^5$ and $L^6$ represent a leaving group,
to obtain a compound represented by the formula:

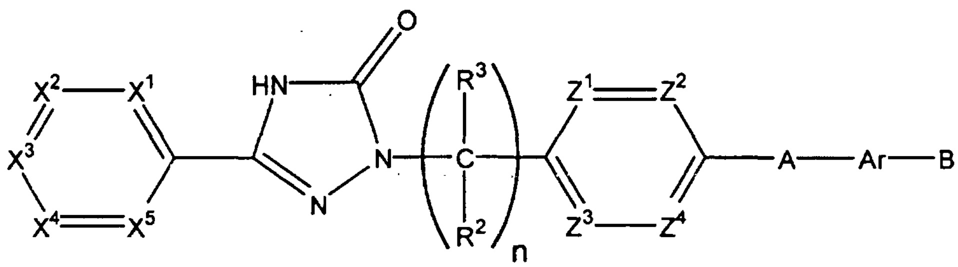

(XIII)

wherein respective symbols are as defined above,
and reacting this compound with a halogenating agent.

As each leaving groups, the same groups as those exemplified for the aforementioned $L^1$ and an alkoxy group, and a phenoxy group can be used.

In the present reaction, the amount of compounds of the formulas (IX) to (XII) is not particularly limited, and those compounds may be used as a solvent in a large excessive amount, but preferably the amount is about 0.8 to 5 equivalent.

Favorable results can occasionally be obtained by the presence of a base or action of a base before or after the reaction for the purpose of promoting the reaction and reducing side products. As such base, for example, alkali metal alcoholate such as sodium ethylate, sodium methylate, potassium tert-butoxide and the like, organic bases such as triethylamine, diisopropylethylamine, pyridine, 4-dimethylaminopyridine, N,N-dimethylaniline and the like, inorganic bases such as potassium carbonate, sodium carbonate, sodium hydroxide, potassium hydroxide, sodium bicarbonate, potassium bicarbonate and the like, metal hydrides such as lithium hydride, sodium hydride, potassium hydride and the like, and organic lithium reagents such as butyllithium, lithiumdiisopropylamide and the like can be used. The amount of a base to be used is not particularly limited as far as it does not adversely affect the reaction, and a base can be used in a large excessive amount also doubling as a solvent.

The present reaction can be carried out using a suitable solvent. The solvent is not particularly limited as far as it does not react with a reaction substrate, a reaction regent and a product to give side products, and the solvent which dissolves both reaction substrate and reaction regent is desirable. As such solvent, aliphatic hydrocarbons such as pentane, hexane, heptane, petroleum ether and the like, aromatic hydrocarbons such as benzene, toluene, xylene and the like, esters such as methyl acetate, ethyl acetate, ethyl formate, ethyl propionate and the like, ketones such as acetone, methyl ethyl ketone and the like, ethers such as diethyl ether, dipropyl ether, diisopropyl ether, dibutyl ether, tetrahydrofuran, dioxane and the like, nitriles such as acetonitrile, propionitrile and the like, acid amides such as dimethylformamide, dimethylacetamide and the like, sulfoxides such as dimethyl sulfoxide and the like, sulfones such as sulfolane and the like, phosphoric acid amides such as hexamethylphosphoramide and the like, halogenated hydrocarbons such as dichloromethane, chloroform, 1,2-dichloroethane, carbon tetrachloride and the like, aromatic amines such as pyridine, picoline, lutidine, quinoline and the like, and mixed solvents thereof, water, and mixed solvents thereof with water are used.

The reaction temperature is usually about -50 to 200°C, preferably about -30 to 150°C. The reaction time is generally about 0.1 to 96 hours, preferably 0.1 to 72 hours, more preferably about 0.1 to 24 hours.

Halogenation of the compound of the formula (XIII) can be carried out according to the known method per se, and examples of a halogenating agent to be used include thionyl chloride, sulfuryl chloride, phosphorus oxychloride, phosphorus pentachloride, hydrogen bromide, phosphorus oxybromide and the like. The amount of the halogenating agent to be used is not particularly limited, but preferably 0.3 to 5 equivalent.

The resulting compound may be supplied for the following reaction as a raw material after isolated or purified by the known means per se such as concentration, concentration under reduced pressure, liquid nature conversion, transference dissolution, solvent extraction, distillation, crystallization, recrystallization, chromatography or the like, or the reaction mixture may also be supplied as a raw material as it is.

Production Process E

Compound (I) wherein $Y^1$ is CH and $Y^2$ is N can be produced by:

(1) reacting the compound represented by the formula:

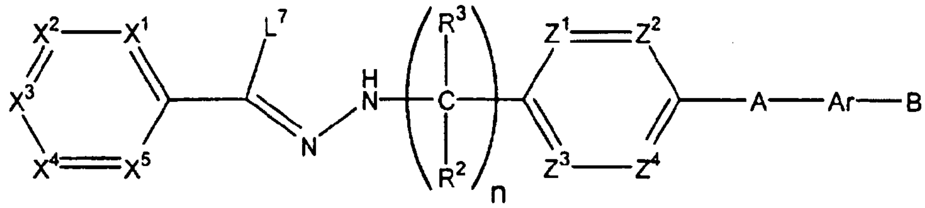

(XIV)

wherein $L^7$ represents a leaving group,
with a compound represented by the formula:

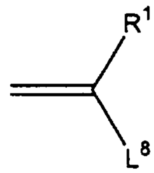

(XV)

wherein $L^8$ represents a leaving group, or
(2) reacting the compound represented by the aforementioned formula (XIV) with a compound represented by the formula:

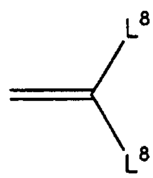

(XVI)

wherein $L^8$ is as defined above,
to obtain a compound represented by the formula:

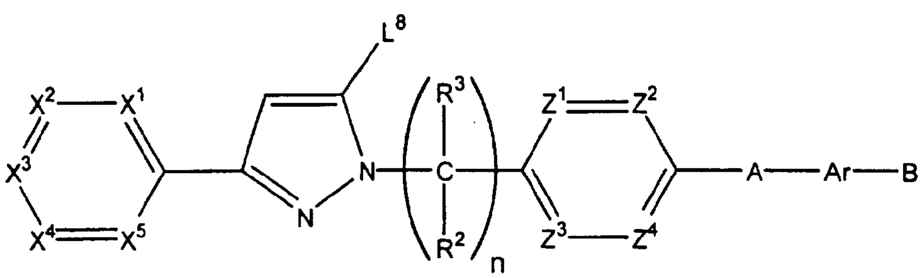

(XVII)

wherein $L^8$ is as defined above, and other symbols are as defined above,
and reacting this compound with a compound represented by the formula:

$$R^1\text{-H} \quad \text{(XVIII)}$$

wherein $R^1$ is as defined in the above 1.

As each leaving groups, the same groups as those exemplified for the aforementioned $L^1$ can be used.

In the present reaction, the amount of compounds of the formulas (XV) and (XVI) is not particularly limited, and the compounds may be used as a solvent in a large excessive amount, but the amount is preferably about 0.8 to 5 equivalent.

Favorable results can occasionally be obtained by the presence of a base or action of a base before or after the reaction for the purpose of promoting the reaction and reducing side products. As such base, for example, alkali metal alcoholate such as sodium ethylate, sodium methylate, potassium tert-butoxide and the like, organic bases such as triethylamine, diisopropylethylamine, pyridine, 4-dimethylaminopyridine, *N,N*-dimethylaniline and the like, inorganic bases such as potassium carbonate, sodium carbonate, sodium hydroxide, potassium hydroxide, sodium bicarbonate, potassium bicarbonate and the like, metal hydrides such as lithium hydride, sodium hydride, potassium hydride and the like, and organic lithium reagents such as butyllithium, lithiumdiisopropylamide and the like can be used. The amount of a base to be used is not particularly limited as far as it does not adversely affect the reaction, and a base can be used in a large excessive amount also doubling as a solvent.

Further favorable results can occasionally be obtained by the presence of a catalyst for the purpose of promoting the reaction and reducing side products. As such catalyst, for example, quaternary ammonium salts such as trioctylmethylammonium chloride, triethylbenzylammonium chloride, tetrabutylammonium chloride and the like, and crown ethers such as 18-crown-6, 15-crown-5 and the like can be used. The amount of a catalyst to be used is not particularly limited as far as it does not adversely affect the reaction, and the amount is preferably about 0.001 to 0.1 equivalent.

The present reaction can be carried out using a suitable solvent. The solvent is not particularly limited as far as it does not react with a reaction substrate, a reaction regent and a product to give side products, and the solvent which dissolves both reaction substrate and reaction regent is desirable. As such solvent, aliphatic hydrocarbons such as pentane, hexane, heptane, petroleum ether and the like, aromatic hydrocarbons such as benzene, toluene, xylene and the like, esters such as methyl acetate, ethyl acetate, ethyl formate, ethyl propionate and the like, ketones such as acetone, methyl ethyl ketone and the like, ethers such as diethyl ether, dipropyl ether, diisopropyl ether, dibutyl ether, tetrahydrofuran, dioxane and the like, nitriles such as acetonitrile, propionitrile and the like, acid amides such as dimethylformamide, dimethylacetamide and the like, sulfoxides such as dimethyl sulfoxide and the like, sulfones such as sulfolane and the like, phosphoric acid amides such as hexamethylphosphoramide and the like, halogenated hydrocarbons such as dichloromethane, chloroform, 1,2-dichloroethane, carbon tetrachloride and the like, aromatic amines such as pyridine, picoline, lutidine, quinoline and the like, and mixed solvents thereof, water, and mixed solvents thereof with water are used.

The reaction temperature is usually about -50 to 200°C, preferably about -30 to 150°C. The reaction time is generally about 0.1 to 96 hours, preferably 0.1 to 72 hours, more preferably about 0.1 to 24 hours.

The reaction of the compound of formula (XVII) and the compound of formula (XVIII) can be carried out according to the known method per se, and the amount of the compound of formula (VIII) to be used is not particularly limited, but is preferably 0.3 to 5 equivalent.

The resulting compound may be supplied for the following reaction as a raw material after isolated or purified by the known means per se such as concentration, concentration under reduced pressure, liquid nature conversion, transference dissolution, solvent extraction, distillation, crystallization, recrystallization, chromatography or the like, or the reaction mixture may also be supplied as a raw material as it is.

Production Process F

Compound (I) wherein $Y^1$ is N and $Y^2$ is N or CH can be produced by reacting the compound represented by the formula:

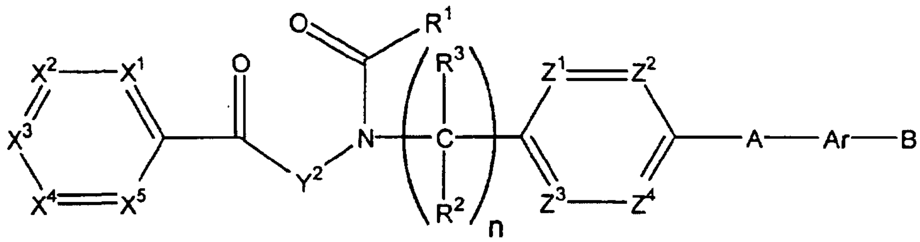

(XIX)

wherein respective symbols are as defined above, with a compound represented by the formula:

$$Acy\text{-}OHNH^3 \quad (XX)$$

wherein Acy represents an acyl group.

In the present reaction, the amount of the compound of the aforementioned formula (XX) is not particularly limited, and the compound may be used as a solvent in a large excessive amount, but the amount is preferably about 0.8 to 5 equivalent.

The present reaction can be carried out using a suitable solvent. The solvent is not particularly limited as far as it does not react with a reaction substrate, a reaction reagent and a product to give side products, and the solvent which dissolves both reaction substrate and reaction regent is desirable. As such solvent, for example, aliphatic hydrocarbons such as pentane, hexane, heptane, petroleum ether and the like, aromatic hydrocarbons such as benzene, toluene, xylene and the like, esters such as methyl acetate, ethyl acetate, ethyl formate, ethyl propionate and the like, ketones such as acetone, methyl ethyl ketone and the like, ethers such as diethyl ether, dipropyl ether, diisopropyl ether, dibutyl ether, tetrahydrofuran, dioxane and the like, nitriles such as acetonitrile, propionitrile and the like, acid amides such as dimethylformamide, dimethylacetamide and the like, sulfoxides such as dimethyl sulfoxide and the like, sulfones such as sulfolane and the like, phosphoric acid amides such as hexamethylphosphoramide and the like, halogenated hydrocarbons such as dichloromethane, chloroform, 1,2-dichloroethane, carbon tetrachloride and the like, aromatic amines such as pyridine, picoline, lutidine, quinoline and the like, and mixed solvents thereof, water, and mixed solvents thereof with water are used.

The reaction temperature is usually about -50 to 200°C, preferably about -30 to 150°C. The reaction time is generally about 0.1 to 96 hours, preferably 0.1 to 72 hours, more preferably about 0.1 to 24 hours.

The resulting compound may be supplied for the following reaction as a raw material after isolated or purified by the known means per se such as concentration, concentration under reduced pressure, liquid nature conversion, transference dissolution, solvent extraction, distillation, crystallization, recrystallization, chromatography or the like, or the reaction mixture may also be supplied as a raw material as it is.

Production Process G

Compound (I) wherein $Y^1$ is N and $Y^2$ is CH or N can be produced by reacting the compound represented by the formula:

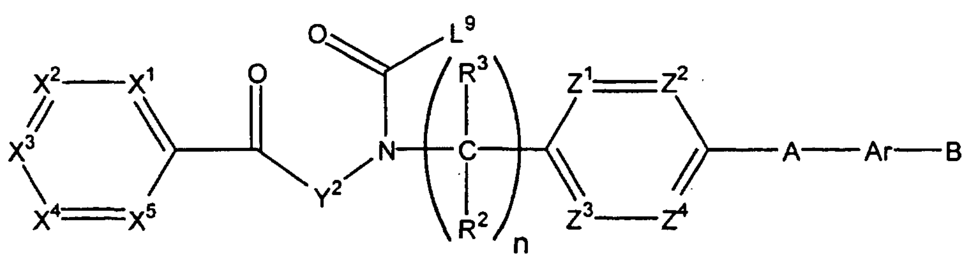

(XXI)

wherein $L^9$ represents a leaving group, with a compound represented by the formula:

Acy-$OHNH_3$ (XXII)

wherein Acy represents an acyl group,
to obtain a compound represented by the formula:

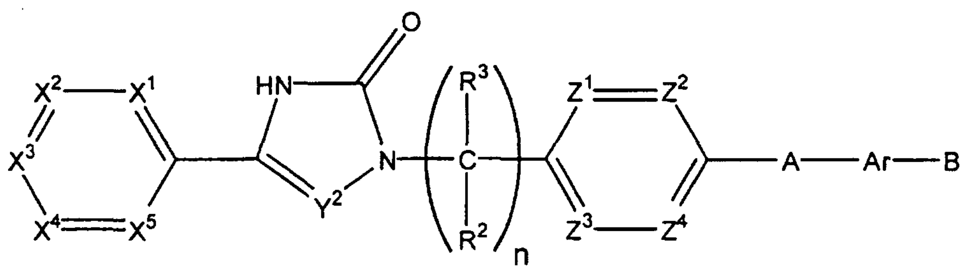

(XXIII)

wherein respective symbols are as defined above,
and reacting this compound with a halogenating agent.

As each leaving groups, the same groups as those exemplified for the aforementioned $L^1$ can be used.

In the present reaction, the amount of the compound of the formula (XXII) is not particularly limited, and the compound may be used as a solvent in a large excessive amount, but the amount is preferably about 0.8 to 5 equivalent.

The present reaction can be carried out using a suitable solvent. The solvent is not particularly limited as far as it does not react with a reaction substrate, a reaction reagent and a product to give side products, and the solvent which dissolves both reaction substrate and reaction regent is desirable. As such solvent, aliphatic hydrocarbons such as pentane, hexane, heptane, petroleum ether and the like, aromatic hydrocarbons such as benzene, toluene, xylene and the like, esters such as methyl acetate, ethyl acetate, ethyl formate, ethyl propionate and the like, ketones such as acetone, methyl ethyl ketone and the like, ethers such as diethyl ether, dipropyl ether, diisopropyl ether, dibutyl ether, tetrahydrofuran, dioxane and the like, nitriles such as acetonitrile, propionitrile and the like, acid amides such as dimethylformamide, dimethylacetamide and the like, sulfoxides such as dimethyl sulfoxide and the like, sulfones such as sulfolane and the like, phosphoric acid amides such as hexamethylphosphoramide and the like, halogenated hydrocarbons such as dichloromethane, chloroform, 1,2-dichloroethane, carbon tetrachloride and the like, aromatic amines such as pyridine, picoline, lutidine, quinoline and the like, and mixed solvents thereof, water, and mixed solvents thereof with water are used.

The reaction temperature is usually about -50 to 200°C, preferably about -30 to 150°C. The reaction time is generally about 0.1 to 96 hours, preferably 0.1 to 72 hours, more preferably about 0.1 to 24 hours.

Halogenation of the compound of formula (XXIII) can be carried out according to the known method per se and, as the halogenating agent, the same halogenating agent as that described above can be used, and the amount thereof to be used is not particularly limited, but is preferably 0.3 to 5 equivalent.

The resulting compound may be supplied for the following reaction as a raw material after isolated or purified by the known means per se such as concentration, concentration under reduced pressure, liquid nature conversion, transference dissolution, solvent extraction, distillation, crystallization, recrystallization, chromatography or the like, or the reaction mixture may also be supplied as a raw material as it is.

Production Process H

Compound of the formula (I) can be produced by reacting the compound represented by the formula:

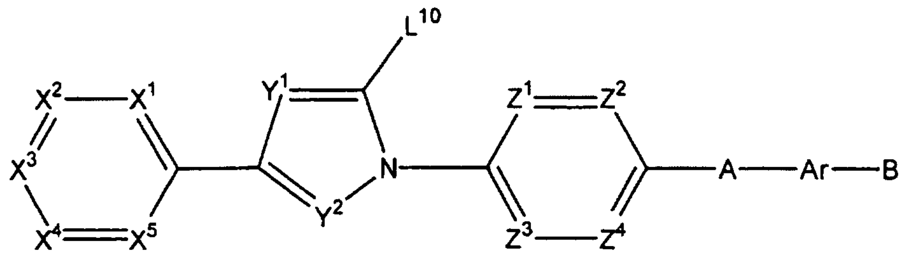

(XXIV)

wherein $L^{10}$ represents a leaving group and other symbols are as defined above, with a compound represented by the formula:

$$R^1\text{-H} \quad \text{(XVIII)}$$

wherein $R^1$ is as defined above.

The present reaction can be carried out according to the same manner as that of the reaction between the compound of formula (XVII) and the compound of formula (XVIII) in Production Process E.

Starting raw materials in the aforementioned Production Processes A to H are known, or can be prepared by a known method per se.

Compounds (I) and (XXV) of the present invention are effective for controlling a hygiene pest and an animal and plant parasitic pest, and exhibit a strong insecticidal activity by treating an animal and a plant which are parasitized by a pest. In addition, Compounds (I) and (XXIV) of the present invention have little phytotoxic effects on a plant, and have little toxicity to fishes and, thus, have both safe and advantageous nature as an agent for controlling pests for hygiene, the livestock industry, pets, horticulture and agriculture.

When Compound (I) or (XXV) is used as a pest controller, in particular, as an agricultural chemical such as an insecticide, the compound is used in a form which general agricultural chemicals and veterinary drugs can take, that is, a dosage form such as an emulsion, a solution, a microemulsion, a flowable formulation, an oil solution, a wettable powder, a powder, a granule, a fine granule, a seed coating agent, a smoking agent, a tablet, a microcapsule, a spray formulation, an EW agent, an ointment, a poison bait, a capsule, a pellet, an injectable, a shampoo preparation and the like, by dissolving or dispersing one kind or two kinds or more (preferably, one kind or more, and not more than three kinds) of Compound (I) or (XXV) or a salt thereof as an active ingredient in a suitable liquid carrier, or mixing with or being adsorbed on a suitable solid carrier depending on a use purpose. To these preparations, if needed, an emulsifying agent, a suspending agent, a developer, a penetrant, a wetting agent, a thickener, a stabilizer or the like may be added, and they can be prepared by a known method per se. As a liquid carrier (solvent) to be used, for example, solvents such as water, alcohols (e.g. methyl alcohol, ethyl alcohol, n-propyl alcohol, isopropyl alcohol, ethylene glycol etc.), ketones (e.g. acetone, methyl ethyl ketone, methyl isobutyl ketone, cyclohexanone etc.), ethers (e.g. tetrahydrofuran, ethylene glycol monomethyl ether, diethylene glycol monomethyl ether, propylene glycol monomethyl ether etc,), aliphatic hydrocarbons (e.g. kerosine, kerosene, fuel oil, machine oil etc.), aromatic hydrocarbons (e.g. toluene, xylene, solvent naphtha, methylnaphthalene etc.), halogenated hydrocarbons (e.g. dichloromethane, chloroform, carbon tetrachloride etc.), acid amides (e.g. *N,N*-dimethylformamide, *N,N*-dimethylacetamide, *N*-methylpyrrolidone etc.), esters (e.g. ethyl acetate, butyl acetate, fatty acid glycerin ester, γ-butyrolactone etc.), nitriles (e.g. acetonitrile, propionitrile etc.), vegetable oils (e.g. rape seed oil, cotton seed oil etc.), and the like are suitable. These can be appropriately used by mixing one kind or two kinds or more (preferably one kind or more, and not more than three kinds) at a suitable ratio.

As a solid carrier (diluent, bulking agent), a vegetable powder (e.g. soybean powder, tobacco powder, wheat powder, woodmeal etc.), a mineral powder (e.g. clays such as kaolin, bentonite, acid clay etc., talcs such as talc powder, agalmatolite powder etc., silicas such as diatomaceous earth, mick powder etc.), alumina, a sulfur powder, an active carbon, calcium carbonate, potassium chloride, ammonium sulfate, sodium hydrogen carbonate, lactose, urea and the like are used, and these can be appropriately used by mixing one kind or two kinds or more (preferably one kind or more, and not more than three kinds) at a suitable ratio.

In addition, as an ointment base materials, for example, one kind or two kinds or more (preferably, one kind or more, and not more than three kinds) of materials selected from the group consisting of polyethylene glycol, pectin, polyhydric alcohol ester of higher fatty acid such as monostearic acid glycerin ester and the like, cellulose derivative

such as methylcellulose and the like, sodium alginate, bentonite, higher alcohol, polyhydric alcohol such as glycerin and the like, vaseline, white vaseline, liquid paraffin, lard, various vegetable oils, lanolin, dehydrated lanolin, hardened oil, resins and the like, or these materials wherein following various surfactants are added thereto are appropriately used.

As a surfactant used as an emulsifying agent, a developer, a penetrant, a dispersant and the like, depending on the necessity, nonionic and anionic surfactants such as soaps, polyoxyethylene alkyl aryl ethers [e.g. Neugen (trade name) , E · A142 (trade name); manufactured by Dai-ichi Kogyo Seiyaku Co., Ltd., Nonal (trade name); manufactured by Toho Chemical Industries Co., Ltd.], alkyl sulfate salts [e.g. Emar 10 (trade name), Emar 40 (trade name); manufactured by Kao Corporation], alkylbenzene sulfonic acid salts [e.g. Neogen (trade name), Neogen T(trade name); manufactured by Dai-ichi Kogyo Seiyaku Co., Ltd., Neoperex ; manufactured by Kao Corporation], polyoxyethylene alkyl ethers [e.g. Neugen ET-135 (trade name); manufactured by Dai-ichi Kogyo Seiyaku Co., Ltd.], polyoxyethylene polyoxypropylene block polymers [e.g. Newpol PE-64 (trade name); manufactured by Sanyo Chemical Industries, Ltd.], polyhydric alcohol esters [e.g. Tween 20 (trade name), Tween 80 (trade name); manufactured by Kao Corporation], alkylsulfosuccinic acid salts [e.g. Sanmolin OT20 (trade name); manufactured by Sanyo Chemical Industries, Ltd., Newcalgen EX70 (trade name); manufactured by Takemoto Oil & Fat Co., Ltd.], alkylnaphthalene sulfonic acid salts [e.g. Newcalgen EX70 (trade name); manufactured by Takemoto Oil & Fat Co., Ltd.], alkenyl sulfonic acid salts [e.g. Solpol 5115 (trade name); manufactured by Toho Chemical Industries Co., Ltd.] and the like are appropriately used, and these can be appropriately used by mixing one kind or two kinds or more (preferably, one kind or more, and not more than three kinds) at a suitable ratio. Alternatively, Compound (I) or (XXIV) can also be used appropriately by compounding with, for example, other insecticide (pyrethroid insecticide, organic phosphorus insecticide, carbamate insecticide, neonicotinoid insecticide, natural insecticide etc.), an acaricide, a machine oil, a nematodecide, a herbicide, a plant hormone agent, a plant growth regulating substance, an antibacterial agent (e.g. copper antibacterial agent, organic chlorine antibacterial agent, organic sulfur antibacterial agent, phenol antibacterial agent etc.), a synergist, an attractant, a repellent, a drug harm alleviating agent, a pigment, a fertilizer, an animal feed (feed for livestock such as cow pig and hence chicken, feed for pet animal such as dog and cat, feed for raised fish such as young yellowtail and sea bream), veterinary medicaments (medicaments for treating or preventing diseases of livestock, pet animal, raised fish), a veterinary nutrient and the like.

The ratio of Compound (I) or (XXV) contained in the agricultural chemical and veterinary drug composition (insecticide and anthelmintic) of the present invention is usually about 0.1 to 80% by weight, preferably about 1 to 20% by weight relative to the total amount of the composition. Specifically, when the compound is used as an emulsion, a solution or a wettable powder (e.g. granular wettable powder), usually about 1 to 80% by weight, preferably about 1 to 20% by weight is suitable. When used as an oil solution or a powder, usually about 0.1 to 50% by weight, preferably about 0.1 to 20% by weight is suitable. When used in a granule, usually about 5 to 50% by weight, preferably about 1 to 20% by weight is suitable.

Other agricultural chemical active ingredient (e.g. an insecticide, a herbicide, an acaricide and/or an antibacterial agent) which is compounded in the agricultural chemical composition of the present invention is used usually in the range of about 1 to 80% by weight, preferably about 1 to 20% by weight relative to the total amount of the preparation.

The content of an additive other than the aforementioned active ingredients differs depending on a kind or a content of an agricultural chemical active ingredient or a dosage form of a preparation, and is usually about 0.001 to 99.9% by weight, preferably about 1 to 99% by weight. More specifically, it is preferable to add a surfactant at usually about 1 to 20% by weight, more preferably about 1 to 15% by weight, a flowing aid at about 1 to 20% by weight, and a carrier at about 1 to 90% by weight, preferably at about 1 to 70% by weight relative to the total amount of the composition. Specifically, when a solution is prepared, it is preferable to add a surfactant at usually about 1 to 20% by weight, preferably 1 to 10% by weight, and water at about 20 to 90% by weight. An emulsion or a wettable powder (e.g. granular wettable powder) should be diluted with water appropriately (e.g. about 100 to 5,000-fold) for use to spray.

Typical examples of the compound (including isomers and salts thereof) which can be used by mixing with the Compound (I), (XXV) or salts thereof of the present invention are shown below.

A insecticide, an acaricide and a nematodecide: acephate, acequinocyl, acetamiprid, acetoprole, acrinathrin, alanycarb, aldrin, allethrin, Aluminium phosphide, amidoflumet, amitraz, Arsenic acid, avermectin-B, bendiocarb, benfluthrin, benfuracarb, bensultap, benzoximate, bifenthrin, bistrifluron, BPMC, bromopropylate, buprofezin, butathiofos, cadusafos, Calcium cyanamide, Calcium polysulfide, carbaryl:NAC, carbofuran, carbosulfan, cartap, chlordane, chlorethoxyfos, chlorfenvinphos:CVP, chlorfluazuron, chlorphenapyr, chlorpyrifos, chlorpyrifos-methyl, chromafenozide, cloethocarb, clofentezine, clothianidin, cyanophos:CYAP, cycloprothrin, cyfluthrin, cyhalothrin, cypermethrin, cyromazine, dichlorodiisopropyl ether, 1,3-Dichloropropene, DDT, deltamethrin, diafenthiuron, diazinon, dichlofenthion, dichlorvos:DDVP, dicofol, dieldrin, dienochlor, diflubenzuron, dimethoate, dimethylvinphos, dinotefuran, disulfoton, DSP, emamectin-benzoate, endosulfan, EPN, esfenvalerate, ethiofencarb, ethion, ethiprole, ethofenprox, ethoprophos, etoxazole, etrimfos, fenazaquin, fenbutatin oxide, fenitrothion:MEP, fenobucarb, fenothiocarb, fenoxycarb, fenpropathrin, fenpyroximate, fenthion, fenvalerate, fipronil, fluacrypyrim, fluazinam, fluazuron, flucycloxuron, flucythrinate,

flufenerim, flufenoprox, flufenoxuron, flumethrin, flonicamid, fluproxyfen, flupyrazofos, flurimfen, fluvalinate, formetanate, formothion, fosthiazate, furathiocarb, halfenprox, hexaflumuron, hexythiazox, Hydrogen phosphide, hydroprene, imidacloprid, imiprothrin, indoxacarb, isofenphos, isoprocarb, isoxathion, lufenuron, levamisol, machine oil, malathion, mesulfenfos, metam-ammonium, metam-sodium, methidathion, methiocarb, methomyl, methoxychlor, methoxyfenozide, methyl bromide, metofluthrin, metolcarb:MTMC, metoxadiazone, milbemycin-A, monocrotophos, naled:BRP, nicotine-sulfate, novaluron, noviflumuron, nidinotefuran, nitenpyram, oxamyl, oxydeprofos:ESP, parathion, permethrin, phenthoate:PAP, phosalone, phosmet:PMP, pirimicarb, pirimiphos-methyl, Potassium oleate, prallethrin, profenofos, propaphos, propargite:BPPS, propoxur, prothiofos, protrifenbute, pymetrozine, pyraclofos, pyrethrins, pyridaben, pyridafenthion, pyridalyl, pyrimidifen, pyriproxyfen, quinalphos, resmethrin, salithion, silafluofen, spinosad, spirodiclofen, sulfur, sulfluramid, sulprofos, tebufenozide, tebufenpyrad, tebupirimfos, teflubenzuron, tefluthrin, temephos, tetrachlorvinphos, tetradifon, thiacloprid, thiamethoxam, thiocyclam, thiodicarb, thiometon, TI-809, tolfenpyrad, tralomethrin, triazamate, trichlorfon:DEP, triflumuron, vamidothion, vaniliprole, XMC, xylylcarb

An antibacterial agent: acibenzolar-S-methyl, amobam, ampropylfos, anilazine, azoxystrobin, benalaxyl, benodanil, benomyl, benthiavalicarb, benthiazole, bethoxazin, bitertanol, blasticidin-S, Bordeaux mixture, bromuconazole, buthiobate, calcium hypochlorite, calcium polysulfide, captan, carbendazol, carboxin, carpropamid, chlobenthiazone, chloroneb, chloropicrin, chlorothalonil:TPN, chlorthiophos, cinnamaldehyde, clozylacon, CNA (2,6- d ichloro-4-nitroaniline), copper hydroxide, copper sulfate, cyazofamid, cyfluphenamid, cymoxanil, cyproconazole, cyprodinil, cyprofuram, dazomet, debacarb, dichlofluanid, D-D (1,3-Dichloropropene), diclocymet, diclomezine, diethofencarb, difenoconazole, diflumetorim, dimefluazole, dimethirimol, dimethomorph, diniconazole-M, dinocap, edifenphos, epoxiconazole, nickel dimethyl dithiocarbamate, etaconazole, ethaboxam, ethirimol, etridiazole, famoxadone, fenamidone, fenarimol, fenbuconazole, Fendazosulam, fenhexamid, fenoxanil, fenpiclonil, fenpropidin, fenpropimorph, fentiazon, fentin hydroxide, ferimzone, fluazinam, fludioxonil, flumetover, fluoroimide, fluotrimazole, fluquinconazole, flusilazole, flusulfamide, flutolanil, flutriafol, fosetyl-Al, fthalide, fuberidazole, furalaxyl, furametpyr, furcarbanil, furconazole-cis, hexaconazole, hymexazol, IBP, imazalil, imibenconazole, iminoctadine-albesilate, iminoctadine-triacetate, iodocarb, ipconazole, iprodione, iprovalicarb, isoprothiolane, kasugamycin, kresoxim-methyl, mancozeb, maneb, mepanipyrim, mepronil, metalaxyl, metalaxyl-M, metam-sodium, methasulfocarb, methyl bromide, metconazole, methfuroxam, metominostrobin, metrafenone, metsulfovax, mildiomycin, milneb, myclobutanil, myclozolin, nabam, nicobifen, orysastrobin, ofurace, oxadixyl, oxolinic acid, oxpoconazole, oxycarboxin, oxytetracycline, pefurazoate, penconazole, pencycuron, picoxystrobin, polycarbamate, polyoxin, potassium hydrogen carbonate, probenazole, prochloraz, procymidone, propamocarb-hydrochloride, propiconaole, propineb, prothiocarb, pyracarbolid, pyraclostrobin, pyrazophos, pyributicarb, pyrifenox, pyrimethanil, pyroquilon, quinoxyfen, quintozene:PCNB, silthiopham, simeconazole, sipconazole, sodium bicarbonate, sodium hypochlorite, spiroxamine, SSF-129 ((E)-2[2-(2,5-dimethylphenoxymethyl)phenyl]-2-methoxyimino-N-methylacetamide), streptomycin, Sulfur, tebuconazole, tecloftalam, tetraconazole, thiabendazole, thiadinil, thiram:TMTD, thifluzamide, thiophanate-methyl, tolclofos-methyl, TPN, triadimefon, triadimenol, triazoxide, triclamide, tricyclazole, tridemorph, triflumizole, trifloxystrobin, triforine, triticonazole, validamycin, vinclozolin, viniconazole, zineb, ziram, zoxamide

A herbicide, a plant hormone agent, a plant growth regulating substance: abscisic acid, acetochlor, acifluorfen-sodium, alachlor, alloxydim, ametryn, amicarbazone, amidosulfuron, aminoethoxyvinylglycine, AC94,377, amiprofos-methyl, ancymidol, asulam, atrazine, azimsulfuron, beflubutamid, benfluralin, benfuresate, bensulfuron-methyl, bensulide:SAP, bentazone, benthiocarb, benzamizole, benzfendizone, benzobicyclon, benzofenap, benzyl adenine, benzylaminopurine, bialaphos, bifenox, Brassinolide, bromacil, bromobutide, butachlor, butafenacil, butamifos, butylate, cafenstrole, calcium carbonate, calcium peroxide, carbaryl, chlomethoxynil, chloridazon, chlorimuron-ethyl, chlorphthlim, chlorpropham, chlorsulfuron, chlorthal-dimethyl, chlorthiamid:DCBN, choline chloride, cinidon-ethyl, cinmethylin, cinosulfuron, clethodim, clomeprop, cloxyfonac-sodium, chlormequat chloride, 4-CPA (4-chlorophenoxyacetic acid), cliprop, clofencet, cumyluron, cyanazine, cyclanilide, cyclosulfamron, cyhalofop-butyl, 2,4-D salts (2,4-dichlorophenoxyacetic acid salts), dichlorprop:2,4-DP, daimuron, dalapon:DPA, dimethenamid-P, daminozide, dazomet, n-Decyl alcohol, dicamba-sodium:MDBA, dichlobenil:DBN, diflufenican, dikegulac, dimepiperate, dimethametryn, dimethenamid, diquat, dithiopyr, diuron, endothal, epocholeone, esprocarb, ethephon, ethidimuron, ethoxysulfuron, ethychlozate, etobenzanid, fenarimol, fenoxaprop-ethyl, fentrazamide, flazasulfuron, florasulam, fluazifop-butyl, fluazolate, flucarbazone, flufenacet, flufenpyr, flumetralin, flumioxazin, flupropanate-sodium, flupyrsulfuron-methyl-sodium, flurprimidol, fluthiacet-methyl, foramsulfuron, forchlorfenuron, formesafen, gibberellin, glufosinate, glyphosate, halosulfuron-methyl, hexazinone, imazamox, imazapic, imazapyr, imazaquin, imazosulfuron, inabenfide, Indole acetic acid:IAA, Indole butyric acid, iodosulfuron, ioxynil-octanoate, isouron, isoxachlortole, isoxadifen, karbutilate, lactofen, lenacil, linuron, Maleic hydrazide, mecoprop:MCPP, MCP salts (2-Methyl-4-chlorophenoxyacetic acid salts), MCPA-thioethyl, MCPB (2-Methyl-4-chlorophenoxybutanoic acid ethyl ester), mefenacet, mefluidide, mepiquat, mesosulfuron, mesotrione, methyl daimuron, metamifop, metolachlor, metribuzin, metsulfuron-methyl, molinate, naphthylacetic acid, NAD (1-naphthaleneacetamide, naproanilide, napropamide, n-decyl alcohol, nicosulfuron, n-phenylphthalamic acid, orbencarb, oxadiazon, oxaziclomefone, oxine-sulfate, paclobutrazol, paraquat, Pelargonic acid, pendimethalin, penoxsulam, pentoxazone, pethoxamide, phenmedipham, picloram, picolinafen, piperonyl butoxide, piperophos, pretilachlor, primisulfuron-methyl, procarbazone, prodiamine, profluazol, profoxydim, prohexadione-calcium, prohydrojasmon, prometryn, propanil, propoxycarbazone, propyzamide, pyraclonil, pyraflufen-ethyl, pyrazolate, pyrazosulfuron-ethyl, pyrazoxyfen, pyribenzoxim, pyributicarb, pyridafol, pyridate, pyriftalid, pyriminobac-methyl, pyrithiobac, quiclorac, quinoclamine, quizalofop-ethyl, rimsulfuron, sethoxydim, siduron, simazine, simetryn, Sodium chlorate, sulfosulfuron, swep:MCC, tebuthiuron, tepraloxydim, terbacil, terbucarb:MBPMC, thenylchlor, thiazafluron, thidiazuron, thifensulfuron-methyl, triaziflam, tribufos, triclopyr, tridiphane, trifloxysulfuron, trifluralin, trinexapac-ethyl, tritosulfuron, uniconazole-P, vemolate:PPTC

In addition, the Compound (I), (XXV) or salts thereof of the present invention may be used by mixture with a synergist such as piperonyl butoxide, sesamex, sulfoxide, MGK 264, N-declyimidazole, WARF-antiresistant, TBPT, TPP, IBP, PSCP, $CH_3I$, t-phenylbutenone, diethylmaleate, DMC, FDMC, ETP, ETN and the like, and also may be used by mixture with a drug harm alleviating agent such as benoxacor, cloquintocet-mexyl, cyometrinil, daimuron, dichlormid, fenchlorazole-ethyl, fenclorim, flurazole, fluxofenim, furilazole, mefenpyr-diethyl MG191, naphthalic anhydride, oxabetrinil and the like.

Furthermore, the Compound (I), (XXV) or salts thereof of the present invention may be used by mixture with a controlling agent for an outside-parasitic arthropod such as pyrethroid agents and IGR agents (juvenile hormone-like substances such as methoprene, fenoxycarb and the like, chitin synthase inhibitors such as lufenuron, flufenoxuron, novaluron, hexaflumuron, teflubenzuron, diflubenzuron, triflumuron and the like, or insect growth regulating agents such as cyromazine, pyriproxyfen and the like), or may be used by mixture with a controlling agent for inner parasite such as an above-mentioned IGR agent as in vivo administration agent for animal, a controlling agent for filaria (macrolide compounds such as selamectin, ivermectin, milbemycin, moxidectin etc.) or the like, and further may be used by mixture with an antibacterial agent for animal, vaccine, therapeutic agent, nutritional supplement and bait.

The preparation containing Compound (I) of the present invention or a salt thereof is particularly effective for controlling pests, specifically, such as Hemiptera such as *Eurydema rugosum, Scotinophara lurida, Riptortus clavatus, Stephanitis nashi, Laodelphax striatellus, Nilaparvata lugens, Nephotettix cincticeps, Unaspis yanonensis, Aphis glycines, Lipaphis erysimi, Brevicoryne brassicae, Aphis gossypii, Myzus persicae, Aulacorthum solani, Aphis spiraecola, Bemisia tabaci, Trialeurodes vaporariorum, Sogatella furcifera, Empoasca onukii, Pseudococus comstocki, Planococcus citri, Icerya purchasi, Plautia stali, Eysarcoris parvus* and the like; Lepidoptera such as *Spodoptera litura, Plutella xylostella, Pieris rapae crucivora, Chilo suppressalis, Autographa nigrisigna, Helicoverpa assulta, Pseudaletia separata, Mamestra brassicae, Adoxophyes orana fasciata, Notarcha derogata, Cnaphalocrocis medinalis, Phthorimaea operculella, Chilo polychrysus, Typoryza incertulas, Spodoptera exigua, Agrotis segetum, Agrotis ipsilon, Heliothis armigera, Heliothis virescens, Heliothis zea, Naranga aenescens, Ostrinia nubilalis, Ostrinia furnacalis, Parnara guttata, Adoxophyes sp., Caloptilia theivora, Phyllonorycter ringoneella, Carposina niponensis, Grapholita molestaCydia pomonella* and the like;

Coleoptera such as *Epilachna vigintioctopun c tata, Aulacophorafemoralis, Phyllotreta striolata, Oulema oryzae, Echinocnemus squameus, Lissorhoptrus oryzophilus, Anthonomus grandis, Callosobruchus chinensis, Sphenophorus venatus, Popillia japonica, Anomala cuprea, Diabrotica spp., Leptinotarsa decemlineata, Agriotes spp., Lasioderma serricorne, Anthrenus verbasci, Tribolium castaneum, Lyctus brunneus, Anoplophora malasiaca, Tomicus piniperda* and the like;

Diptera such as *Musca domestica, Culex popiens pallens, Tabanus trigonus, Delia antiqua*, *Delia platura, Anopheles sinensis, Agromyza oryzae, Hydrellia griseola, Chlorops oryzae, Dacus cucurbitae, Ceratitis capitata, Liriomyza trifolii* and the like;

Orthoptera such as *Locusta migratoria, Gryllotalpa africana, Oxya yezoensis, Oxya japonica* and the like;

Thysanoptera such as *Thrips tabaci, Thrips parmi, Frankliniella occidentalis, Baliothrips biformis, Scirtothrips dorsalis* and the like;

Hymenoptera such as *Athalia rosae, Acromyrmex spp., Solenopsis spp.* and the like;

Blattodea such as *Blattella germanica, Periplaneta fuliginosa, Periplaneta japonica, Periplaneta americana* and the like;

Acarina such as, Tetranychidae such as *Tetranychus urticae, Panonychus citri, Tetranychus kanzawai, Tetranychus cinnabarinus, Tetranychus viennensis, Tetranychus desertorum, Panonychus ulmi* and the like, Eriophyidae such as *Aculops pelekassi, Aculops lycopersici, Aceria diospyri, Aculus fockeui, Eriophyes chibaensis* and the like, Tarsonemidae such as *Polyphagotarsonemus* latus, *Phytonemus pallidus* and the like, Acaridae such as *Rhizoglyphus echinopus* and the like, Tenuipalpidae, Eupodidae and the like;

Nematoda such as *Aphelenchoides besseyi, Meloidogyne incognita, Pratylenchus penetrans, Nothotylenchus acris* and the like;

Isoptera such as *Coptotermes formosanus, Reticulitermes speratus, Odontotermes formosanus, Cryptotermes domesticus* and the like.

Furthermore, the preparation containing Compound (I) of the present invention or a salt thereof can be used in the field of treatment for disease of livestock and in livestock farming, and also for maintaining public sanitation by

exterminating an arthropod and parasite which parasitize inside and/or outside of vertebrates such as human, cow, sheep, goat, pig, poultry, dog, cat, fish and the like. Examples of pests include, for example, Ixodes spp., Boophilus spp. (e.g. *Boophilus microplus*), Amblyomma spp., Hyalomma spp., Rhipicephalus spp. (e.g. *Rhipicephalus appendiculatus*), Haemaphysalis spp., dermacentor spp., Ornithodoros spp. (e.g. *Ornithodoros moubata*), *Dermahyssus gallinae*, Sarcoptes spp. (e.g. *Sarcoptes scabiei),* Psoroptes spp., Chorioptes spp., Demodex spp., Eutrombicula spp., Aedes spp., Anopheles spp., Culex spp, Culicodes spp, Musca spp., Hypoderma spp., Gasterophilus spp., Haematobia spp, Tabanus spp, Simulium spp., Triatoma spp., Phthiraptera (e.g. Damalinia spp., Linognathus spp., Haematopinus spp), Ctenocephalides spp.Xenosylla spp), monomorium pharaonis and Nematoda (for example, Trichostrongylus (e. g. *Nippostrongylus brasiliensis, Trichostrongylus axei*, *Trichostrongylus colubriformis*), Trichinella (e.g. *Trichinella spiralis*), *Haemonchus contortus,* Nematodirus (e.g. *Nematodirus battus*), *Ostertagia circumcincta*, Cooperia spp. , Hymenolepis nana) and the like.

The agricultural chemical composition containing the Compound (I) of the present invention or a salt thereof has a superior pesticidal activity and can be used as an extremely lower toxic, therefore safe and excellent agricultural chemical composition (pesticide). And the agricultural chemical composition of the present invention can be used in a manner similar to a conventional agricultural chemical composition and consequently can yield very substantial benefits compared to existing lines. The arising above-mentioned pests (insect pests etc.) can be exterminated by spraying the agricultural chemical composition of the present invention on paddy, field, orchard, non-tillage farm, house and the like by a known method per se and bringing the pests into contact with the composition or feeding the pests. As an alternative mode, for example, the arthropod and parasite living with said vertebrates can be exterminated by administrating the agricultural chemical composition of the present invention to inside (internal parts of the body) or outside (body surface) of the above-mentioned vertebrates. In addition, sanitary pests arising from the excrement of the animal can be exterminated by feeding a livestock animal.

More specifically, for example, the agricultural chemical composition of the present invention can be used to the targeting pests by a method such as treatment to seed, treatment to nursery box, treatment to planting holes, soil-mixing treatment, spraying to stalks and leaves, ULV spray, immersion treatment, application, dust coating, fertilizer-mixing treatment, injection to tree trunk, poison bait, smoking, affusion, subaqueous application for paddy and the like. The amount of application can be changed within a wide range depending on the application time, application place, application method and the like, and it is desirable to apply so that the active ingredient (Compound (I) or a salt thereof) per hectare comes generally to about 0.3 g to 3,000 g, preferably about 50 g to 1,000 g. When the agricultural chemical composition of the present invention is a wettable powder, it may be diluted with water to use so that the final concentration of active ingredient comes to the range of about 0.1 to 1,000 ppm, preferably about 10 to 500 ppm. Furthermore, recently advances have been made in the technologies of genetically-modified crops (a herbicide resistant crop, a pest resistant crop integrated a gene producing pest-control proteins, a disease resistant crop integrated a gene producing substances resistant to diseases, a flavor-improved crop, a storage stability-improved crop, a yield-improved crop, etc.), insect pheromone (a disrupting agent of communication of Tortricidae, Mamestra brassicae, etc.) and IPM (integrated pests management) using a counterpest insect. The agricultural chemical composition of the present invention can be used together with these technologies or can be used by systematizing with them.

## Examples

The present invention will be further illustrated by the following Reference Examples, Production Examples, Formulation Examples and Test Examples; however, the present invention is not limited to these examples.

The elution in the column chromatography for Production Examples was carried out under the observation by TLC (Thin Layer Chromatography). In the TLC observation, kieselgel $60F_{254}$ (70 to 230 meshes) manufactured by Merck & Co., Inc. was used as TLC plate; the solvent used as an elution solvent in column chromatography was used as developing solvent; and a UV detector was used for detection. Kieselgel 60 (70 to 230 meshes) manufactured by Merck & Co., Inc. was used as silica gel for column chromatography. NMR spectra were proton NMR, and were determined with Bruker AC-200P (200 MHz) spectrometers using tetramethylsilane as internal standard. All delta values were shown in ppm. When a mixed solvent was used as developing solvent, the numeric value in parentheses shows a mixing ratio of solvents by volume. The abbreviations used in the following Examples, Reference Examples and tables have the following meanings. Me: methyl group, Et: ethyl group, Ph: phenyl group, Pr-n (or n-Pr): n-propyl, Pr-i (or i-Pr, or $^{i}$Pr): isopropyl, Bu-n (or n-Bu): n-butyl, Bu-i (or i-Bu): isobutyl, Bu-s (or s-Bu): sec-butyl, Bu-t (or t-Bu): tert-butyl, s: singlet, br: broad, brs: broad singlet, d: doublet, t: triplet, q: quartet, qu: quintet, sep: septet, m: multiplet, dd: double doublet, dt: double triplet, J: coupling constant, Hz: herz, %: % by weight, mp: melting point, and room temperature means the temperature of about 15 to 25°C.

Reference Example 1

4-[3-(2-chloro-6-fluorophenyl)-5-methyl-1*H*-1,2,4-triazol-1-yl]phenylmethanol (Compound 1)

0.19 g (5.0 mmol) of lithium aluminum hydride was suspended in 15 ml of THF, and 0.45 g (1.3 mmol) of ethyl 4-[3-(2-chloro-6-fluorophenyl)-5-methyl-1H-1,2,4-triazol-1-yl]benzoate dissolved in 15 ml of THF was added dropwise thereto under ice-cooling. After stirring for 15 mins. under ice-cooling, ethyl acetate was added thereto, followed by adding 4.4 ml of 20% aqueous solution of sodium hydroxide. The resulting solution was filtered through Celite, and the residue obtaind by concentrating the filtrate under reduced pressure was washed with hexane to give 0.37 g (1.2 mmol, 93%) of 4-[3-(2-chloro-6-fluorophenyl)-5-methyl-1H-1,2,4-triazol-1-yl]phenylmethanol as a white crystal.
mp: 120-122°C
$^{1}H$ NMR ($CDCl_3$) $\delta$ ppm: 2. 63 (3H, s), 4.79 (2H, s), 7.06-7.15 (1H, m), 7.29-7.41 (2H, m), 7.53 (4H, s)
$^{19}F$ NMR ($CDCl_3$) $\delta$ ppm: -110.50 - -110.58 (m) IR (nujol) $\nu$ $cm^{-1}$: 1612, 1571, 1528, 1509,1401,1378, 1352, 1252, 1048, 1039, 895

The compounds shown in the following tables 1-3 were prepared according to the same manner as that of Reference Example 1.

Table 1

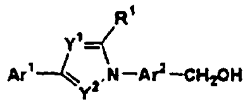

| No. | $Ar^1$ | $Y^1$ | $Y^2$ | $R^1$ | $Ar^2$ | mp. |
|---|---|---|---|---|---|---|
| 1 | 2-Cl-6-F-$C_6H_3$ | N | N | $CH_3$ | 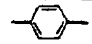 | 120-122 |
| 2 | 2-Cl-6-F-$C_6H_3$ | N | N | Et | 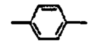 | 110-111 |
| 3 | 2-Cl-6-F-$C_6H_3$ | N | N | *i*-Pr | 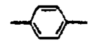 | 138-140 |
| 4 | 2-Cl-6-F-$C_6H_3$ | N | N | *c*-Pr | 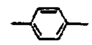 | 129-141 |
| 5 | 2-Cl-6-F-$C_6H_3$ | N | N | $CH_2Cl$ | 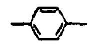 | 99-100 |
| 6 | 2-Cl-6-F-$C_6H_3$ | N | N | $CHCl_2$ | 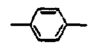 | oil[1)] |
| 7 | 2-Cl-6-F-$C_6H_3$ | N | N | $CH_2OMe$ | 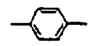 | oil[2)] |
| 8 | 2-Cl-6-F-$C_6H_3$ | N | N | $CH_2SMe$ | 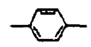 | oil[3)] |
| 9 | 2-Cl-6-F-$C_6H_3$ | N | N | H | 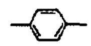 | 137-139 |
| 10 | 2-Cl-6-F-$C_6H_3$ | N | N | $NH_2$ | 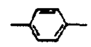 | 172-174 |
| 11 | 2-Cl-6-F-$C_6H_3$ | N | N | $NMe_2$ | 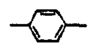 | oil[4)] |
| 12 | 2-Cl-6-F-$C_6H_3$ | N | N | Cl | 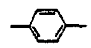 | 141-142 |
| 13 | 2-Cl-6-F-$C_6H_3$ | N | N | $CF_3$ | 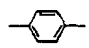 | oil[5)] |
| 14 | 2-Cl-6-F-$C_6H_3$ | N | N | $C_2F_5$ | 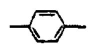 | oil[6)] |
| 15 | 2-Cl-6-F-$C_6H_3$ | N | N | CN | 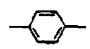 | oil[7)] |
| 16 | 2,6-$F_2$-$C_6H_3$ | N | N | $CH_3$ | 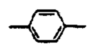 | 141-142 |
| 17 | 2,6-$F_2$-$C_6H_3$ | N | N | H | 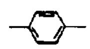 | 145-146 |
| 18 | 2,6-$F_2$-$C_6H_3$ | N | N | Cl | 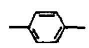 | 141-142 |
| 19 | 2,6-$F_2$-$C_6H_3$ | N | N | Et | 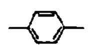 | 106-108 |
| 20 | 2,6-$F_2$-$C_6H_3$ | N | N | CN | 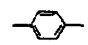 | 124-125 |

1) 2.7 (1H, br), 4.78 (2H, s), 6.79 (1H, s), 7.10 - 7.14 (1H, m), 7.31 - 7.39 (2H, m), 7.54 (2H, d *J* =8.3 Hz), 7.58 (2H, d *J* =8.3 Hz)

2) 3.48 (3H, s), 4.62 (2H, s), 4.73 (2H, s), 7.08 - 7.15 (1H, m), 7.27 - 7.38 (2H, m), 7.48 (2H, d *J* =8.3 Hz), 7.68 (2H, d *J* =8.3 Hz)

3) 2.23 (1H, t *J* =5.6 Hz), 2.27 (3H, s), 3.85 (2H, s), 4.78 (2H, t *J* =5.6 Hz), 7.10 - 7.12 (1H, m), 7.32 - 7.37 (2H, m), 7.51 - 7.53 (2H, m), 7.64 - 7.67 (2H, m)

4) 2.37 (6H, s), 3.63 (2H, s), 4.79 (2H, s), 7.10 - 7.11 (1H, m), 7.29 - 7.36 (2H, m), 7.50 - 7.53 (2H, m), 7.82 - 7.85 (2H, m)

5) 4.81 (2H, s), 7.09 - 7.18 (1H, m), 7.31 - 7.48 (6H, m)

6) 4.81 (2H, s), 7.11 - 7.15 (1H, m), 7.32 - 7.43 (2H, m), 7.54 (4H, s)

7) 2.11 (1H, br), 4.82 (2H, s), 7.11 - 7.21 (1H, m), 7.32 - 7.48 (2H, m), 7.57 - 7.67 (1H, m), 7.78 - 7.85 (2H, m)

Table 2

| No. | $Ar^1$ | $Y^1$ | $Y^2$ | $R^1$ | $Ar^2$ | mp. |
|---|---|---|---|---|---|---|
| 21 | $2,6-F_2-C_6H_3$ | N | N | $CF_3$ | 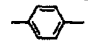 | 100-102 |
| 22 | $2,6-F_2-C_6H_3$ | N | N | $NH_2$ | 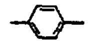 | 169-170 |
| 23 | $2,6-F_2-C_6H_3$ | N | N | $CF_3$ | 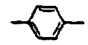 | oil[8] |
| 24 | $2,6-Cl_2-C_6H_3$ | N | N | $CH_3$ | 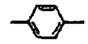 | 143-145 |
| 25 | $2,6-Cl_2-C_6H_3$ | N | N | CN | 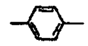 | oil[9] |
| 26 | $2,6-Cl_2-C_6H_3$ | N | N | H | 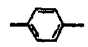 | 173-176 |
| 27 | $2,6-Cl_2-C_6H_3$ | N | N | Cl | 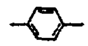 | 151-152 |
| 28 | $2,6-Cl_2-C_6H_3$ | N | N | Et | 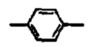 | 170-172 |
| 29 | $2,4,6-Cl_3-C_6H_2$ | N | N | $CH_3$ | 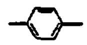 | oil[10] |
| 30 | $2-F-C_6H_3$ | N | N | $CH_3$ | 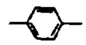 | 128-129 |
| 31 | $2-Cl-C_6H_3$ | N | N | $CH_3$ | 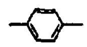 | 130-131 |
| 32 | $2-CF_3-C_6H_4$ | N | N | $CH_3$ | 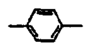 | 139-140 |
| 33 | $2,6-Me_2-C_6H_3$ | N | N | $CH_3$ | 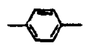 | 167-170 |
| 34 | $2,3,5,6-F_4-C_6H$ | N | N | $CH_3$ | 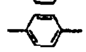 | 151-512 |
| 35 | $2-F-6-CF_3-C_6H_3$ | N | N | $CH_3$ | 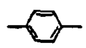 | oil[11] |
| 36 | $2,4,6-F_3-C_6H_2$ | N | N | $CH_3$ | 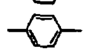 | 132-134 |
| 37 | $2-(OCF_3)-C_6H_4$ | N | N | $CH_3$ | 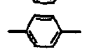 | 113-114 |
| 38 | $3-Cl-2,6-F_2-C_6H_2$ | N | N | $CH_3$ | 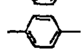 | 117-118 |
| 39 | $2-Cl-3,6-F_2-C_6H_2$ | N | N | $CH_3$ | 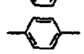 | oil[12] |
| 40 | $2,6-OMe_2-C_6H_3$ | N | N | $CH_3$ | 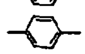 | 193-195 |
| 41 | $2,6-(CF_3)_2-C_6H_3$ | N | N | $CH_3$ | 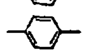 | 166-168 |
| 42 | $2-Cl-6-F-C_6H_3$ | N | N | $CH_3$ | 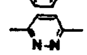 | amorphous[13] |
| 43 | $2,6-Cl_2$-pyridin-4-yl | N | N | H | 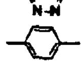 | 153-156 |
| 44 | 3-Cl-pyridin-2-yl | N | N | $CH_3$ | 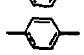 | 188-200 |
| 45 | 2-Cl-pyridin-3-yl | N | N | $CH_3$ | 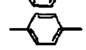 | 115-116 |

8) 2.29 (1H, br), 4.78 (2H, s), 7.36 (1H, dd *J*=9.0, 7.1 Hz), 7.43 (1H, d *J*=7.1 Hz), 7.43 (1H, d *J*=9.0 Hz), 7.51 - 7.57 (4H, m)

9) 2.04 (1H, br), 4.80 (2H, s), 7.39 (1H, dd *J*=9.3, 6.7 Hz), 7.45 (1H, d *J*=6.7 Hz), 7.54 (1H, d *J*=9.3 Hz), 7.57 - 7.61 (2H, m), 7.78 - 7.82 (2H, m)

10) 2.61 (3H, s), 3.5 (1H, br), 4.73 (2H, s), 7.43 (2H, s), 7.50 (1H, s)

11) 2.58 (3H, s), 3.4 (1H, br), 4.71 (2H, s), 7.34 - 7.39 (1H, m), 7.46 (4H, s), 7.54 - 7.61 (2H, m)

12) 2.60 (3H, s), 3.4 (1H, br), 4.72 (2H, s), 7.05 - 7.11 (1H, m), 7.22 (1H, ddd *J*=9.2, 8.2, 4.7 Hz), 7.48 (4H, s)

13) 3.08 (3H, s), 5.06 (2H, s), 7.10-7.18 (1H, m), 7.30 - 7.44 (2H, m), 7.79 (1H, d *J*=8.9 Hz), 8.20 (1H, d *J*=8.9 Hz),

Table 3

| No. | $Ar^1$ | $Y^1$ | $Y^2$ | $R^1$ | $Ar^2$ | mp. |
|---|---|---|---|---|---|---|
| 46 | 2-Cl-6-F-$C_6H_3$ | N | N | $CH_3$ | 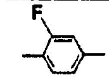 | 138-140 |
| 47 | 2-Cl-6-F-$C_6H_3$ | N | N | $CH_3$ | 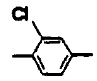 | 153-159 |
| 48 | 2-Cl-6-F-$C_6H_3$ | N | N | $CH_3$ | 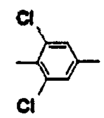 | 163-166 |
| 49 | 2-Cl-6-F-$C_6H_3$ | N | N | $CH_3$ | 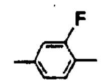 | 136-137 |
| 50 | 2-Cl-6-F-$C_6H_3$ | N | N | $CH_3$ | 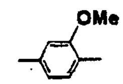 | amorphous[14)] |
| 51 | 2-Cl-6-F-$C_6H_3$ | N | N | $CH_3$ | 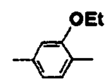 | oil[15)] |
| 52 | 2-Cl-6-F-$C_6H_3$ | CH | N | Cl | 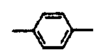 | oil[16)] |
| 53 | 2-Cl-6-F-$C_6H_3$ | CH | N | H | 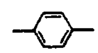 | 129-131 |
| 54 | 2-Cl-6-F-$C_6H_3$ | CH | N | $OCH_3$ | 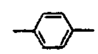 | oil[17)] |
| 55 | 2-Cl-6-F-$C_6H_3$ | CH | N | CN | 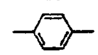 | 127-128 |
| 56 | 2-Cl-6-F-$C_6H_3$ | CH | N | $CH_3$ | 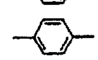 | oil[18)] |
| 57 | 2-F-$C_6H_3$ | N | CH | $CH_3$ | 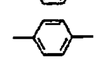 | 158-159 |
| 58 | 2-F-$C_6H_3$ | N | CH | Cl | 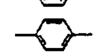 | 139-140 |
| 59 | 3-Cl-pyridin-2-yl | N | N | H | 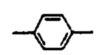 | 171-172 |
| 60 | 3-Cl-pyridin-2-yl | N | N | $CF_3$ | 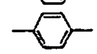 | 93-94 |
| 61 | 3-Cl-pyridin-2-yl | N | N | $CF_3$ | 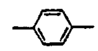 | 136-138 |

14) 2.60 (3H, s), 3.87 (3H, s), 4.70 (2H, s), 7.04 - 7.42 (6H, m)

15) 1.45 (3H, t *J* =7.0 Hz), 2.61 (3H, s), 4.11 (3H, q *J* =7.0 Hz), 4.73 (2H, s), 6.98 - 7.14 (3H, m), 7.28 - 7.46 (3H, m)

16) 2.4 (1H, br), 4.70 (2H, d *J* =3.4 Hz), 6.60 (1H, d *J* =0.8 Hz), 7.06 - 7.11 (1H, m), 7.28 - 7.31 (2H, m), 7.42 - 7.45 (2H, m), 7.58 - 7.61 (2H, m)

17) 2.52 (1H, br), 3.98 (3H, s), 4.62 (2H, s), 5.87 (1H, s), 7.04 - 7.09 (1H, m), 7.25 - 7.28 (2H, m), 7.33 - 7.36 (2H, m), 7.67 - 7.70 (2H, m)

18) 2.39 (3H, s), 4.69 (2H, s), 6.38 (1H, s), 7.01 - 7.49 (7H, m)

Reference Example 2

3-(2-chloro-6-fluorophenyl)-5-methyl-1*H*-1,2,4-triazole

The mixture of 2-chloro-6-fluorobenzamide (4.00 g, 23.0 mmol) and *N,N*-dimethylacetamide dimethylacetal (13 ml) was stirred under reflux with heating for 1 hour. The residue obtained by concentration under reduced pressure

was washed with petroleum ether to give yellow crystals (5.66 g). The resulting crystals were suspended in toluene (40 ml), and hydrazine hydrate (2.23 ml, 46 mmol) was added thereto, then the solution was refluxed with heating for 4 hours. To the reaction mixture was added ice-water (40 ml), and the precipitated crystals were collected by filtration. The obtained crystals were recrystallized from ethyl acetate to give 3-(2-chloro-6-fluorophenyl)-5-methyl-1*H*-1,2,4-triazole (1.80 g, 8.51 mmol, 37%) as colorless crystals.
mp: 232-233°C
$^1$H NMR (DMSO-$d_6$) δ ppm: 2.43 (3H, s), 7.31-7.45 (3H, m), 13.90 (1H, br)
$^{19}$F NMR (DMSO-$d_6$) δ ppm: -110.1 - -110 . 2 (m)
IR (nujol) ν $cm^{-1}$: 3300, 3189, 3079, 1664,1633,1604, 1576, 1519, 1455, 1252, 902, 792

3-(2,6-dichlorophenyl)-5-methyl-1*H*-1,2,4-triazole (mp:221-226°C) and 3-(2,6-difluorophenyl)-5-methyl-1*H*-1,2,4-triazole (mp: 195-198°C) were synthesized according to the same manner.

Production Example 1

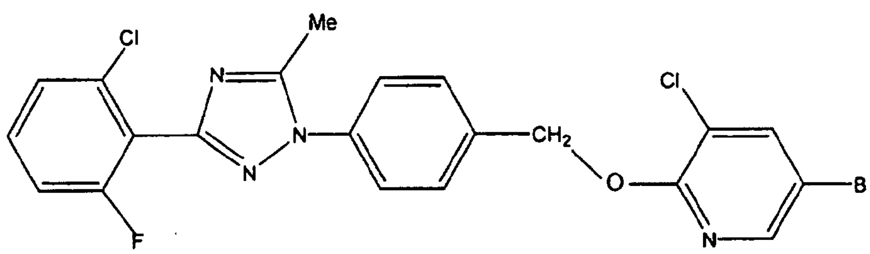

3-(2-chloro-6-fluorophenyl)-1-[4-(3-chloro-5-trifluoromethylpyridin-2-yloxymethyl)phenyl]-5-methyl-1*H*-1,2,4-triazole (T-21) (B represents trifluoromethyl)

To a solution of 47 mg (1.2 mmol) of sodium hydride (60% in oil) in 5 ml of DMF was added a solution of 0.25 g (0.79 mmol) of 4-[3-(2-chloro-6-fluorophenyl)-5-methyl-1*H*-1,2,4-triazol-1-yl]phenylmethanol in DMF (5 ml) under ice-cooling. Then, a solution of 0.17 g of 3-chloro-2-methanesulfonyl-5-trifluoromethylpyridine in DMF (5 ml) was added thereto and stirred for 24 hours at 50°C. Further, to the solution was added 47 mg (1.2 mmol) of sodium hydride (60% in oil) and stirred for 31 hours at 50°C. The reaction mixture was poured into 50 ml of water and extracted with 50 ml of ethyl acetate. The organic layer was dried, concentrated and the resulting residue was subjected to silica gel column chromatography (hexane : ethyl acetate = 3 : 1 → 2 : 1) to give 0 .18 g (0.36 mmol, 46%) of 3-(2-chloro-6-fluorophenyl)-1-[4-(3-chloro-5-trifluoromethylpyridin-2-yloxymethyl)phenyl]-5-methyl-1*H*-1,2,4-triazole as colorless crystals.
mp: 96-97°C
$^1$H NMR ($CDCl_3$) δ ppm: 2.66 (3H, s) , 5.60 (2H, s) , 7.06-7.16 (1H, m), 7.29-7.38 (2H, m), 7.58 (2H, d, J=8.8 Hz), 7.67 (2H, d, J=8.8 Hz), 7.90 (1H, d, J=2.1 Hz), 8.35 (1H, d, J=1.2 Hz)
$^{19}$F NMR ($CDCl_3$) δ ppm: -110.5 - -110. 6 (1F, m), -62.0 (3F, s)
IR (nujol) ν $cm^{-1}$: 1604, 1514, 1480, 1455,1406,1316, 1248, 1163, 1130, 1073, 898, 791, 762

Production Example 2

1-[4-[3-chloro-5-(trifluoromethyl)pyridin-2-yloxymethyl]phenyl]-4-(2-fluorophenyl)-2-methylimidazole (I-63)

To a suspension of 4-[4-(2-fluorophenyl)-2-methylimidazol-1-yl]phenylmethanol (0.48 g, 1.7 mmol) and 3-chloro-2-methanesulfonyl-5-trifluoromethylpyridine (0.51 g, 2.4 mmol) in THF (8 ml) was added 0 . 09 g (2.3 mmol) of sodium hydride (60% in oil), and stirred for 15 minutes at room temperature and further overnight at 50°C. The reaction mixture was filtered through Celite, and washed with acetone. The filtrate and washings were combined and concentrated, and the resulting residue was purified by subjecting to silica gel column chromatography (hexane : ethyl acetate = 3 : 2) to give 0.73 g (1.6 mmol, 94%) of 1-[4-(3-chloro-5-(trifluoromethyl)pyridin-2-yloxymethyl]phenyl]-4-(2-fluorophenyl)-2-methylimidazole as colorless crystals.
mp: 109°C
$^1$H NMR ($CDCl_3$) δ ppm: 2.47 (3H, s) , 5.60 (2H, s) , 7.05-7.27 (3H, m), 7.36-7.70 (5H, m), 7.88-7.93 (1H, m), 8.15-8.24 (1H, m), 8.34-8.40 (1H, m)
$^{19}$F NMR ($CDCl_3$) δ ppm: -114.73 (1F, br.s), -61.96 (3F, s)

IR (nujol) ν $cm^{-1}$: 1604, 1519, 1463, 1407, 1320, 1167, 1124, 1068, 753

Production Example 3

5-chloro-3-(2-chloro-6-fluorophenyl)-1-[4-(3-chloro-5-trifluoromethylpyridin-2-yloxymethyl)phenyl]pyrazole (P-21) To 0.46 g (11.5 mmol) of sodium hydride (60% in oil) was added 10 ml of DMF, and thereto was added a solution of 3.22 g (9.55 mmol) of 4-[5-chloro-3-(2-chloro-6-fluorophenyl)pyrazol-1-yl]phenylmethanol dissolved in DMF (30 ml) under ice-cooling. The reaction solution was stirred for 20 minutes and then was added dropwise 2.50 g (9.63 mmol) of 3-chloro-2-methanesulfonyl-5-trifluoromethylpyridine dissolved in DMF (10 ml). Then the resulting solution was stirred for 6 hours at room temperature, and thereto was added 80 ml of ice-water, 80 ml ofethyl acetate and 50 ml of saturated brine. The ethyl acetate layer was separated, and water layer was extracted with 50 ml of ethyl acetate. The ethyl acetate layers were combined, and washed with two 50 ml of saturated brine. The resulting ethyl acetate layer was dried over anhydrous magnesium sulfate, and the solvent was distilled away to give 6.34 g of yellow oil. A pale yellow crystal was obtained by purifying with silica gel column chromatography (ethyl acetate : hexane = 1 : 3). The crystal was washed with hexane to give 3.96 g (7.66 mmol, 80%) of 5-chloro-3-(2-chloro-6-fluorophenyl)-1-[4-(3-chloro-5-trifluoromethylpyridin-2-yloxymethyl)phenyl]pyrazole as yellow crystal.
mp: 120-121°C
$^1H$ NMR ($CDCl_3$) δ ppm: 5.58 (2H, s), 6.61 (1H, d J=1.0Hz), 7.08-7.12 (1H, m), 7.28-7.31 (2H, m), 7.60-7.70 (4H, m), 7.88 (1H, d J=2.2Hz), 8.34-8.35 (1H, m)
$^{19}F$ NMR ($CDCl_3$) δ ppm: -110.9 - -110.8 (1F, m), -62.0 (3F, s)
IR (nujol) ν $cm^{-1}$: 3126, 1606, 1464, 1448, 1331, 1125, 1074, 895, 826, 784

Production Example 4

3-(2-chloro-6-fluorophenyl)-1-[4-(3-chloro-5-trifluoromethyl-2-pyridyloxymethyl)phenylmethyl]-5-methyl-1*H*-1,2,4-triazine (T-171)

3-(2-chloro-6-fluorophenyl)-5-methyl-1*H*-1,2,4-triazine (0.22 g, 0.68 mmol), 4-chloromethylphenyl 3-chloro-5-trifluoromethyl-2-pyridyl ether (142.3 mg, 0.672 mmol), potassium carbonate (0.10 g, 7.2 mmol) and 18-crown-6 (a earpickful) were dissolved in DM (15 ml), and stirred for 2.5 hours on oil bath of 60°C. To the reaction solution was added water (5 ml) and saturated aqueous solution of ammonium chloride (15 ml), and extracted three times with 15 ml of ethyl acetate. The organic phases were combined and washed three times with 15 ml of saturated brine. The extract was dried over anhydrous magnesium sulfate, the inorganic salts were filtered off, and concentrated under reduced pressure to give 0.59 g of pale yellow oil. By purifying with silica gel column chromatography (hexane : ethyl acetate = 55 : 45), 0.19 g (0.38 mmol) of 3-(2-chloro-6-fluorophenyl)-1-[4-(3-chloro-5- trifluoromethyl-2-pyridyloxymethyl)phenylmethyl]-5-methyl-1*H*-1,2,4-triazine was obtained as colorless amorphous. Yield 55%
$^1H$ NMR ($CDCl_3$) δ ppm: 2.51 (3H, s) , 5.41 (2H, s), 7.05-7.36 (7H, m), 7.98-7.99 (1H, m), 8.25-8.28 (1H, m) $^{19}F$ NMR ($CDCl_3$) δ ppm: -110.81 - -110.74 (1F, m), -62.15 (3F, s)
IR (nujol) ν $cm^{-1}$: 1600, 1510, 1462, 1402, 1325, 1199, 1167, 1135, 1069

The compounds shown in the following tables 4-38 were produced according to the same manner.

Table 4

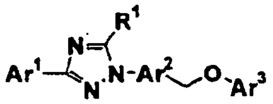

| No. | $R^1$ | $Ar^1$ | $Ar^2$ | $Ar^3$ | mp. |
|---|---|---|---|---|---|
| T-1 | $CH_3$ | 2-Cl-6-F-$C_6H_3$ | 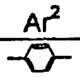 | 2-Cl-$C_6H_4$ | 121-122 |
| T-2 | $CH_3$ | 2-Cl-6-F-$C_6H_3$ | 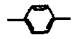 | 3-Cl-$C_6H_4$ | 118-119 |
| T-3 | $CH_3$ | 2-Cl-6-F-$C_6H_3$ | 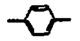 | 4-Cl-$C_6H_4$ | 124-126 |
| T-4 | $CH_3$ | 2-Cl-6-F-$C_6H_3$ | 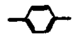 | 4-F-$C_6H_4$ | 130-131 |
| T-5 | $CH_3$ | 2-Cl-6-F-$C_6H_3$ | 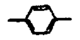 | 4-$CF_3$-$C_6H_4$ | 127-128 |
| T-6 | $CH_3$ | 2-Cl-6-F-$C_6H_3$ | 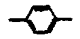 | 4-CN-$C_6H_4$ | 125-126 |
| T-7 | $CH_3$ | 2-Cl-6-F-$C_6H_3$ | 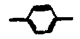 | 4-MeO-$C_6H_4$ | 124-126 |
| T-8 | $CH_3$ | 2-Cl-6-F-$C_6H_3$ | 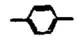 | 4-$NO_2$-$C_6H_4$ | 132-133 |
| T-9 | $CH_3$ | 2-Cl-6-F-$C_6H_3$ | 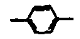 | 2,4-$Cl_2$-$C_6H_3$ | 138-140 |
| T-10 | $CH_3$ | 2-Cl-6-F-$C_6H_3$ | 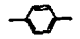 | 3,4-$Cl_2$-$C_6H_3$ | 151-152 |
| T-11 | $CH_3$ | 2-Cl-6-F-$C_6H_3$ | 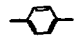 | 2,6-$Cl_2$-$C_6H_3$ | 123-124 |
| T-12 | $CH_3$ | 2-Cl-6-F-$C_6H_3$ | 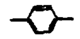 | 2-Cl-4-$CF_3$-$C_6H_3$ | 120-121 |
| T-13 | $CH_3$ | 2-Cl-6-F-$C_6H_3$ | 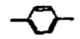 | 2-Cl-4-F-$C_6H_3$ | 120-122 |
| T-14 | $CH_3$ | 2-Cl-6-F-$C_6H_3$ | 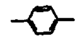 | 2,4,6-$Cl_3$-$C_6H_2$ | 127-128 |
| T-15 | $CH_3$ | 2-Cl-6-F-$C_6H_3$ | 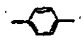 | 2,6-$Cl_2$-4-$CF_3$-$C_6H_2$ | 87-88 |
| T-16 | $CH_3$ | 2-Cl-6-F-$C_6H_3$ | 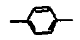 | 3-$CF_3$-pyridin-2-yl | 87-89 |
| T-17 | $CH_3$ | 2-Cl-6-F-$C_6H_3$ | 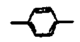 | 5-$CF_3$-pyridin-2-yl | 114-115 |
| T-18 | $CH_3$ | 2-Cl-6-F-$C_6H_3$ | 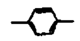 | 5-Cl-pyridin-2-yl | 126-127 |
| T-19 | $CH_3$ | 2-Cl-6-F-$C_6H_3$ | 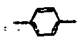 | 5-CN-pyridin-2-yl | 136-138 |
| T-20 | $CH_3$ | 2-Cl-6-F-$C_6H_3$ | 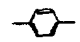 | 3,5-$Cl_2$-pyridin-2-yl | 137-139 |
| T-21 | $CH_3$ | 2-Cl-6-F-$C_6H_3$ | 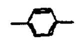 | 3-Cl-5-$CF_3$-pyridin-2-yl | 96-97 |
| T-22 | $CH_3$ | 2-Cl-6-F-$C_6H_3$ | 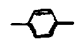 | 6-Cl-pyridazin-3-yl | 143-145 |
| T-23 | $CH_3$ | 2-Cl-6-F-$C_6H_3$ | 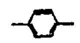 | 6-CN-pyridazin-3-yl | 169-170 |
| T-24 | $CH_3$ | 2-Cl-6-F-$C_6H_3$ | 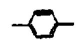 | 5-Cl-pyrimidin-2-yl | 152-153 |
| T-25 | $CH_3$ | 2-Cl-6-F-$C_6H_3$ | 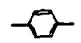 | 5-$CF_3$-1,3,4-thiadiazol-2-yl | 92-94 |
| T-26 | $CH_3$ | 2-Cl-6-F-$C_6H_3$ | 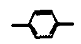 | 4'-Cl-biphenyl | 155-156 |
| T-27 | $CH_3$ | 2-Cl-6-F-$C_6H_3$ | 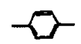 | 4-$Cl_2C$=CH-$C_6H_4$ | 139-140 |
| T-28 | $CH_3$ | 2-Cl-6-F-$C_6H_3$ | 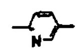 | 3-Cl-5-$CF_3$-pyridin-2-yl | 65~87 |
| T-29 | $CH_3$ | 2-Cl-6-F-$C_6H_3$ | 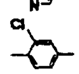 | 2,4,6-$Cl_3$-$C_6H_2$ | 138-139 |
| T-30 | $CH_3$ | 2-Cl-6-F-$C_6H_3$ | 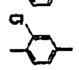 | 3-Cl-5-$CF_3$-pyridin-2-yl | amorphous[1)] |
| T-31 | $CH_3$ | 2-Cl-6-F-$C_6H_3$ | 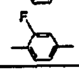 | 3-Cl-5-$CF_3$-pyridin-2-yl | 118-120 |

1) 2.46 (3H, s), 5.59 (2H, s), 7.06 - 7.15 (1H, m), 7.31 - 7.41 (2H, m), 7.55 (2H, d $J$ = 1.0 Hz), 7.73 (1H, s), 7.91 (1H, d $J$ = 2.1 Hz), 8.34 - 8.36 (1H, m)

Table 5

| No. | $R^1$ | $Ar^1$ | $Ar^2$ | $Ar^3$ | mp. |
|---|---|---|---|---|---|
| T-32 | $CH_3$ | 2-Cl-6-F-$C_6H_3$ | 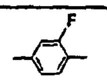 | 3-Cl-5-$CF_3$-pyridin-2-yl | 83-84 |
| T-33 | $CH_3$ | 2-Cl-6-F-$C_6H_3$ | 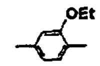 | 3-Cl-5-$CF_3$-pyridin-2-yl | 67-69 |
| T-34 | $CH_3$ | 2-Cl-6-F-$C_6H_3$ | 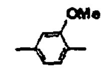 | 3-Cl-5-$CF_3$-pyridin-2-yl | |
| T-35 | $CH_3$ | 2-Cl-6-F-$C_6H_3$ | 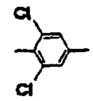 | 2,4,6-$Cl_3$-$C_6H_2$ | 203-205 |
| T-36 | $CH_3$ | 2-Cl-6-F-$C_6H_3$ | 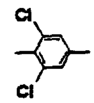 | 3-Cl-5-$CF_3$-pyridin-2-yl | 106-107 |
| T-37 | H | 2-Cl-6-F-$C_6H_3$ | 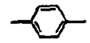 | 4-Cl-$C_6H_4$ | 104-106 |
| T-38 | H | 2-Cl-6-F-$C_6H_3$ | 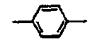 | 3-Cl-5-$CF_3$-pyridin-2-yl | 133-135 |
| T-39 | $NH_2$ | 2-Cl-6-F-$C_6H_3$ | 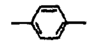 | 4-Cl-$C_6H_4$ | 180-182 |
| T-40 | $NH_2$ | 2-Cl-6-F-$C_6H_3$ | 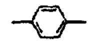 | 3-Cl-5-$CF_3$-pyridin-2-yl | 173-175 |
| T-41 | $NMe_2$ | 2-Cl-6-F-$C_6H_3$ | 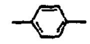 | 3-Cl-5-$CF_3$-pyridin-2-yl | 105-106 |
| T-42 | Cl | 2-Cl-6-F-$C_6H_3$ | 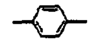 | 4-Cl-$C_6H_4$ | 114-115 |
| T-43 | Cl | 2-Cl-6-F-$C_6H_3$ | 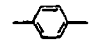 | 3-Cl-5-$CF_3$-pyridin-2-yl | 88-89 |
| T-44 | F | 2-Cl-6-F-$C_6H_3$ | 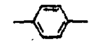 | 3-Cl-5-$CF_3$-pyridin-2-yl | 104-106 |
| T-45 | MeO | 2-Cl-6-F-$C_6H_3$ | 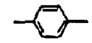 | 4-Cl-$C_6H_4$ | 132-133 |
| T-46 | MeO | 2-Cl-6-F-$C_6H_3$ | 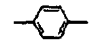 | 3-Cl-5-$CF_3$-pyridin-2-yl | 85 |
| T-47 | $CF_3$ | 2-Cl-6-F-$C_6H_3$ | 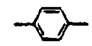 | 3-Cl-5-$CF_3$-pyridin-2-yl | 128-130 |
| T-48 | Et | 2-Cl-6-F-$C_6H_3$ | 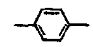 | 3-Cl-5-$CF_3$-pyridin-2-yl | 97-98 |
| T-49 | MeS | 2-Cl-6-F-$C_6H_3$ | 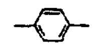 | 3-Cl-5-$CF_3$-pyridin-2-yl | 98-100 |
| T-50 | MeSO | 2-Cl-6-F-$C_6H_3$ | 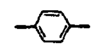 | 3-Cl-5-$CF_3$-pyridin-2-yl | 110-112 |
| T-51 | $MeSO_2$ | 2-Cl-6-F-$C_6H_3$ | 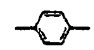 | 3-Cl-5-$CF_3$-pyridin-2-yl | 184-186 |
| T-52 | CN | 2-Cl-6-F-$C_6H_3$ | 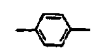 | 3-Cl-5-$CF_3$-pyridin-2-yl | 103-104 |
| T-53 | $CH_2Cl$ | 2-Cl-6-F-$C_6H_3$ | 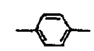 | 3-Cl-5-$CF_3$-pyridin-2-yl | oil[4)] |

[4)] 4.76 (1H, s), 5.62 (2H, s), 7.13 - 7.15 (1H, m), 7.34 - 7.39 (2H, m), 7.69 - 7.73 (4H, m), 7.90 (1H, m), 8.35 - 8.36 (1H, m)

Table 6

| No. | $R^1$ | $Ar^1$ | $Ar^2$ | $Ar^3$ | mp. |
|---|---|---|---|---|---|
| T-54 | $CH_2F$ | 2-Cl-6-F-$C_6H_3$ | 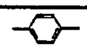 | 3-Cl-5-$CF_3$-pyridin-2-yl | oil[5)] |
| T-55 | $CH_2OH$ | 2-Cl-6-F-$C_6H_3$ | 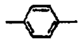 | 3-Cl-5-$CF_3$-pyridin-2-yl | 168-170 |
| T-56 | $CH_2OCH_3$ | 2-Cl-6-F-$C_6H_3$ | 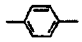 | 3-Cl-5-$CF_3$-pyridin-2-yl | 84-86 |
| T-57 | $CH_2OCH_2OCH_3$ | 2-Cl-6-F-$C_6H_3$ | 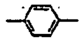 | 3-Cl-5-$CF_3$-pyridin-2-yl | oil[6)] |
| T-58 | $CH_2N(CH_3)_2$ | 2-Cl-6-F-$C_6H_3$ | 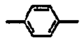 | 3-Cl-5-$CF_3$-pyridin-2-yl | oil[7)] |
| T-59 | $CH_2SCH_3$ | 2-Cl-6-F-$C_6H_3$ | 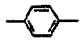 | 3-Cl-5-$CF_3$-pyridin-2-yl | oil[8)] |
| T-60 | $CH_2SO_2CH_3$ | 2-Cl-6-F-$C_6H_3$ | 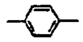 | 3-Cl-5-$CF_3$-pyridin-2-yl | 132-134 |
| T-61 | $CH_2CN$ | 2-Cl-6-F-$C_6H_3$ | 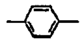 | 3-Cl-5-$CF_3$-pyridin-2-yl | oil[9)] |
| T-62 | $CH_3$ | 2-F-$C_6H_4$ | 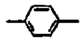 | 3-Cl-5-$CF_3$-pyridin-2-yl | 148-149 |
| T-63 | $CH_2OCH_3$ | 2-F-$C_6H_4$ | 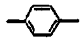 | 3-Cl-5-$CF_3$-pyridin-2-yl | 106-107 |
| T-64 | $CH_3$ | 2-Cl-$C_6H_4$ | 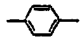 | 3-Cl-5-$CF_3$-pyridin-2-yl | 107-108 |
| T-65 | $CH_3$ | 2,6-$F_2$-$C_6H_4$ | 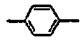 | 4-Cl-$C_6H_4$ | 127-128 |
| T-66 | $CH_3$ | 2,6-$F_2$-$C_6H_4$ | 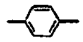 | 5-$CF_3$-pyridin-2-yl | 156-157 |
| T-67 | $CH_3$ | 2,6-$F_2$-$C_6H_4$ | 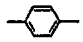 | 3-Cl-5-$CF_3$-pyridin-2-yl | 156-157 |
| T-68 | $NH_2$ | 2,6-$F_2$-$C_6H_4$ | 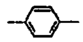 | 4-Cl-$C_6H_4$ | 172-174 |
| T-69 | Cl | 2,6-$F_2$-$C_6H_4$ | 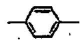 | 4-Cl-$C_6H_4$ | 97-98 |
| T-70 | $CH_3$ | 2,6-$Cl_2$-$C_6H_4$ | 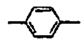 | 4-Cl-$C_6H_4$ | 163-164 |
| T-71 | $CH_3$ | 2,6-$Cl_2$-$C_6H_4$ | 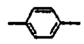 | 2,4-$Cl_2$-$C_6H_3$ | 176-177 |
| T-72 | $CH_3$ | 2,6-$Cl_2$-$C_6H_4$ | 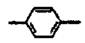 | 5-$CF_3$-pyridin-2-yl | 103-104 |
| T-73 | $CH_3$ | 2,6-$Cl_2$-$C_6H_4$ | 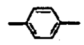 | 3-Cl-5-$CF_3$-pyridin-2-yl | 123-125 |
| T-74 | $CH_3$ | 2,6-$Me_2$-$C_6H_4$ | 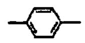 | 3-Cl-5-$CF_3$-pyridin-2-yl | oil[11)] |
| T-75 | H | 2,6-$Cl_2$-pyridin-4-yl | 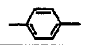 | 3-Cl-5-$CF_3$-pyridin-2-yl | 114-115 |

5) 5.55 (2H, d $J_{H-F}$ = 48.4 Hz), 5.61 (2H, s), 7.11 - 7.16 (1H, m), 7.32 - 7.41 (2H, m), 7.67 - 7.73 (4H, m), 7.90 (1H, d $J$ = 0.98 Hz), 8.35 (1H, d $J$ = 0.98 Hz)

6) 4.06 (2H, s), 5.62 (2H, s), 7.10 - 7.15 (1H, m), 7.32 - 7.40 (2H, m), 7.60 (2H, d $J$ = 8.3 Hz), 7.72 (2H, d $J$ = 8.3 Hz), 7.90 - 7.91 (1H, m), 8.35 (1H, m)

7) 1.44 (3H, t $J$ = 7.1 Hz), 2.98 (6H, s), 4.45 (2H, q $J$ = 7.1 Hz), 4.52 (2H, s), 7.14 - 7.19 (1H, m), 7.35 - 7.46 (2H, m), 7.69 (2H, d $J$ = 8.5 Hz), 8.26 - 8.28 (2H, m)

8) 2.29 (3H, s), 3.87 (2H, s), 5.60 (2H, s), 7.10 - 7.12 (1H, m), 7.32 - 7.37 (2H, m), 7.64 - 7.73 (4H, m), 7.89 - 7.90 (1H, m), 8.35 (1H, m)

9) 5.58 (2H, s), 5.72 (2H, s), 7.11 - 7.14 (1H, m), 7.32 - 7.37 (2H, m), 7.63 (2H, d $J$ = 8.50 Hz), 7.71 - 7.72 (2H, m), 7.86 - 7.89 (2H, m), 8.30 - 8.34 (2H, m)

11) 2.46 (3H, s), 5.59 (2H, s), 7.06 - 7.15 (1H, m), 7.31 - 7.41 (2H, m), 7.55 (2H, d $J$ = 1.0 Hz), 7.73 (1H, s), 7.91 (1H, d $J$ = 2.1 Hz), 8.34 - 8.36 (1H, m)

Table 7

| No. | $R^1$ | $Ar^1$ | $Ar^2$ | $Ar^3$ | mp. |
|---|---|---|---|---|---|
| T-76 | H | 2,6-$F_2$-$C_6H_3$ | 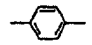 | 3-Cl-5-$CF_3$-pyridin-2-yl | 171-172 |
| T-77 | $CF_3$ | 2,6-$F_2$-$C_6H_3$ | 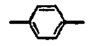 | 3-Cl-5-$CF_3$-pyridin-2-yl | 157-159 |
| T-78 | Cl | 2,6-$F_2$-$C_6H_3$ | 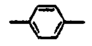 | 3-Cl-5-$CF_3$-pyridin-2-yl | 143-144 |
| T-79 | CN | 2,6-$F_2$-$C_6H_3$ | 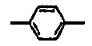 | 3-Cl-5-$CF_3$-pyridin-2-yl | 140-141 |
| T-80 | $CH_3$ | 2-Cl-6-F-$C_6H_3$ | 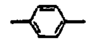 | 3,4,5,6-$Cl_4$-pyridin-2-yl | 190.5-192.5 |
| T-81 | $CH_3$ | 2-Cl-6-F-$C_6H_3$ | 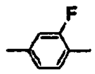 | 3,5-$Cl_2$-pyridin-2-yl | 126-127 |
| T-82 | $CH_3$ | 2,6-$Cl_2$-$C_6H_3$ | 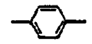 | 3,5-$Cl_2$-pyridin-2-yl | 181-184 |
| T-83 | H | 2,6-$F_2$-$C_6H_3$ | 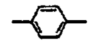 | 3,5-$Cl_2$-pyridin-2-yl | 176-177 |
| T-84 | H | 2,6-$F_2$-$C_6H_3$ | 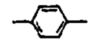 | 3-Cl-5-$CF_3$-pyridin-2-yl | 136-137 |
| T-85 | $CH_3$ | 2,6-$F_2$-$C_6H_3$ | 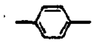 | 3,5-$Cl_2$-pyridin-2-yl | 165-167 |
| T-86 | $CH_3$ | 2,6-$F_2$-$C_6H_3$ | 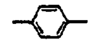 | 2-Cl-4-$CF_3$-$C_6H_3$ | 97-98 |
| T-87 | $C_2H_5$ | 2,6-$F_2$-$C_6H_3$ | 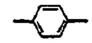 | 3,5-$Cl_2$-pyridin-2-yl | 118-119 |
| T-88 | $C_2H_5$ | 2,6-$F_2$-$C_6H_3$ | 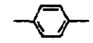 | 3-Cl-5-$CF_3$-pyridin-2-yl | 127-129 |
| T-89 | $C_2H_5$ | 2,6-$F_2$-$C_6H_3$ | 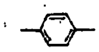 | 2-Cl-4-$CF_3$-$C_6H_3$ | 74-75 |
| T-90 | $CF_3$ | 2,6-$F_2$-$C_6H_3$ | 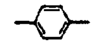 | 3,5-$Cl_2$-pyridin-2-yl | 126-127 |
| T-91 | $CF_3$ | 2,6-$F_2$-$C_6H_4$ | 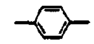 | 2-Cl-4-$CF_3$-$C_6H_3$ | 93-94 |
| T-92 | *i*-$C_3H_7$ | 2-Cl-6-F-$C_6H_3$ | 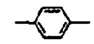 | 3-Cl-5-$CF_3$-pyridin-2-yl | 100-101.5 |
| T-93 | *c*-$C_3H_5$ | 2-Cl-6-F-$C_6H_3$ | 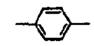 | 3-Cl-5-$CF_3$-pyridin-2-yl | 92.5-94 |
| T-94 | $CHCl_2$ | 2-Cl-6-F-$C_6H_3$ | 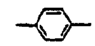 | 3-Cl-5-$CF_3$-pyridin-2-yl | 105-106 |
| T-95 | $C_2H_5$ | 2-Cl-6-F-$C_6H_3$ | 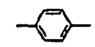 | 2-Cl-4-$CF_3$-$C_6H_3$ | oil[12] |
| T-96 | $C_2F_5$ | 2-Cl-6-F-$C_6H_3$ | 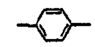 | 3-Cl-5-$CF_3$-pyridin-2-yl | oil[13] |
| T-97 | Cl | 2,6-$F_2$-$C_6H_4$ | 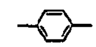 | 2-Cl-4-$CF_3$-$C_6H_3$ | 103-104 |
| T-98 | Cl | 2,6-$F_2$-$C_6H_4$ | 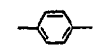 | 3,5-$Cl_2$-pyridin-2-yl | 169-170 |

12) 1.42(3H, t *J*=7.5Hz), 2.94(2H, q *J*=7.5Hz), 5.29(2H, s), 7.04(1H, d *J*=8.6Hz), 7.08 - 7.13(1H, m), 7.29 - 7.38(2H, m), 7.49(1H, d *J*=8.6Hz), 7.59(2H, AB *J*=8.6Hz), 7.63(2H, AB *J*=8.6Hz), 7.69(1H, d *J*=1.9Hz)

13) 5.62(2H, s), 7.11 - 7.15(1H, m), 7.32~7.43(2H, m), 7.59(2H, AB *J*=8.4Hz), 7.67(2H, AB *J*=8.4Hz), 7.90(1H, d *J*=2.2Hz), 8.35(1H, m)

Table 8

| No. | $R^1$ | $Ar^1$ | $Ar^2$ | $Ar^3$ | mp. |
|---|---|---|---|---|---|
| T-99 | CN | 2,6-$F_2$-$C_6H_3$ | 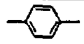 | 2-Cl-4-$CF_3$-$C_6H_3$ | 136-137 |
| T-100 | $CF_3$ | 2,6-$F_2$-$C_6H_3$ | 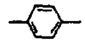 | 3,5-$Cl_2$-pyridin-2-yl | 190-101 |
| T-101 | H | 2,6-$Cl_2$-$C_6H_3$ | 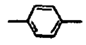 | 2-Cl-4-$CF_3$-$C_6H_3$ | 126-127 |
| T-102 | H | 2,6-$Cl_2$-$C_6H_3$ | 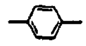 | 3,5-$Cl_2$-pyridin-2-yl | 179-181 |
| T-103 | $CH_3$ | 2,6-$Cl_2$-$C_6H_3$ | 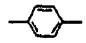 | 2-Cl-4-$CF_3$-$C_6H_3$ | 107-108 |
| T-104 | $C_2H_5$ | 2,6-$Cl_2$-$C_6H_3$ | 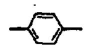 | 3,5-$Cl_2$-pyridin-2-yl | 137-138 |
| T-105 | $C_2H_5$ | 2,6-$Cl_2$-$C_6H_3$ | 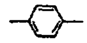 | 3-Cl-5-$CF_3$-pyridin-2-yl | oil[14)] |
| T-106 | $H_2C$=CH | 2-Cl-6-F-$C_6H_3$ | 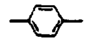 | 3-Cl-5-$CF_3$-pyridin-2-yl | oil[15)] |
| T-107 | CHO | 2-Cl-6-F-$C_6H_3$ | 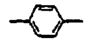 | 3-Cl-5-$CF_3$-pyridin-2-yl | amorphous[16)] |
| T-108 | $CH_3$ | 2-Cl-6-F-$C_6H_3$ | 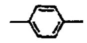 | 5-Cl-6-$CF_3$-pyrimidin-4-yl | oil[17)] |
| T-109 | $CH_3$ | 2-Cl-6-F-$C_6H_3$ | 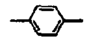 | 4-$CF_3$-pyrimidin-2-yl | oil[18)] |
| T-110 | $CH_3$ | 2-Cl-6-F-$C_6H_3$ | 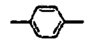 | 5-Cl-4-$CHF_2$-pyrimidin-6-yl | 135-136 |
| T-111 | $C_2H_5$ | 2,6-$Cl_2$-$C_6H_3$ | 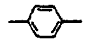 | 2-Cl-4-$CF_3$-$C_6H_3$ | oil[19)] |
| T-112 | $CF_3$ | 2,6-$Cl_2$-$C_6H_3$ | 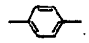 | 3-Cl-5-$CF_3$-pyridin-2-yl | oil[20)] |
| T-113 | $CF_3$ | 2,6-$Cl_2$-$C_6H_3$ | 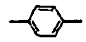 | 3,5-$Cl_2$-pyridin-2-yl | 115-117 |
| T-114 | Cl | 2,6-$Cl_2$-$C_6H_3$ | 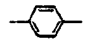 | 3,5-$Cl_2$-pyridin-2-yl | 156-157 |
| T-115 | Cl | 2,6-$Cl_2$-$C_6H_3$ | 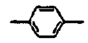 | 2-Cl-4-$CF_3$-$C_6H_3$ | 131-133 |
| T-116 | CN | 2,6-$Cl_2$-$C_6H_3$ | 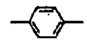 | 3,5-$Cl_2$-pyridin-2-yl | 163-165 |
| T-117 | CN | 2,6-$Cl_2$-$C_6H_3$ | 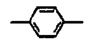 | 2-Cl-4-$CF_3$-$C_6H_3$ | 152-154 |
| T-118 | $CF_3$ | 2,6-$Cl_2$-$C_6H_3$ | 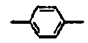 | 2-Cl-4-$CF_3$-$C_6H_3$ | oil[21)] |

14) 1.42 (3H, t J= 7.6 Hz), 2.95 (2H, q J=7.6 Hz), 5.60 (2H, s), 7.27 - 7.32 (1H, m), 7.38 - 7.42 (2H, m), 7.55 - 7.66 (4H, m), 7.89 (1H, d J = 2.2 Hz), 8.34-8.36 (1H, m)

15) 5.61 (2H, s), 5.72 (1H, dd J=10.9, 1.4 Hz), 6.53 (1H, dd J=17.3, 1.4 Hz), 6.69 (1H, dd J=17.3, 10.9 Hz), 7.09~7.14 (1H, m), 7.30~7.39 (2H, m), 7.58 (2H, AB J=8.4Hz), 7.66 (2H, AB J=8.4 Hz), 7.90 (1H, d J=2.2 Hz), 8.35~8.36 (1H, m).

16) 5.62 (2H, s), 7.14 - 7.18 (1H, m), 7.35 - 7.45 (2H, m), 7.63 - 7.68 (4H, m), 7.90 (1H, s), 8.35 (1H, s), 10.12 (1H, s)

17) 2.66 (3H, s), 5.65 (2H, s), 7.09 - 7.16 (1H, m), 7.29 - 7.40 (4H, m), 7.60 - 7.70 (2H, m), 7.89 - 7.90 (1H, m), 8.75 (1H, s)

18) 2.64 (3H, s), 5.57 (2H, s), 7.07 - 7.15 (1H, m), 7.27 - 7.39 (3H, m), 7.54 - 7.60 (2H, m), 7.66 - 7.72 (2H, m), 8.79 (1H, d J=4.8 Hz)

19) 1.42 (3H, t, J= 7.5 Hz), 2.95 (2H, q J=7.5 Hz), 5.28 (2H, s), 7.03 (1H, d J=8.5 Hz), 7.30 (1H, dd J=8.8, 7.3 Hz), 7.40 (1H, d J=7.3 Hz), 7.40 (1H, d J = 8.8 Hz), 7.46-7.49 (1H, m), 7.58-7.65 (4H, m), 7.67-7.69 (1H, m)

20) 5.62 (2H, s), 7.36 (1H, dd J=9.0, 6.9 Hz), 7.43 (1H, d J=6.9 Hz), 7.45 (1H, d J=9.0 Hz), 7.60 - 7.70 (4H, m), 7.90 (1H, d J = 2.1 Hz), 8.34 - 8.36 (1H, m)

21) 5.30 (2H, s), 7.03 (1H, d J=8.6 Hz), 7.36 (1H, dd J=9.1, 7.0 Hz), 7.43 (2H, dd J=9.1, 7.0 Hz), 7.49 (1H, dd J=8.6, 1.8 Hz), 7.62 - 7.70 (5H, m)

Table 9

| No. | $R^1$ | $Ar^1$ | $Ar^2$ | $Ar^3$ | mp. |
|---|---|---|---|---|---|
| T-119 | $CH_3$ | 3-Cl-pyridin-2-yl | 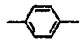 | 3-Cl-5-$CF_3$-pyridin-2-yl | oil[22)] |
| T-120 | $CHF_2$ | 2-Cl-6-F-$C_6H_3$ | 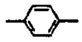 | 3-Cl-5-$CF_3$-pyridin-2-yl | oil[23)] |
| T-121 | H | 2-Cl-6-F-$C_6H_3$ | 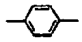 | 3,5-$Cl_2$-pyridin-2-yl | 182.5-184.5 |
| T-122 | Cl | 2-Cl-6-F-$C_6H_3$ | 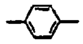 | 3,5-$Cl_2$-pyridin-2-yl | 142-143.5 |
| T-123 | CN | 2-Cl-6-F-$C_6H_3$ | 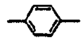 | 3,5-$Cl_2$-pyridin-2-yl | 141.5-142.5 |
| T-124 | $CF_3$ | 2-Cl-6-F-$C_6H_3$ | 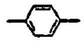 | 3,5-$Cl_2$-pyridin-2-yl | 90-91 |
| T-125 | H | 2-Cl-6-F-$C_6H_3$ | 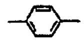 | 2-Cl-4-$CF_3$-$C_6H_3$ | 112.5-113.5 |
| T-126 | Cl | 2-Cl-6-F-$C_6H_3$ | 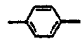 | 2-Cl-4-$CF_3$-$C_6H_3$ | oil[24)] |
| T-127 | CN | 2-Cl-6-F-$C_6H_3$ | 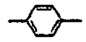 | 2-Cl-4-$CF_3$-$C_6H_3$ | 112.5-113 |
| T-128 | $CF_3$ | 2-Cl-6-F-$C_6H_3$ | 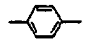 | 2-Cl-4-$CF_3$-$C_6H_3$ | oil[25)] |
| T-129 | $CH_3$ | 2-$CF_3$-$C_6H_4$ | 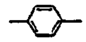 | 3-Cl-5-$CF_3$-pyridin-2-yl | oil[26)] |
| T-130 | $CH_3$ | 2-Cl-pyridin-3-yl | 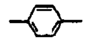 | 3-Cl-5-$CF_3$-pyridin-2-yl | 159-160 |
| T-131 | $CH_3$ | 2,4,6-$Cl_3$-$C_6H_2$ | 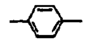 | 3-Cl-5-$CF_3$-pyridin-2-yl | oil[27)] |
| T-132 | HC≡C | 2-Cl-6-F-$C_6H_3$ | 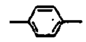 | 3-Cl-5-$CF_3$-pyridin-2-yl | oil[28)] |
| T-133 | $CH_3$ | 2-F-6-$CF_3$-$C_6H_3$ | 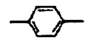 | 3-Cl-5-$CF_3$-pyridin-2-yl | oil[29)] |
| T-134 | $CH_3$ | 2,3,5,6-$F_4$-$C_6H_1$ | 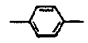 | 3-Cl-5-$CF_3$-pyridin-2-yl | 139-140 |
| T-135 | $CH_3$ | 2,4,6-$F_3$-$C_6H_2$ | 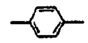 | 3-Cl-5-$CF_3$-pyridin-2-yl | 149-150 |
| T-136 | $CH_3$ | 2-$OCF_3$-$C_6H_4$ | 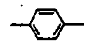 | 3-Cl-5-$CF_3$-pyridin-2-yl | 85-86 |
| T-137 | $CH_3$ | 3-Cl-2,6-$F_2$-$C_6H_2$ | 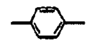 | 3-Cl-5-$CF_3$-pyridin-2-yl | 108-109 |
| T-138 | $CH_3$ | 2-Cl-3,6-$F_2$-$C_6H_2$ | 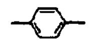 | 3-Cl-5-$CF_3$-pyridin-2-yl | 98-99 |

22) 2.65 (3H, s), 5.62 (2H, s), 7.31 (1H, dd $J$=8.1, 4.6 Hz), 7.59 (2H, AB $J$=8.5 Hz), 7.65 (2H, AB $J$=8.5 Hz), 7.85 (1H, dd $J$=8.1, 1.4 Hz), 7.90 (1H, d $J$=2.1 Hz), 8.36 (1H, s), 8.68 (1H, dd $J$=4.6, 1.3 Hz).

23) 5.61 (2H, s), 6.92 (1H, t $J$=52.4 Hz), 7.12 - 7.16 (1H, m), 7.33 - 7.43 (2H, m), 7.68 (4H, s), 7.90 (1H, d $J$=2.1 Hz), 8.35 (1H, m).

24) 5.29 (2H, s), 7.04 (1H, d $J$=8.6 Hz), 7.10 - 7.15 (1H, m), 7.32 - 7.42 (2H, m), 7.48 - 7.50 (1H, m), 7.66 (2H, AB $J$=8.5 Hz), 7.69 (1H, d $J$=2.2 Hz), 7.74 (2H, AB $J$=8.5 Hz)

25) 5.31 (2H, s), 7.04 (1H, d $J$=8.6 Hz), 7.14 (1H, t $J$=8.6 Hz), 7.33 - 7.44 (2H, m), 7.50 (1H, d $J$=8.5 Hz), 7.63 - 7.69 (5H, m).

26) 2.63 (3H, s), 5.59 (2H, s), 7.51 - 7.66 (6H, m), 7.78 - 7.88 (2H, m), 7.89 (1H, dd $J$=2.2, 0.3 Hz), 8.34 - 8.36 (1H, m)

27) 2.65 (3H, s), 5.60 (2H, s), 7.44 (2H, s), 7.56 - 7.60 (2H, m), 7.63 - 7.68 (2H, m), 7.89 (1H, d, $J$ = 2.2 Hz), 8.34-8.36 (1H, m)

28) 3.58 (1H, s), 5.59 (2H, s), 7.09 - 7.14 (1H, m), 7.31 - 7.40 (2H, m), 7.65 (2H, AB $J$=8.6 Hz), 7.89 - 7.90 (1H, m), 7.54 (2H, AB $J$=8.6 Hz), 8.34 - 8.35 (1H, m).

29) 2.64 (3H, s), 5.59 (2H, s), 7.35 - 7.40 (1H, m), 7.52 - 7.67 (6H, m), 7.89 (1H, dd $J$=2.3, 0.4 Hz), 8.34 - 8.36 (1H, m)

Table 10

| No. | $R^1$ | $Ar^1$ | $Ar^2$ | $Ar^3$ | mp. |
|---|---|---|---|---|---|
| T-139 | $CH_3$ | 2-Cl-6-F-$C_6H_3$ | 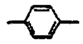 | 5-$CF_3$-pyrimidin-2-yl | 110-111 |
| T-140 | $CH_3$ | 2,6-$(OCH_3)_2C_6H_3$ | 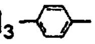 | 3-Cl-5-$CF_3$-pyridin-2-yl | amorphous[30)] |
| T-141 | $CH_3$ | 2-Cl-6-F-$C_6H_3$ | 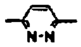 | 3-Cl-5-$CF_3$-pyridin-2-yl | 111-112 |
| T-142 | $CH_3$ | 2,6-$(CF_3)_2C_6H_3$ | 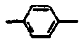 | 3-Cl-5-$CF_3$-pyridin-2-yl | oil[31)] |
| T-143 | $COOCH_3$ | 2-Cl-6-F-$C_6H_3$ | 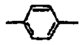 | 3-Cl-5-$CF_3$-pyridin-2-yl | 145.5-146.5 |
| T-144 | $CH_3$ | 3-Cl-pyridin-2-yl | 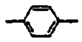 | 3,5-$Cl_2$-pyridin-2-yl | 91-94 |
| T-145 | H | 3-Cl-pyridin-2-yl | 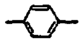 | 3,5-$Cl_2$-pyridin-2-yl | 178-180 |
| T-146 | Cl | 3-Cl-pyridin-2-yl | 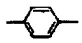 | 3,5-$Cl_2$-pyridin-2-yl | |
| T-147 | CN | 3-Cl-pyridin-2-yl | 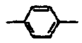 | 3,5-$Cl_2$-pyridin-2-yl | 196-198 |
| T-148 | $CF_3$ | 3-Cl-pyridin-2-yl | 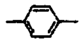 | 3,5-$Cl_2$-pyridin-2-yl | 102-103 |
| T-149 | H | 3-Cl-pyridin-2-yl | 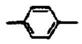 | 3-Cl-5-$CF_3$-pyridin-2-yl | 171-172 |
| T-150 | Cl | 3-Cl-pyridin-2-yl | 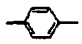 | 3-Cl-5-$CF_3$-pyridin-2-yl | 88-90 |
| T-151 | CN | 3-Cl-pyridin-2-yl | 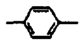 | 3-Cl-5-$CF_3$-pyridin-2-yl | 138-141 |
| T-152 | $CF_3$ | 3-Cl-pyridin-2-yl | 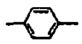 | 3-Cl-5-$CF_3$-pyridin-2-yl | 124-125 |
| T-153 | $CH_3$ | 3-Cl-pyridin-2-yl | 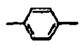 | 2-Cl-4-$CF_3$-$C_6H_3$ | |
| T-154 | H | 3-Cl-pyridin-2-yl | 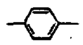 | 2-Cl-4-$CF_3$-$C_6H_3$ | |
| T-155 | Cl | 3-Cl-pyridin-2-yl | 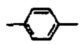 | 2-Cl-4-$CF_3$-$C_6H_3$ | |
| T-156 | CN | 3-Cl-pyridin-2-yl | 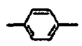 | 2-Cl-4-$CF_3$-$C_6H_3$ | |
| T-157 | $CF_3$ | 3-Cl-pyridin-2-yl | 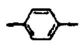 | 2-Cl-4-$CF_3$-$C_6H_3$ | |
| T-158 | $CH_3$ | 3-Cl-pyridin-2-yl | 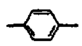 | 2,4-$Cl_2$-$C_6H_3$ | |
| T-159 | H | 3-Cl-pyridin-2-yl | 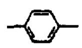 | 2,4-$Cl_2$-$C_6H_3$ | |
| T-160 | Cl | 3-Cl-pyridin-2-yl | 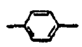 | 2,4-$Cl_2$-$C_6H_3$ | |
| T-161 | CN | 3-Cl-pyridin-2-yl | 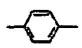 | 2,4-$Cl_2$-$C_6H_3$ | |
| T-162 | $CF_3$ | 3-Cl-pyridin-2-yl | 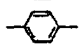 | 2,4-$Cl_2$-$C_6H_3$ | |
| T-163 | $CH_3$ | 3-Cl-pyridin-2-yl | 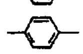 | 4-Cl-$C_6H_4$ | |
| T-164 | $CH_3$ | 3-Cl-pyridin-2-yl | 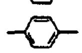 | 4-$CF_3$-$C_6H_4$ | |
| T-165 | $CH_3$ | 3-Cl-pyridin-2-yl | 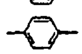 | 2-Cl-4-F-$C_6H_3$ | |
| T-166 | $CH_3$ | 3-Cl-pyridin-2-yl | 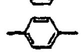 | 6-Cl-pyridazin-3-yl | |
| T-167 | $CH_3$ | 3-Cl-pyridin-2-yl | 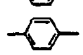 | 5-Cl-pyrimidin-2-yl | |
| T-168 | $CH_3$ | 3-Cl-pyridin-2-yl | 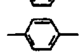 | 5-$CF_3$-1,3,4-thiadiazol-2-yl | |
| T-169 | $CH_3$ | 3-Cl-pyridin-2-yl | 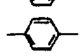 | 5-$CF_3$-pyrimidin-2-yl | |

30) 2.63 (3H, s), 3.78 (6H, s), 5.58 (2H, s), 6.62 (2H, d *J*=8.4 Hz), 7.33 (1H, t *J*=8.4 Hz), 7.57 - 7.63 (4H, m), 7.89 (1H, d *J* = 2.1 Hz), 8.34 - 8.35 (1H, m)

31) 2.63 (3H, s), 5.59 (2H, s), 7.54 - 7.58 (2H, m), 7.63 - 7.66 (2H, m), 7.68 - 7.73 (1H, m), 7.88 (1H, dd *J* = 2.3, 0.4 Hz), 7.98 (2H, d *J*=8.0 Hz), 8.33 - 8.35 (1H,

Table 11

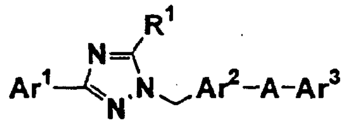

| No. | $R^1$ | $Ar^1$ | $Ar^2$ | A | $Ar^3$ | mp. |
|---|---|---|---|---|---|---|
| T-170 | $CH_3$ | 2-Cl-6-F-$C_6H_3$ | 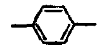 | — | 3-Cl-5-$CF_3$-pyridin-2-yl | 126-127 |
| T-171 | $CH_3$ | 2-Cl-6-F-$C_6H_3$ | 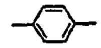 | O | 3-Cl-5-$CF_3$-pyridin-2-yl | amorphous[44)] |
| T-172 | $CH_3$ | 2-Cl-6-F-$C_6H_3$ | 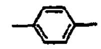 | $CH_2O$ | 3-Cl-5-$CF_3$-pyridin-2-yl | amorphous[45)] |
| T-173 | H | 2-Cl-6-F-$C_6H_3$ | 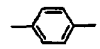 | — | 3-Cl-5-$CF_3$-pyridin-2-yl | |
| T-174 | H | 2-Cl-6-F-$C_6H_3$ | 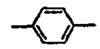 | O | 3-Cl-5-$CF_3$-pyridin-2-yl | |
| T-175 | H | 2-Cl-6-F-$C_6H_3$ | 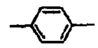 | $CH_2O$ | 3-Cl-5-$CF_3$-pyridin-2-yl | |
| T-176 | Cl | 2-Cl-6-F-$C_6H_3$ | 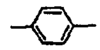 | — | 3-Cl-5-$CF_3$-pyridin-2-yl | |
| T-177 | Cl | 2-Cl-6-F-$C_6H_3$ | 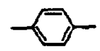 | O | 3-Cl-5-$CF_3$-pyridin-2-yl | |
| T-178 | Cl | 2-Cl-6-F-$C_6H_3$ | 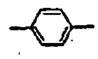 | $CH_2O$ | 3-Cl-5-$CF_3$-pyridin-2-yl | |
| T-179 | CN | 2-Cl-6-F-$C_6H_3$ | 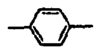 | — | 3-Cl-5-$CF_3$-pyridin-2-yl | |
| T-180 | CN | 2-Cl-6-F-$C_6H_3$ | 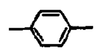 | O | 3-Cl-5-$CF_3$-pyridin-2-yl | |
| T-181 | CN | 2-Cl-6-F-$C_6H_3$ | 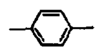 | $CH_2O$ | 3-Cl-5-$CF_3$-pyridin-2-yl | |
| T-182 | $CF_3$ | 2-Cl-6-F-$C_6H_3$ | 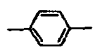 | — | 3-Cl-5-$CF_3$-pyridin-2-yl | |
| T-183 | $CF_3$ | 2-Cl-6-F-$C_6H_3$ | 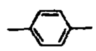 | O | 3-Cl-5-$CF_3$-pyridin-2-yl | |
| T-184 | $CF_3$ | 2-Cl-6-F-$C_6H_3$ | 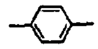 | $CH_2O$ | 3-Cl-5-$CF_3$-pyridin-2-yl | |

44) 2.51 (3H, s), 5.41 (1H, s), 7.05 - 7.36 (7H, m), 7.98 - 7.99 (1H, m), 8.25 - 8.28 (1H, m)

45) 2.48 (3H, s), 5.39 (2H, s), 5.51 (2H, s), 7.07 - 7.11 (1H, m), 7.23 - 7.35 (4H, m), 7.47 - 7.50 (2H, m), 7.86 - 7.87 (1H, m), 8.32 - 8.34 (1H, m)

Table 12

| No. | $R^1$ | $Ar^1$ | $Ar^2$ | A | $Ar^3$ | mp. |
|---|---|---|---|---|---|---|
| T-185 | $CH_3$ | 2-Cl-6-F-$C_6H_3$ | 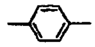 | — | 3,5-$Cl_2$-pyridin-2-yl | |
| T-186 | $CH_3$ | 2-Cl-6-F-$C_6H_3$ | 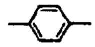 | O | 3,5-$Cl_2$-pyridin-2-yl | 129-130 |
| T-187 | $CH_3$ | 2-Cl-6-F-$C_6H_3$ | 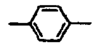 | $CH_2O$ | 3,5-$Cl_2$-pyridin-2-yl | |
| T-188 | H | 2-Cl-6-F-$C_6H_3$ | 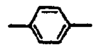 | — | 3,5-$Cl_2$-pyridin-2-yl | |
| T-189 | H | 2-Cl-6-F-$C_6H_3$ | 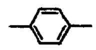 | O | 3,5-$Cl_2$-pyridin-2-yl | |
| T-190 | H | 2-Cl-6-F-$C_6H_3$ | 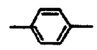 | $CH_2O$ | 3,5-$Cl_2$-pyridin-2-yl | |
| T-191 | Cl | 2-Cl-6-F-$C_6H_3$ | 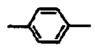 | — | 3,5-$Cl_2$-pyridin-2-yl | |
| T-192 | Cl | 2-Cl-6-F-$C_6H_3$ | 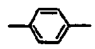 | O | 3,5-$Cl_2$-pyridin-2-yl | |
| T-193 | Cl | 2-Cl-6-F-$C_6H_3$ | 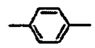 | $CH_2O$ | 3,5-$Cl_2$-pyridin-2-yl | |
| T-194 | CN | 2-Cl-6-F-$C_6H_3$ | 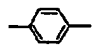 | — | 3,5-$Cl_2$-pyridin-2-yl | |
| T-195 | CN | 2-Cl-6-F-$C_6H_3$ | 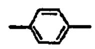 | O | 3,5-$Cl_2$-pyridin-2-yl | |
| T-196 | CN | 2-Cl-6-F-$C_6H_3$ | 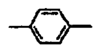 | $CH_2O$ | 3,5-$Cl_2$-pyridin-2-yl | |
| T-197 | $CF_3$ | 2-Cl-6-F-$C_6H_3$ | 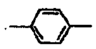 | — | 3,5-$Cl_2$-pyridin-2-yl | |
| T-198 | $CF_3$ | 2-Cl-6-F-$C_6H_3$ | 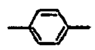 | O | 3,5-$Cl_2$-pyridin-2-yl | |
| T-199 | $CF_3$ | 2-Cl-6-F-$C_6H_3$ | 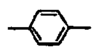 | $CH_2O$ | 3,5-$Cl_2$-pyridin-2-yl | |
| T-200 | $CH_3$ | 2,6-$Cl_2$-$C_6H_3$ | 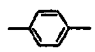 | — | 3,5-$Cl_2$-pyridin-2-yl | |
| T-201 | $CH_3$ | 2,6-$Cl_2$-$C_6H_3$ | 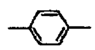 | O | 3,5-$Cl_2$-pyridin-2-yl | 176-177 |
| T-202 | $CH_3$ | 2,6-$Cl_2$-$C_6H_3$ | 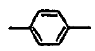 | $CH_2O$ | 3,5-$Cl_2$-pyridin-2-yl | |
| T-203 | H | 2,6-$Cl_2$-$C_6H_3$ | 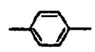 | — | 3,5-$Cl_2$-pyridin-2-yl | |
| T-204 | H | 2,6-$Cl_2$-$C_6H_3$ | 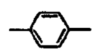 | O | 3,5-$Cl_2$-pyridin-2-yl | |
| T-205 | H | 2,6-$Cl_2$-$C_6H_3$ | 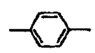 | $CH_2O$ | 3,5-$Cl_2$-pyridin-2-yl | |
| T-206 | Cl | 2,6-$Cl_2$-$C_6H_3$ | 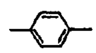 | — | 3,5-$Cl_2$-pyridin-2-yl | |
| T-207 | Cl | 2,6-$Cl_2$-$C_6H_3$ | 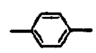 | O | 3,5-$Cl_2$-pyridin-2-yl | |
| T-208 | Cl | 2,6-$Cl_2$-$C_6H_3$ | 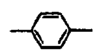 | $CH_2O$ | 3,5-$Cl_2$-pyridin-2-yl | |
| T-209 | CN | 2,6-$Cl_2$-$C_6H_3$ | 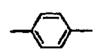 | — | 3,5-$Cl_2$-pyridin-2-yl | |
| T-210 | CN | 2,6-$Cl_2$-$C_6H_3$ | 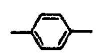 | O | 3,5-$Cl_2$-pyridin-2-yl | |
| T-211 | CN | 2,6-$Cl_2$-$C_6H_3$ | 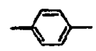 | $CH_2O$ | 3,5-$Cl_2$-pyridin-2-yl | |

Table 13

| No. | $R^1$ | $Ar^1$ | $Ar^2$ | A | $Ar^3$ | mp. |
|---|---|---|---|---|---|---|
| T-212 | $CF_3$ | 2,6-$Cl_2$-$C_6H_3$ | 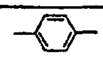 | — | 3,5-$Cl_2$-pyridin-2-yl | |
| T-213 | $CF_3$ | 2,6-$Cl_2$-$C_6H_3$ | 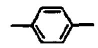 | O | 3,5-$Cl_2$-pyridin-2-yl | |
| T-214 | $CF_3$ | 2,6-$Cl_2$-$C_6H_3$ | 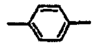 | $CH_2O$ | 3,5-$Cl_2$-pyridin-2-yl | |
| T-215 | $CH_3$ | 2,6-$Cl_2$-$C_6H_3$ | 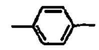 | — | 3-Cl-5-$CF_3$-pyridin-2-yl | |
| T-216 | $CH_3$ | 2,6-$Cl_2$-$C_6H_3$ | 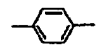 | O | 3-Cl-5-$CF_3$-pyridin-2-yl | oil[101)] |
| T-217 | $CH_3$ | 2,6-$Cl_2$-$C_6H_3$ | 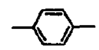 | $CH_2O$ | 3-Cl-5-$CF_3$-pyridin-2-yl | |
| T-218 | H | 2,6-$Cl_2$-$C_6H_3$ | 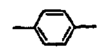 | — | 3-Cl-5-$CF_3$-pyridin-2-yl | |
| T-219 | H | 2,6-$Cl_2$-$C_6H_3$ | 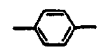 | O | 3-Cl-5-$CF_3$-pyridin-2-yl | |
| T-220 | H | 2,6-$Cl_2$-$C_6H_3$ | 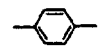 | $CH_2O$ | 3-Cl-5-$CF_3$-pyridin-2-yl | |
| T-221 | Cl | 2,6-$Cl_2$-$C_6H_3$ | 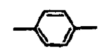 | — | 3-Cl-5-$CF_3$-pyridin-2-yl | |
| T-222 | Cl | 2,6-$Cl_2$-$C_6H_3$ | 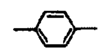 | O | 3-Cl-5-$CF_3$-pyridin-2-yl | |
| T-223 | Cl | 2,6-$Cl_2$-$C_6H_3$ | 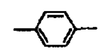 | $CH_2O$ | 3-Cl-5-$CF_3$-pyridin-2-yl | |
| T-224 | CN | 2,6-$Cl_2$-$C_6H_3$ | 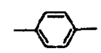 | — | 3-Cl-5-$CF_3$-pyridin-2-yl | |
| T-225 | CN | 2,6-$Cl_2$-$C_6H_3$ | 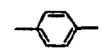 | O | 3-Cl-5-$CF_3$-pyridin-2-yl | |
| T-226 | CN | 2,6-$Cl_2$-$C_6H_3$ | 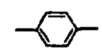 | $CH_2O$ | 3-Cl-5-$CF_3$-pyridin-2-yl | |
| T-227 | $CF_3$ | 2,6-$Cl_2$-$C_6H_3$ | 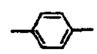 | — | 3-Cl-5-$CF_3$-pyridin-2-yl | |
| T-228 | $CF_3$ | 2,6-$Cl_2$-$C_6H_3$ | 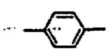 | O | 3-Cl-5-$CF_3$-pyridin-2-yl | |
| T-229 | $CF_3$ | 2,6-$Cl_2$-$C_6H_3$ | 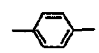 | $CH_2O$ | 3-Cl-5-$CF_3$-pyridin-2-yl | |
| T-230 | $CH_3$ | 2,6-$F_2$-$C_6H_3$ | 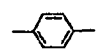 | — | 3-Cl-5-$CF_3$-pyridin-2-yl | |
| T-231 | $CH_3$ | 2,6-$F_2$-$C_6H_3$ | 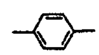 | O | 3-Cl-5-$CF_3$-pyridin-2-yl | oil[102)] |
| T-232 | $CH_3$ | 2,6-$F_2$-$C_6H_3$ | 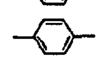 | $CH_2O$ | 3-Cl-5-$CF_3$-pyridin-2-yl | |
| T-233 | H | 2,6-$F_2$-$C_6H_3$ | 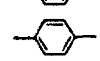 | — | 3-Cl-5-$CF_3$-pyridin-2-yl | |
| T-234 | H | 2,6-$F_2$-$C_6H_3$ | 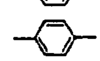 | O | 3-Cl-5-$CF_3$-pyridin-2-yl | |
| T-235 | H | 2,6-$F_2$-$C_6H_3$ | 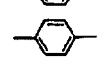 | $CH_2O$ | 3-Cl-5-$CF_3$-pyridin-2-yl | |
| T-236 | Cl | 2,6-$F_2$-$C_6H_3$ | 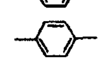 | — | 3-Cl-5-$CF_3$-pyridin-2-yl | |
| T-237 | Cl | 2,6-$F_2$-$C_6H_3$ | 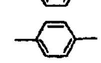 | O | 3-Cl-5-$CF_3$-pyridin-2-yl | |
| T-238 | Cl | 2,6-$F_2$-$C_6H_3$ | 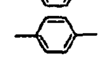 | $CH_2O$ | 3-Cl-5-$CF_3$-pyridin-2-yl | |

101) 2.51 (3H, s), 5.42 (1H, s), 7.17-7.19 (2H, m), 7.28 - 7.33 (3H, m), 7.39 - 7.40 (2H, m), 7.99 (1H, d $J$=2.0 Hz), 8.27 (1H, s)

102) 2.52 (3H, s), 5.41 (1H, s), 6.98-7.02 (2H, m), 7.17 - 7.19 (2H, m), 7.32 - 7.36 (3H, m), 7.98 (1H, d $J$=2.2 Hz), 8.26 (1H, d $J$=2.2 Hz))

Table 14

| No. | $R^1$ | $Ar^1$ | $Ar^2$ | A | $Ar^3$ | mp. |
|---|---|---|---|---|---|---|
| T-239 | CN | 2,6-$F_2$-$C_6H_3$ | 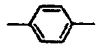 | — | 3-Cl-5-$CF_3$-pyridin-2-yl | |
| T-240 | CN | 2,6-$F_2$-$C_6H_3$ | 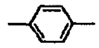 | O | 3-Cl-5-$CF_3$-pyridin-2-yl | |
| T-241 | CN | 2,6-$F_2$-$C_6H_3$ | 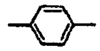 | $CH_2O$ | 3-Cl-5-$CF_3$-pyridin-2-yl | |
| T-242 | $CF_3$ | 2,6-$F_2$-$C_6H_3$ | 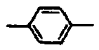 | — | 3-Cl-5-$CF_3$-pyridin-2-yl | |
| T-243 | $CF_3$ | 2,6-$F_2$-$C_6H_3$ | 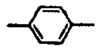 | O | 3-Cl-5-$CF_3$-pyridin-2-yl | |
| T-244 | $CF_3$ | 2,6-$F_2$-$C_6H_3$ | 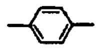 | $CH_2O$ | 3-Cl-5-$CF_3$-pyridin-2-yl | |
| T-245 | $CH_3$ | 2,6-$F_2$-$C_6H_3$ | 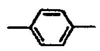 | — | 3,5-$Cl_2$-pyridin-2-yl | |
| T-246 | $CH_3$ | 2,6-$F_2$-$C_6H_3$ | 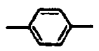 | O | 3,5-$Cl_2$-pyridin-2-yl | 161-162 |
| T-247 | $CH_3$ | 2,6-$F_2$-$C_6H_3$ | 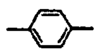 | $CH_2O$ | 3,5-$Cl_2$-pyridin-2-yl | |
| T-248 | H | 2,6-$F_2$-$C_6H_3$ | 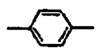 | — | 3,5-$Cl_2$-pyridin-2-yl | |
| T-249 | H | 2,6-$F_2$-$C_6H_3$ | 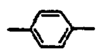 | O | 3,5-$Cl_2$-pyridin-2-yl | |
| T-250 | H | 2,6-$F_2$-$C_6H_3$ | 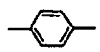 | $CH_2O$ | 3,5-$Cl_2$-pyridin-2-yl | |
| T-251 | Cl | 2,6-$F_2$-$C_6H_3$ | 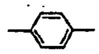 | — | 3,5-$Cl_2$-pyridin-2-yl | |
| T-252 | Cl | 2,6-$F_2$-$C_6H_3$ | 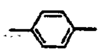 | O | 3,5-$Cl_2$-pyridin-2-yl | |
| T-253 | Cl | 2,6-$F_2$-$C_6H_3$ | 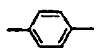 | $CH_2O$ | 3,5-$Cl_2$-pyridin-2-yl | |
| T-254 | CN | 2,6-$F_2$-$C_6H_3$ | 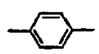 | — | 3,5-$Cl_2$-pyridin-2-yl | |
| T-255 | CN | 2,6-$F_2$-$C_6H_3$ | 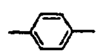 | O | 3,5-$Cl_2$-pyridin-2-yl | |
| T-256 | CN | 2,6-$F_2$-$C_6H_3$ | 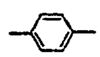 | $CH_2O$ | 3,5-$Cl_2$-pyridin-2-yl | |
| T-257 | $CF_3$ | 2,6-$F_2$-$C_6H_3$ | 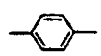 | — | 3,5-$Cl_2$-pyridin-2-yl | |
| T-258 | $CF_3$ | 2,6-$F_2$-$C_6H_3$ | 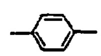 | O | 3,5-$Cl_2$-pyridin-2-yl | |
| T-259 | $CF_3$ | 2,6-$F_2$-$C_6H_3$ | 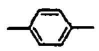 | $CH_2O$ | 3,5-$Cl_2$-pyridin-2-yl | |

Table 15

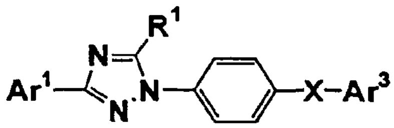

| No. | $R^1$ | $Ar^1$ | X | $Ar^3$ | mp |
|---|---|---|---|---|---|
| T-260 | $CH_3$ | 2-Cl-6-F-$C_6H_3$ | $CH_2S$ | 4-Cl-$C_6H_4$ | 124-125 |
| T-261 | $CH_3$ | 2-Cl-6-F-$C_6H_3$ | $CH_2NH$ | 4-Cl-$C_6H_4$ | 109-111 |
| T-262 | $CH_3$ | 2-Cl-6-F-$C_6H_3$ | $OCH_2$ | 4-Cl-$C_6H_4$ | 149-151 |
| T-263 | $CH_3$ | 2-Cl-6-F-$C_6H_3$ | $OCH_2$ | 2,4,6-$Cl_3$-$C_6H_2$ | 146-148 |
| T-264 | $CH_3$ | 2-Cl-6-F-$C_6H_3$ | O | 3-Cl-5-$CF_3$-pyridin-2-yl | 84-85 |
| T-265 | $CH_3$ | 2-Cl-6-F-$C_6H_3$ | NH | 5-$CF_3$-pyridin-2-yl | 78-81 |
| T-266 | $CH_3$ | 2-Cl-6-F-$C_6H_3$ | — | 4-$CF_3O$-$C_6H_4$ | 92 |
| T-267 | $CH_3$ | 2-Cl-6-F-$C_6H_3$ | $CH(CH_3)O$ | 3-Cl-5-$CF_3$-pyridin-2-yl | amorphous[32)] |
| T-268 | $CH_3$ | 2-Cl-6-F-$C_6H_3$ | $CH(CH_3)O$ | 3,5-$Cl_2$-pyridin-2-yl | amorphous[33)] |
| T-269 | $CH_3$ | 2-Cl-6-F-$C_6H_3$ | $OCH_2$ | 3-Cl-5-$CF_3$-pyridin-2-yl | 166-167 |
| T-270 | $CH_3$ | 2-Cl-6-F-$C_6H_3$ | $OCH(CH_3)$ | 3-Cl-5-$CF_3$-pyridin-2-yl | amorphous[36)] |
| T-271 | $CH_3$ | 2-Cl-6-F-$C_6H_3$ | $CH(CN)O$ | 3-Cl-5-$CF_3$-pyridin-2-yl | 168-169 |
| T-272 | $CH_3$ | 2-Cl-6-F-$C_6H_3$ | — | 3-Cl-5-$CF_3$-pyridin-2-yl | 189-190 |
| T-273 | Cl | 2-Cl-6-F-$C_6H_3$ | $CH_2S$ | 4-Cl-$C_6H_4$ | |
| T-274 | Cl | 2-Cl-6-F-$C_6H_3$ | $CH_2NH$ | 4-Cl-$C_6H_4$ | |
| T-275 | Cl | 2-Cl-6-F-$C_6H_3$ | $OCH_2$ | 4-Cl-$C_6H_4$ | |
| T-276 | Cl | 2-Cl-6-F-$C_6H_3$ | $OCH_2$ | 2,4,6-$Cl_3$-$C_6H_2$ | |
| T-277 | Cl | 2-Cl-6-F-$C_6H_3$ | O | 3-Cl-5-$CF_3$-pyridin-2-yl | |
| T-278 | Cl | 2-Cl-6-F-$C_6H_3$ | NH | 5-$CF_3$-pyridin-2-yl | |
| T-279 | Cl | 2-Cl-6-F-$C_6H_3$ | — | 4-$CF_3O$-$C_6H_4$ | |
| T-280 | Cl | 2-Cl-6-F-$C_6H_3$ | $CH(CH_3)O$ | 3-Cl-5-$CF_3$-pyridin-2-yl | |
| T-281 | Cl | 2-Cl-6-F-$C_6H_3$ | $CH(CH_3)O$ | 3,5-$Cl_2$-pyridin-2-yl | |
| T-282 | Cl | 2-Cl-6-F-$C_6H_3$ | $OCH_2$ | 3-Cl-5-$CF_3$-pyridin-2-yl | |
| T-283 | Cl | 2-Cl-6-F-$C_6H_3$ | $OCH(CH_3)$ | 3-Cl-5-$CF_3$-pyridin-2-yl | |
| T-284 | Cl | 2-Cl-6-F-$C_6H_3$ | $CH(CN)O$ | 3-Cl-5-$CF_3$-pyridin-2-yl | |
| T-285 | Cl | 2-Cl-6-F-$C_6H_3$ | — | 3-Cl-5-$CF_3$-pyridin-2-yl | |

32) 1.74 (3H, d *J*=6.6 Hz), 2.63 (3H, s), 6.37 (1H, q *J*=6.6 Hz), 7.06-7.15 (1H, m), 7.27-7.39 (2H, m), 7.50-7.67 (4H, m), 7.85-7.88 (1H, m), 8.25-8.30 (1H, m)

33) 1.71 (3H, d *J*=6.6 Hz), 2.63 (3H, s), 6.26 (1H, q *J*=6.6 Hz), 7.06-7.14 (1H, m), 7.27-7.38 (2H, m), 7.49-7.63 (4H, m), 7.64 (1H, d *J*=2.4 Hz), 7.93 (1H, d *J*=2.4 Hz)

36) 1.78 (3H, d *J*=6.5 Hz), 2.56 (3H, s), 5.92 (1H, q *J*=6.5 Hz), 6.94-7.00 (2H, m), 7.05-7.12 (1H, m), 7.26-7.43 (4H, m), 7.95-7.99 (1H, m), 8.86-8.82 (1H, m)

Table 16

| No. | $R^1$ | $Ar^1$ | X | $Ar^3$ | mp |
|---|---|---|---|---|---|
| T-286 | H | 2-Cl-6-F-$C_6H_3$ | $CH_2S$ | 4-Cl-$C_6H_4$ | |
| T-287 | H | 2-Cl-6-F-$C_6H_3$ | $CH_2NH$ | 4-Cl-$C_6H_4$ | |
| T-288 | H | 2-Cl-6-F-$C_6H_3$ | $OCH_2$ | 4-Cl-$C_6H_4$ | |
| T-289 | H | 2-Cl-6-F-$C_6H_3$ | $OCH_2$ | 2,4,6-$Cl_3$-$C_6H_2$ | |
| T-290 | H | 2-Cl-6-F-$C_6H_3$ | O | 3-Cl-5-$CF_3$-pyridin-2-yl | |
| T-291 | H | 2-Cl-6-F-$C_6H_3$ | NH | 5-$CF_3$-pyridin-2-yl | |
| T-292 | H | 2-Cl-6-F-$C_6H_3$ | — | 4-$CF_3O$-$C_6H_4$ | |
| T-293 | H | 2-Cl-6-F-$C_6H_3$ | $CH(CH_3)O$ | 3-Cl-5-$CF_3$-pyridin-2-yl | |
| T-294 | H | 2-Cl-6-F-$C_6H_3$ | $CH(CH_3)O$ | 3,5-$Cl_2$-pyridin-2-yl | |
| T-295 | H | 2-Cl-6-F-$C_6H_3$ | $OCH_2$ | 3-Cl-5-$CF_3$-pyridin-2-yl | |
| T-296 | H | 2-Cl-6-F-$C_6H_3$ | $OCH(CH_3)$ | 3-Cl-5-$CF_3$-pyridin-2-yl | |
| T-297 | H | 2-Cl-6-F-$C_6H_3$ | CH(CN)O | 3-Cl-5-$CF_3$-pyridin-2-yl | |
| T-298 | H | 2-Cl-6-F-$C_6H_3$ | — | 3-Cl-5-$CF_3$-pyridin-2-yl | |
| T-299 | CN | 2-Cl-6-F-$C_6H_3$ | $CH_2S$ | 4-Cl-$C_6H_4$ | |
| T-300 | CN | 2-Cl-6-F-$C_6H_3$ | $CH_2NH$ | 4-Cl-$C_6H_4$ | |
| T-301 | CN | 2-Cl-6-F-$C_6H_3$ | $OCH_2$ | 4-Cl-$C_6H_4$ | |
| T-302 | CN | 2-Cl-6-F-$C_6H_3$ | $OCH_2$ | 2,4,6-$Cl_3$-$C_6H_2$ | |
| T-303 | CN | 2-Cl-6-F-$C_6H_3$ | O | 3-Cl-5-$CF_3$-pyridin-2-yl | |
| T-304 | CN | 2-Cl-6-F-$C_6H_3$ | NH | 5-$CF_3$-pyridin-2-yl | |
| T-305 | CN | 2-Cl-6-F-$C_6H_3$ | — | 4-$CF_3O$-$C_6H_4$ | |
| T-306 | CN | 2-Cl-6-F-$C_6H_3$ | $CH(CH_3)O$ | 3-Cl-5-$CF_3$-pyridin-2-yl | |
| T-307 | CN | 2-Cl-6-F-$C_6H_3$ | $CH(CH_3)O$ | 3,5-$Cl_2$-pyridin-2-yl | |
| T-308 | CN | 2-Cl-6-F-$C_6H_3$ | $OCH_2$ | 3-Cl-5-$CF_3$-pyridin-2-yl | |
| T-309 | CN | 2-Cl-6-F-$C_6H_3$ | $OCH(CH_3)$ | 3-Cl-5-$CF_3$-pyridin-2-yl | |
| T-310 | CN | 2-Cl-6-F-$C_6H_3$ | CH(CN)O | 3-Cl-5-$CF_3$-pyridin-2-yl | |
| T-311 | CN | 2-Cl-6-F-$C_6H_3$ | — | 3-Cl-5-$CF_3$-pyridin-2-yl | |
| T-312 | $CF_3$ | 2-Cl-6-F-$C_6H_3$ | $CH_2S$ | 4-Cl-$C_6H_4$ | |
| T-313 | $CF_3$ | 2-Cl-6-F-$C_6H_3$ | $CH_2NH$ | 4-Cl-$C_6H_4$ | |
| T-314 | $CF_3$ | 2-Cl-6-F-$C_6H_3$ | $OCH_2$ | 4-Cl-$C_6H_4$ | |
| T-315 | $CF_3$ | 2-Cl-6-F-$C_6H_3$ | $OCH_2$ | 2,4,6-$Cl_3$-$C_6H_2$ | |
| T-316 | $CF_3$ | 2-Cl-6-F-$C_6H_3$ | O | 3-Cl-5-$CF_3$-pyridin-2-yl | |
| T-317 | $CF_3$ | 2-Cl-6-F-$C_6H_3$ | NH | 5-$CF_3$-pyridin-2-yl | |
| T-318 | $CF_3$ | 2-Cl-6-F-$C_6H_3$ | — | 4-$CF_3O$-$C_6H_4$ | |
| T-319 | $CF_3$ | 2-Cl-6-F-$C_6H_3$ | $CH(CH_3)O$ | 3-Cl-5-$CF_3$-pyridin-2-yl | |
| T-320 | $CF_3$ | 2-Cl-6-F-$C_6H_3$ | $CH(CH_3)O$ | 3,5-$Cl_2$-pyridin-2-yl | |
| T-321 | $CF_3$ | 2-Cl-6-F-$C_6H_3$ | $OCH_2$ | 3-Cl-5-$CF_3$-pyridin-2-yl | |
| T-322 | $CF_3$ | 2-Cl-6-F-$C_6H_3$ | $OCH(CH_3)$ | 3-Cl-5-$CF_3$-pyridin-2-yl | |
| T-323 | $CF_3$ | 2-Cl-6-F-$C_6H_3$ | CH(CN)O | 3-Cl-5-$CF_3$-pyridin-2-yl | |
| T-324 | $CF_3$ | 2-Cl-6-F-$C_6H_3$ | — | 3-Cl-5-$CF_3$-pyridin-2-yl | |

Table 17

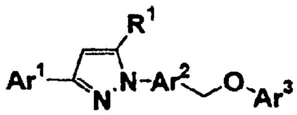

| No. | $R^1$ | $Ar^1$ | $Ar^2$ | $Ar^3$ | mp. |
|---|---|---|---|---|---|
| P-1 | $CH_3$ | 2-Cl-6-F-$C_6H_3$ | 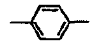 | 2-Cl-$C_6H_4$ | |
| P-2 | $CH_3$ | 2-Cl-6-F-$C_6H_3$ | 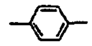 | 3-Cl-$C_6H_4$ | |
| P-3 | $CH_3$ | 2-Cl-6-F-$C_6H_3$ | 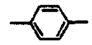 | 4-Cl-$C_6H_4$ | |
| P-4 | $CH_3$ | 2-Cl-6-F-$C_6H_3$ | 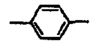 | 4-F-$C_6H_4$ | |
| P-5 | $CH_3$ | 2-Cl-6-F-$C_6H_3$ | 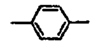 | 4-$CF_3$-$C_6H_4$ | |
| P-6 | $CH_3$ | 2-Cl-6-F-$C_6H_3$ | 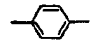 | 4-CN-$C_6H_4$ | |
| P-7 | $CH_3$ | 2-Cl-6-F-$C_6H_3$ | 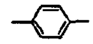 | 4-MeO-$C_6H_4$ | |
| P-8 | $CH_3$ | 2-Cl-6-F-$C_6H_3$ | 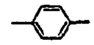 | 4-$NO_2$-$C_6H_4$ | |
| P-9 | $CH_3$ | 2-Cl-6-F-$C_6H_3$ | 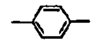 | 2,4-$Cl_2$-$C_6H_3$ | |
| P-10 | $CH_3$ | 2-Cl-6-F-$C_6H_3$ | 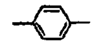 | 3,4-$Cl_2$-$C_6H_3$ | |
| P-11 | $CH_3$ | 2-Cl-6-F-$C_6H_3$ | 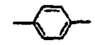 | 2,6-$Cl_2$-$C_6H_3$ | |
| P-12 | $CH_3$ | 2-Cl-6-F-$C_6H_3$ | 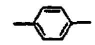 | 2-Cl-4-$CF_3$-$C_6H_3$ | |
| P-13 | $CH_3$ | 2-Cl-6-F-$C_6H_3$ | 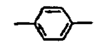 | 2-Cl-4-F-$C_6H_3$ | |
| P-14 | $CH_3$ | 2-Cl-6-F-$C_6H_3$ | 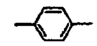 | 2,4,6-$Cl_3$-$C_6H_2$ | |
| P-15 | $CH_3$ | 2-Cl-6-F-$C_6H_3$ | 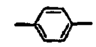 | 2,6-$Cl_2$-4-$CF_3$-$C_6H_2$ | |
| P-16 | $CH_3$ | 2-Cl-6-F-$C_6H_3$ | 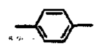 | 3-$CF_3$-pyridin-2-yl | |
| P-17 | $CH_3$ | 2-Cl-6-F-$C_6H_3$ | 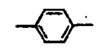 | 5-$CF_3$-pyridin-2-yl | |
| P-18 | $CH_3$ | 2-Cl-6-F-$C_6H_3$ | 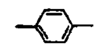 | 5-Cl-pyridin-2-yl | |
| P-19 | $CH_3$ | 2-Cl-6-F-$C_6H_3$ | 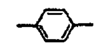 | 5-CN-pyridin-2-yl | |
| P-20 | $CH_3$ | 2-Cl-6-F-$C_6H_3$ | 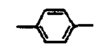 | 3,5-$Cl_2$-pyridin-2-yl | |
| P-21 | $CH_3$ | 2-Cl-6-F-$C_6H_3$ | 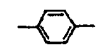 | 3-Cl-5-$CF_3$-pyridin-2-yl | 97-98 |
| P-22 | $CH_3$ | 2-Cl-6-F-$C_6H_3$ | 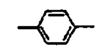 | 6-Cl-pyridazin-3-yl | |
| P-23 | $CH_3$ | 2-Cl-6-F-$C_6H_3$ | 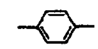 | 6-CN-pyridazin-3-yl | |
| P-24 | $CH_3$ | 2-Cl-6-F-$C_6H_3$ | 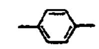 | 5-Cl-pyrimidin-2-yl | |
| P-25 | $CH_3$ | 2-Cl-6-F-$C_6H_3$ | 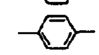 | 5-$CF_3$-1,3,4-thiadiazol-2-yl | |
| P-26 | $CH_3$ | 2-Cl-6-F-$C_6H_3$ | 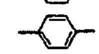 | 4'-Cl-biphenyl | |
| P-27 | $CH_3$ | 2-Cl-6-F-$C_6H_3$ | 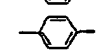 | 4-$Cl_2C$=CH-$C_6H_4$ | |
| P-28 | $CH_3$ | 2-Cl-6-F-$C_6H_3$ | 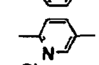 | 3-Cl-5-$CF_3$-pyridin-2-yl | |
| P-29 | $CH_3$ | 2-Cl-6-F-$C_6H_3$ | 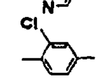 | 2,4,6-$Cl_3$-$C_6H_2$ | |

Table 18

| No. | $R^1$ | $Ar^1$ | $Ar^2$ | $Ar^3$ | mp. |
|---|---|---|---|---|---|
| P-30 | $CH_3$ | 2-Cl-6-F-$C_6H_3$ | 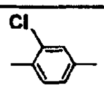 | 3-Cl-5-$CF_3$-pyridin-2-yl | |
| P-31 | $CH_3$ | 2-Cl-6-F-$C_6H_3$ | 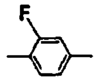 | 3-Cl-5-$CF_3$-pyridin-2-yl | |
| P-32 | $CH_3$ | 2-Cl-6-F-$C_6H_3$ | 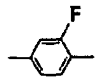 | 3-Cl-5-$CF_3$-pyridin-2-yl | |
| P-33 | $CH_3$ | 2-Cl-6-F-$C_6H_3$ | 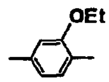 | 3-Cl-5-$CF_3$-pyridin-2-yl | |
| P-34 | $CH_3$ | 2-Cl-6-F-$C_6H_3$ | 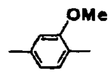 | 3-Cl-5-$CF_3$-pyridin-2-yl | |
| P-35 | $CH_3$ | 2-Cl-6-F-$C_6H_3$ | 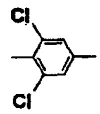 | 2,4,6-$Cl_3$-$C_6H_2$ | |
| P-36 | $CH_3$ | 2-Cl-6-F-$C_6H_3$ | 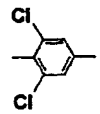 | 3-Cl-5-$CF_3$-pyridin-2-yl | |
| P-37 | H | 2-Cl-6-F-$C_6H_3$ | 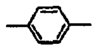 | 4-Cl-$C_6H_4$ | |
| P-38 | H | 2-Cl-6-F-$C_6H_3$ | 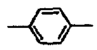 | 3-Cl-5-$CF_3$-pyridin-2-yl | oil[46)] |
| P-39 | $NH_2$ | 2-Cl-6-F-$C_6H_3$ | 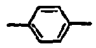 | 4-Cl-$C_6H_4$ | |
| P-40 | $NH_2$ | 2-Cl-6-F-$C_6H_3$ | 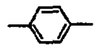 | 3-Cl-5-$CF_3$-pyridin-2-yl | |
| P-41 | $NMe_2$ | 2-Cl-6-F-$C_6H_3$ | 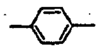 | 3-Cl-5-$CF_3$-pyridin-2-yl | |
| P-42 | Cl | 2-Cl-6-F-$C_6H_3$ | 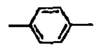 | 4-Cl-$C_6H_4$ | |
| P-43 | Cl | 2-Cl-6-F-$C_6H_3$ | 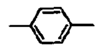 | 3-Cl-5-$CF_3$-pyridin-2-yl | 108-110 |
| P-44 | F | 2-Cl-6-F-$C_6H_3$ | 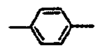 | 3-Cl-5-$CF_3$-pyridin-2-yl | |
| P-45 | MeO | 2-Cl-6-F-$C_6H_3$ | 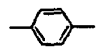 | 4-Cl-$C_6H_4$ | |
| P-46 | MeO | 2-Cl-6-F-$C_6H_3$ | 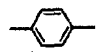 | 3-Cl-5-$CF_3$-pyridin-2-yl | oil[47)] |
| P-47 | $CF_3$ | 2-Cl-6-F-$C_6H_3$ | 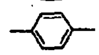 | 3-Cl-5-$CF_3$-pyridin-2-yl | |

46) 5.54 (2H, s), 6.66-6.67 (1H, m), 7.07-7.12 (1H, m), 7.25-7.31 (2H, m), 7.56-7.59 (2H, m), 7.76-7.79 (2H, m), 7.86-7.87 (1H, m), 8.01-8.02 (1H, m), 8.34-8.35 (1H, m)

47) 3.99 (3H, s), 5.53 (1H, s), 5.88 (1H, d *J*=0.9 Hz), 7.04-7.09 (1H, m), 7.23-7.30 (2H, m), 7.52-7.56 (2H, m), 7.78-7.82 (2H, m), 7.86 (1H, d *J*=2.2 Hz), 8.33-8.34 (1H, m)

Table 19

| No. | $R^1$ | $Ar^1$ | $Ar^2$ | $Ar^3$ | mp. |
|---|---|---|---|---|---|
| P-48 | Et | 2-Cl-6-F-$C_6H_3$ | 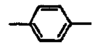 | 3-Cl-5-$CF_3$-pyridin-2-yl | |
| P-49 | MeS | 2-Cl-6-F-$C_6H_3$ | 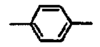 | 3-Cl-5-$CF_3$-pyridin-2-yl | oil[48)] |
| P-50 | MeSO | 2-Cl-6-F-$C_6H_3$ | 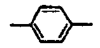 | 3-Cl-5-$CF_3$-pyridin-2-yl | |
| P-51 | $MeSO_2$ | 2-Cl-6-F-$C_6H_3$ | 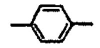 | 3-Cl-5-$CF_3$-pyridin-2-yl | |
| P-52 | CN | 2-Cl-6-F-$C_6H_3$ | 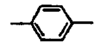 | 3-Cl-5-$CF_3$-pyridin-2-yl | 127-128 |
| P-53 | $CH_2Cl$ | 2-Cl-6-F-$C_6H_3$ | 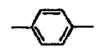 | 3-Cl-5-$CF_3$-pyridin-2-yl | |
| P-54 | $CH_2F$ | 2-Cl-6-F-$C_6H_3$ | 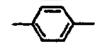 | 3-Cl-5-$CF_3$-pyridin-2-yl | |
| P-55 | $CH_2OH$ | 2-Cl-6-F-$C_6H_3$ | 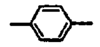 | 3-Cl-5-$CF_3$-pyridin-2-yl | |
| P-56 | $CH_2OCH_3$ | 2-Cl-6-F-$C_6H_3$ | 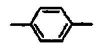 | 3-Cl-5-$CF_3$-pyridin-2-yl | |
| P-57 | $CH_2OCH_2OCH_3$ | 2-Cl-6-F-$C_6H_3$ | 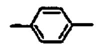 | 3-Cl-5-$CF_3$-pyridin-2-yl | |
| P-58 | $CH_2N(CH_3)_2$ | 2-Cl-6-F-$C_6H_3$ | 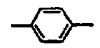 | 3-Cl-5-$CF_3$-pyridin-2-yl | |
| P-59 | $CH_2SCH_3$ | 2-Cl-6-F-$C_6H_3$ | 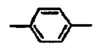 | 3-Cl-5-$CF_3$-pyridin-2-yl | |
| P-60 | $CH_2SO_2CH_3$ | 2-Cl-6-F-$C_6H_3$ | 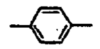 | 3-Cl-5-$CF_3$-pyridin-2-yl | |
| P-61 | $CH_2CN$ | 2-Cl-6-F-$C_6H_3$ | 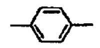 | 3-Cl-5-$CF_3$-pyridin-2-yl | |
| P-62 | $CH_3$ | 2-F-$C_6H_4$ | 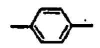 | 3-Cl-5-$CF_3$-pyridin-2-yl | |
| P-63 | $CH_2OCH_3$ | 2-F-$C_6H_4$ | 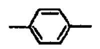 | 3-Cl-5-$CF_3$-pyridin-2-yl | |
| P-64 | $CH_3$ | 2-Cl-$C_6H_4$ | 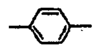 | 3-Cl-5-$CF_3$-pyridin-2-yl | |
| P-65 | $CH_3$ | 2,6-$F_2$-$C_6H_4$ | 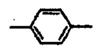 | 4-Cl-$C_6H_4$ | |
| P-66 | $CH_3$ | 2,6-$F_2$-$C_6H_4$ | 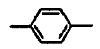 | 5-$CF_3$-pyridin-2-yl | |
| P-67 | $CH_3$ | 2,6-$F_2$-$C_6H_4$ | 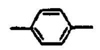 | 3-Cl-5-$CF_3$-pyridin-2-yl | |
| P-68 | $NH_2$ | 2,6-$F_2$-$C_6H_4$ | 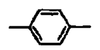 | 4-Cl-$C_6H_4$ | |
| P-69 | Cl | 2,6-$F_2$-$C_6H_4$ | 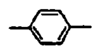 | 4-Cl-$C_6H_4$ | |
| P-70 | $CH_3$ | 2,6-$Cl_2$-$C_6H_4$ | 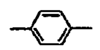 | 4-Cl-$C_6H_4$ | |
| P-71 | $CH_3$ | 2,6-$Cl_2$-$C_6H_4$ | 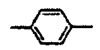 | 2,4-$Cl_2$-$C_6H_3$ | |
| P-72 | $CH_3$ | 2,6-$Cl_2$-$C_6H_4$ | 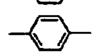 | 5-$CF_3$-pyridin-2-yl | |
| P-73 | $CH_3$ | 2,6-$Cl_2$-$C_6H_4$ | 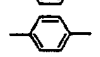 | 3-Cl-5-$CF_3$-pyridin-2-yl | |
| P-74 | $CH_3$ | 2,6-$Me_2$-$C_6H_4$ | 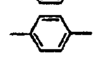 | 3-Cl-5-$CF_3$-pyridin-2-yl | |
| P-75 | H | 2,6-$Cl_2$-pyridin-4-yl | 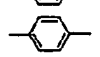 | 3-Cl-5-$CF_3$-pyridin-2-yl | |
| P-76 | Cl | 2-Cl-6-F-$C_6H_3$ | 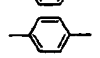 | 2-$CH_3SO_2$-5-$CF_3$-pyridin-3-yl | 132-135 |
| P-77 | Cl | 2-Cl-6-$SCH_3$-$C_6H_3$ | 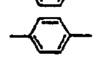 | 3-Cl-5-$CF_3$-pyridin-2-yl | 93-94 |

48) 2.45 (3H, s), 5.57 (1H, s), 6.53 (1H, s), 7.05-7.11 (1H, m), 7.24-7.31 (2H, m), 7.60 (2H, d $J$=8.3 Hz), 7.70 (2H, d $J$=8.3 Hz), 7.87-7.88 (1H, m), 8.34 (1H, br)

Table 20

| No. | $R^1$ | $Ar^1$ | $Ar^2$ | $Ar^3$ | mp. |
|---|---|---|---|---|---|
| P-78 | H | 2,6-$F_2$-$C_6H_3$ | 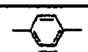 | 3-Cl-5-$CF_3$-pyridin-2-yl | |
| P-79 | $CF_3$ | 2,6-$F_2$-$C_6H_3$ | 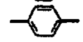 | 3-Cl-5-$CF_3$-pyridin-2-yl | |
| P-80 | Cl | 2,6-$F_2$-$C_6H_3$ | 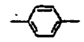 | 3-Cl-5-$CF_3$-pyridin-2-yl | |
| P-81 | CN | 2,6-$F_2$-$C_6H_3$ | 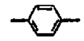 | 3-Cl-5-$CF_3$-pyridin-2-yl | |
| P-82 | $CH_3$ | 2-Cl-6-F-$C_6H_3$ | 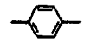 | 3,4,5,6-$Cl_4$-pyridin-2-yl | |
| P-83 | $CH_3$ | 2-Cl-6-F-$C_6H_3$ | 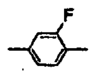 | 3,5-$Cl_2$-pyridin-2-yl | |
| P-84 | $CH_3$ | 2,6-$Cl_2$-$C_6H_3$ | 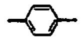 | 3,5-$Cl_2$-pyridin-2-yl | |
| P-85 | H | 2,6-$F_2$-$C_6H_3$ | 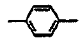 | 3,5-$Cl_2$-pyridin-2-yl | |
| P-86 | H | 2,6-$F_2$-$C_6H_3$ | 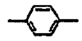 | 3-Cl-5-$CF_3$-pyridin-2-yl | |
| P-87 | $CH_3$ | 2,6-$F_2$-$C_6H_3$ | 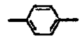 | 3,5-$Cl_2$-pyridin-2-yl | |
| P-88 | $CH_3$ | 2,6-$F_2$-$C_6H_3$ | 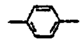 | 2-Cl-4-$CF_3$-$C_6H_3$ | |
| P-89 | $C_2H_5$ | 2,6-$F_2$-$C_6H_3$ | 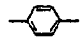 | 3,5-$Cl_2$-pyridin-2-yl | |
| P-90 | $C_2H_5$ | 2,6-$F_2$-$C_6H_3$ | 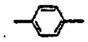 | 3-Cl-5-$CF_3$-pyridin-2-yl | |
| P-91 | $C_2H_5$ | 2,6-$F_2$-$C_6H_3$ | 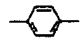 | 2-Cl-4-$CF_3$-$C_6H_3$ | |
| P-92 | $CF_3$ | 2,6-$F_2$-$C_6H_3$ | 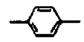 | 3,5-$Cl_2$-pyridin-2-yl | |
| P-93 | $CF_3$ | 2,6-$F_2$-$C_6H_4$ | 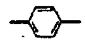 | 2-Cl-4-$CF_3$-$C_6H_3$ | |
| P-94 | *i*-$C_3H_7$ | 2-Cl-6-F-$C_6H_3$ | 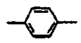 | 3-Cl-5-$CF_3$-pyridin-2-yl | |
| P-95 | *c*-$C_3H_5$ | 2-Cl-6-F-$C_6H_3$ | 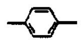 | 3-Cl-5-$CF_3$-pyridin-2-yl | |
| P-96 | $CHCl_2$ | 2-Cl-6-F-$C_6H_3$ | 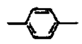 | 3-Cl-5-$CF_3$-pyridin-2-yl | |
| P-97 | $C_2H_5$ | 2-Cl-6-F-$C_6H_3$ | 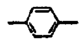 | 2-Cl-4-$CF_3$-$C_6H_3$ | |
| P-98 | $C_2F_5$ | 2-Cl-6-F-$C_6H_3$ | 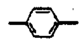 | 3-Cl-5-$CF_3$-pyridin-2-yl | |
| P-99 | Cl | 2,6-$F_2$-$C_6H_4$ | 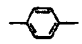 | 2-Cl-4-$CF_3$-$C_6H_3$ | |
| P-100 | Cl | 2,6-$F_2$-$C_6H_4$ | 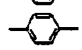 | 3,5-$Cl_2$-pyridin-2-yl | |
| P-101 | CN | 2,6-$F_2$-$C_6H_3$ | 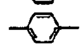 | 2-Cl-4-$CF_3$-$C_6H_3$ | |
| P-102 | $CF_3$ | 2,6-$F_2$-$C_6H_3$ | 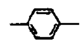 | 3,5-$Cl_2$-pyridin-2-yl | |
| P-103 | H | 2,6-$Cl_2$-$C_6H_3$ | 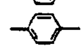 | 2-Cl-4-$CF_3$-$C_6H_3$ | |
| P-104 | H | 2,6-$Cl_2$-$C_6H_3$ | 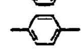 | 3,5-$Cl_2$-pyridin-2-yl | |
| P-105 | $CH_3$ | 2,6-$Cl_2$-$C_6H_3$ | 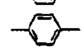 | 2-Cl-4-$CF_3$-$C_6H_3$ | |
| P-106 | $C_2H_5$ | 2,6-$Cl_2$-$C_6H_3$ | 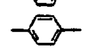 | 3,5-$Cl_2$-pyridin-2-yl | |
| P-107 | $C_2H_5$ | 2,6-$Cl_2$-$C_6H_3$ | 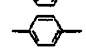 | 3-Cl-5-$CF_3$-pyridin-2-yl | |
| P-108 | $H_2C{=}CH$ | 2-Cl-6-F-$C_6H_3$ | 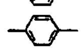 | 3-Cl-5-$CF_3$-pyridin-2-yl | |
| P-109 | CHO | 2-Cl-6-F-$C_6H_3$ | 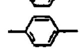 | 3-Cl-5-$CF_3$-pyridin-2-yl | |
| P-110 | $CH_3$ | 2-Cl-6-F-$C_6H_3$ | 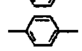 | 5-Cl-6-$CF_3$-pyrimidin-4-yl | |
| P-111 | $CH_3$ | 2-Cl-6-F-$C_6H_3$ | 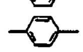 | 4-$CF_3$-pyrimidin-2-yl | |
| P-112 | $CH_3$ | 2-Cl-6-F-$C_6H_3$ | 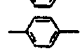 | 5-Cl-4-$CHF_2$-pyrimidin-6-yl | |
| P-113 | $C_2H_5$ | 2,6-$Cl_2$-$C_6H_3$ | 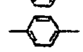 | 2-Cl-4-$CF_3$-$C_6H_3$ | |
| P-114 | $CF_3$ | 2,6-$Cl_2$-$C_6H_3$ | 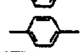 | 3-Cl-5-$CF_3$-pyridin-2-yl | |

Table 21

| No. | $R^1$ | $Ar^1$ | $Ar^2$ | $Ar^3$ | mp. |
|---|---|---|---|---|---|
| P-115 | $CF_3$ | 2,6-$Cl_2$-$C_6H_3$ | 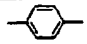 | 3,5-$Cl_2$-pyridin-2-yl | |
| P-116 | Cl | 2,6-$Cl_2$-$C_6H_3$ | 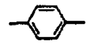 | 3,5-$Cl_2$-pyridin-2-yl | |
| P-117 | Cl | 2,6-$Cl_2$-$C_6H_3$ | 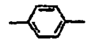 | 2-Cl-4-$CF_3$-$C_6H_3$ | |
| P-118 | CN | 2,6-$Cl_2$-$C_6H_3$ | 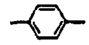 | 3,5-$Cl_2$-pyridin-2-yl | |
| P-119 | CN | 2,6-$Cl_2$-$C_6H_3$ | 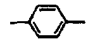 | 2-Cl-4-$CF_3$-$C_6H_3$ | |
| P-120 | $CF_3$ | 2,6-$Cl_2$-$C_6H_3$ | 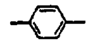 | 2-Cl-4-$CF_3$-$C_6H_3$ | |
| P-121 | $CH_3$ | 3-Cl-pyridin-2-yl | 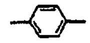 | 3-Cl-5-$CF_3$-pyridin-2-yl | |
| P-122 | $CHF_2$ | 2-Cl-6-F-$C_6H_3$ | 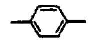 | 3-Cl-5-$CF_3$-pyridin-2-yl | |
| P-123 | H | 2-Cl-6-F-$C_6H_3$ | 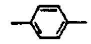 | 3,5-$Cl_2$-pyridin-2-yl | |
| P-124 | Cl | 2-Cl-6-F-$C_6H_3$ | 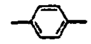 | 3,5-$Cl_2$-pyridin-2-yl | |
| P-125 | CN | 2-Cl-6-F-$C_6H_3$ | 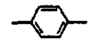 | 3,5-$Cl_2$-pyridin-2-yl | |
| P-126 | $CF_3$ | 2-Cl-6-F-$C_6H_3$ | 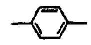 | 3,5-$Cl_2$-pyridin-2-yl | |
| P-127 | H | 2-Cl-6-F-$C_6H_3$ | 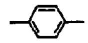 | 2-Cl-4-$CF_3$-$C_6H_3$ | |
| P-128 | Cl | 2-Cl-6-F-$C_6H_3$ | 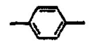 | 2-Cl-4-$CF_3$-$C_6H_3$ | |
| P-129 | CN | 2-Cl-6-F-$C_6H_3$ | 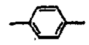 | 2-Cl-4-$CF_3$-$C_6H_3$ | |
| P-130 | $CF_3$ | 2-Cl-6-F-$C_6H_3$ | 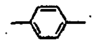 | 2-Cl-4-$CF_3$-$C_6H_3$ | |
| P-131 | $CH_3$ | 2-$CF_3$-$C_6H_4$ | 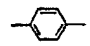 | 3-Cl-5-$CF_3$-pyridin-2-yl | |
| P-132 | $CH_3$ | 2-Cl-pyridin-3-yl | 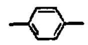 | 3-Cl-5-$CF_3$-pyridin-2-yl | |
| P-133 | $CH_3$ | 2,4,6-$Cl_3$-$C_6H_2$ | 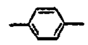 | 3-Cl-5-$CF_3$-pyridin-2-yl | |
| P-134 | HC≡C | 2-Cl-6-F-$C_6H_3$ | 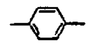 | 3-Cl-5-$CF_3$-pyridin-2-yl | |
| P-135 | $CH_3$ | 2-F-6-$CF_3$-$C_6H_3$ | 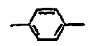 | 3-Cl-5-$CF_3$-pyridin-2-yl | |
| P-136 | $CH_3$ | 2,3,5,6-$F_4$-$C_6H_1$ | 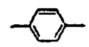 | 3-Cl-5-$CF_3$-pyridin-2-yl | |
| P-137 | $CH_3$ | 2,4,6-$F_3$-$C_6H_2$ | 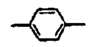 | 3-Cl-5-$CF_3$-pyridin-2-yl | |
| P-138 | $CH_3$ | 2-$OCF_3$-$C_6H_4$ | 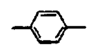 | 3-Cl-5-$CF_3$-pyridin-2-yl | |
| P-139 | $CH_3$ | 3-Cl-2,6-$F_2$-$C_6H_2$ | 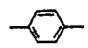 | 3-Cl-5-$CF_3$-pyridin-2-yl | |
| P-140 | $CH_3$ | 2-Cl-3,6-$F_2$-$C_6H_2$ | 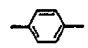 | 3-Cl-5-$CF_3$-pyridin-2-yl | |

Table 22

| No. | $R^1$ | $Ar^1$ | $Ar^2$ | $Ar^3$ | mp. |
|---|---|---|---|---|---|
| P-141 | $CH_3$ | 2-Cl-6-F-$C_6H_3$ | 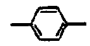 | 5-$CF_3$-pyrimidin-2-yl | |
| P-142 | $CH_3$ | 2,6-$(OCH_3)_2C_6H_3$ | 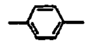 | 3-Cl-5-$CF_3$-pyridin-2-yl | |
| P-143 | $CH_3$ | 2-Cl-6-F-$C_6H_3$ | 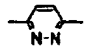 | 3-Cl-5-$CF_3$-pyridin-2-yl | |
| P-144 | $CH_3$ | 2,6-$(CF_3)_2C_6H_3$ | 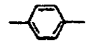 | 3-Cl-5-$CF_3$-pyridin-2-yl | |
| P-145 | $COOCH_3$ | 2-Cl-6-F-$C_6H_3$ | 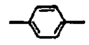 | 3-Cl-5-$CF_3$-pyridin-2-yl | |
| P-146 | $CH_3$ | 3-Cl-pyridin-2-yl | 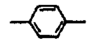 | 3,5-$Cl_2$-pyridin-2-yl | |
| P-147 | H | 3-Cl-pyridin-2-yl | 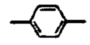 | 3,5-$Cl_2$-pyridin-2-yl | |
| P-148 | Cl | 3-Cl-pyridin-2-yl | 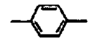 | 3,5-$Cl_2$-pyridin-2-yl | |
| P-149 | CN | 3-Cl-pyridin-2-yl | 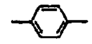 | 3,5-$Cl_2$-pyridin-2-yl | |
| P-150 | $CF_3$ | 3-Cl-pyridin-2-yl | 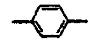 | 3,5-$Cl_2$-pyridin-2-yl | |
| P-151 | H | 3-Cl-pyridin-2-yl | 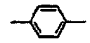 | 3-Cl-5-$CF_3$-pyridin-2-yl | |
| P-152 | Cl | 3-Cl-pyridin-2-yl | 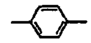 | 3-Cl-5-$CF_3$-pyridin-2-yl | |
| P-153 | CN | 3-Cl-pyridin-2-yl | 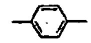 | 3-Cl-5-$CF_3$-pyridin-2-yl | |
| P-154 | $CF_3$ | 3-Cl-pyridin-2-yl | 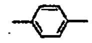 | 3-Cl-5-$CF_3$-pyridin-2-yl | |

Table 23

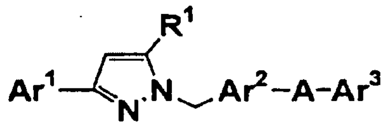

| No. | $R^1$ | $Ar^1$ | $Ar^2$ | A | $Ar^3$ | mp. |
|---|---|---|---|---|---|---|
| P-155 | $CH_3$ | 2-Cl-6-F-$C_6H_3$ | 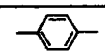 | — | 3-Cl-5-$CF_3$-pyridin-2-yl | |
| P-156 | $CH_3$ | 2-Cl-6-F-$C_6H_3$ | 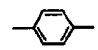 | O | 3-Cl-5-$CF_3$-pyridin-2-yl | |
| P-157 | $CH_3$ | 2-Cl-6-F-$C_6H_3$ | 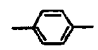 | $CH_2O$ | 3-Cl-5-$CF_3$-pyridin-2-yl | |
| P-158 | H | 2-Cl-6-F-$C_6H_3$ | 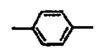 | — | 3-Cl-5-$CF_3$-pyridin-2-yl | |
| P-159 | H | 2-Cl-6-F-$C_6H_3$ | 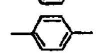 | O | 3-Cl-5-$CF_3$-pyridin-2-yl | |
| P-160 | H | 2-Cl-6-F-$C_6H_3$ | 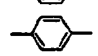 | $CH_2O$ | 3-Cl-5-$CF_3$-pyridin-2-yl | |
| P-161 | Cl | 2-Cl-6-F-$C_6H_3$ | 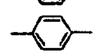 | — | 3-Cl-5-$CF_3$-pyridin-2-yl | |
| P-162 | Cl | 2-Cl-6-F-$C_6H_3$ | 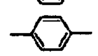 | O | 3-Cl-5-$CF_3$-pyridin-2-yl | |
| P-163 | Cl | 2-Cl-6-F-$C_6H_3$ | 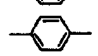 | $CH_2O$ | 3-Cl-5-$CF_3$-pyridin-2-yl | |
| P-164 | CN | 2-Cl-6-F-$C_6H_3$ | 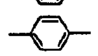 | — | 3-Cl-5-$CF_3$-pyridin-2-yl | |
| P-165 | CN | 2-Cl-6-F-$C_6H_3$ | 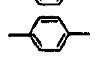 | O | 3-Cl-5-$CF_3$-pyridin-2-yl | |
| P-166 | CN | 2-Cl-6-F-$C_6H_3$ | 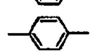 | $CH_2O$ | 3-Cl-5-$CF_3$-pyridin-2-yl | |
| P-167 | $CF_3$ | 2-Cl-6-F-$C_6H_3$ | 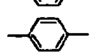 | — | 3-Cl-5-$CF_3$-pyridin-2-yl | |
| P-168 | $CF_3$ | 2-Cl-6-F-$C_6H_3$ | 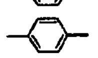 | O | 3-Cl-5-$CF_3$-pyridin-2-yl | |
| P-169 | $CF_3$ | 2-Cl-6-F-$C_6H_3$ | 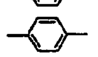 | $CH_2O$ | 3-Cl-5-$CF_3$-pyridin-2-yl | |
| P-170 | $CH_3$ | 2-Cl-6-F-$C_6H_3$ | 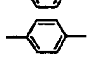 | — | 3,5-$Cl_2$-pyridin-2-yl | |
| P-171 | $CH_3$ | 2-Cl-6-F-$C_6H_3$ | 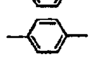 | O | 3,5-$Cl_2$-pyridin-2-yl | |
| P-172 | $CH_3$ | 2-Cl-6-F-$C_6H_3$ | 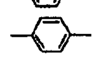 | $CH_2O$ | 3,5-$Cl_2$-pyridin-2-yl | |
| P-173 | H | 2-Cl-6-F-$C_6H_3$ | 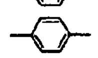 | — | 3,5-$Cl_2$-pyridin-2-yl | |
| P-174 | H | 2-Cl-6-F-$C_6H_3$ | 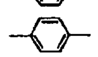 | O | 3,5-$Cl_2$-pyridin-2-yl | |
| P-175 | H | 2-Cl-6-F-$C_6H_3$ | 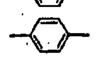 | $CH_2O$ | 3,5-$Cl_2$-pyridin-2-yl | |
| P-176 | Cl | 2-Cl-6-F-$C_6H_3$ | 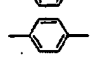 | — | 3,5-$Cl_2$-pyridin-2-yl | |
| P-177 | Cl | 2-Cl-6-F-$C_6H_3$ | 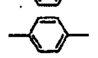 | O | 3,5-$Cl_2$-pyridin-2-yl | |
| P-178 | Cl | 2-Cl-6-F-$C_6H_3$ | 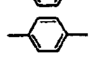 | $CH_2O$ | 3,5-$Cl_2$-pyridin-2-yl | |
| P-179 | CN | 2-Cl-6-F-$C_6H_3$ | 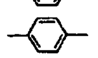 | — | 3,5-$Cl_2$-pyridin-2-yl | |
| P-180 | CN | 2-Cl-6-F-$C_6H_3$ | 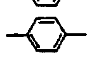 | O | 3,5-$Cl_2$-pyridin-2-yl | |
| P-181 | CN | 2-Cl-6-F-$C_6H_3$ | 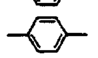 | $CH_2O$ | 3,5-$Cl_2$-pyridin-2-yl | |
| P-182 | $CF_3$ | 2-Cl-6-F-$C_6H_3$ | 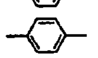 | — | 3,5-$Cl_2$-pyridin-2-yl | |
| P-183 | $CF_3$ | 2-Cl-6-F-$C_6H_3$ | 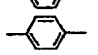 | O | 3,5-$Cl_2$-pyridin-2-yl | |

Table 24

| No. | $R^1$ | $Ar^1$ | $Ar^2$ | A | $Ar^3$ | mp. |
|---|---|---|---|---|---|---|
| P-184 | $CF_3$ | 2-Cl-6-F-$C_6H_3$ | 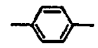 | $CH_2O$ | 3,5-$Cl_2$-pyridin-2-yl | |
| P-185 | $CH_3$ | 2,6-$Cl_2$-$C_6H_3$ | 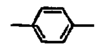 | — | 3,5-$Cl_2$-pyridin-2-yl | |
| P-186 | $CH_3$ | 2,6-$Cl_2$-$C_6H_3$ | 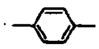 | O | 3,5-$Cl_2$-pyridin-2-yl | |
| P-187 | $CH_3$ | 2,6-$Cl_2$-$C_6H_3$ | 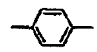 | $CH_2O$ | 3,5-$Cl_2$-pyridin-2-yl | |
| P-188 | H | 2,6-$Cl_2$-$C_6H_3$ | 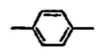 | — | 3,5-$Cl_2$-pyridin-2-yl | |
| P-189 | H | 2,6-$Cl_2$-$C_6H_3$ | 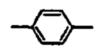 | O | 3,5-$Cl_2$-pyridin-2-yl | |
| P-190 | H | 2,6-$Cl_2$-$C_6H_3$ | 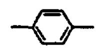 | $CH_2O$ | 3,5-$Cl_2$-pyridin-2-yl | |
| P-191 | Cl | 2,6-$Cl_2$-$C_6H_3$ | 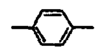 | — | 3,5-$Cl_2$-pyridin-2-yl | |
| P-192 | Cl | 2,6-$Cl_2$-$C_6H_3$ | 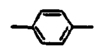 | O | 3,5-$Cl_2$-pyridin-2-yl | |
| P-193 | Cl | 2,6-$Cl_2$-$C_6H_3$ | 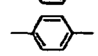 | $CH_2O$ | 3,5-$Cl_2$-pyridin-2-yl | |
| P-194 | CN | 2,6-$Cl_2$-$C_6H_3$ | 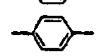 | — | 3,5-$Cl_2$-pyridin-2-yl | |
| P-195 | CN | 2,6-$Cl_2$-$C_6H_3$ | 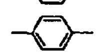 | O | 3,5-$Cl_2$-pyridin-2-yl | |
| P-196 | CN | 2,6-$Cl_2$-$C_6H_3$ | 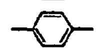 | $CH_2O$ | 3,5-$Cl_2$-pyridin-2-yl | |
| P-197 | $CF_3$ | 2,6-$Cl_2$-$C_6H_3$ | 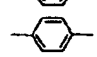 | — | 3,5-$Cl_2$-pyridin-2-yl | |
| P-198 | $CF_3$ | 2,6-$Cl_2$-$C_6H_3$ | 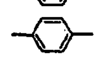 | O | 3,5-$Cl_2$-pyridin-2-yl | |
| P-199 | $CF_3$ | 2,6-$Cl_2$-$C_6H_3$ | 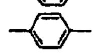 | $CH_2O$ | 3,5-$Cl_2$-pyridin-2-yl | |
| P-200 | $CH_3$ | 2,6-$Cl_2$-$C_6H_3$ | 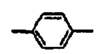 | — | 3-Cl-5-$CF_3$-pyridin-2-yl | |
| P-201 | $CH_3$ | 2,6-$Cl_2$-$C_6H_3$ | 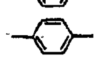 | O | 3-Cl-5-$CF_3$-pyridin-2-yl | |
| P-202 | $CH_3$ | 2,6-$Cl_2$-$C_6H_3$ | 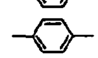 | $CH_2O$ | 3-Cl-5-$CF_3$-pyridin-2-yl | |
| P-203 | H | 2,6-$Cl_2$-$C_6H_3$ | 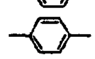 | — | 3-Cl-5-$CF_3$-pyridin-2-yl | |
| P-204 | H | 2,6-$Cl_2$-$C_6H_3$ | 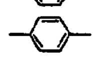 | O | 3-Cl-5-$CF_3$-pyridin-2-yl | |
| P-205 | H | 2,6-$Cl_2$-$C_6H_3$ | 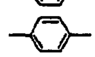 | $CH_2O$ | 3-Cl-5-$CF_3$-pyridin-2-yl | |
| P-206 | Cl | 2,6-$Cl_2$-$C_6H_3$ | 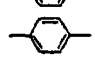 | — | 3-Cl-5-$CF_3$-pyridin-2-yl | |
| P-207 | Cl | 2,6-$Cl_2$-$C_6H_3$ | 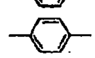 | O | 3-Cl-5-$CF_3$-pyridin-2-yl | |
| P-208 | Cl | 2,6-$Cl_2$-$C_6H_3$ | 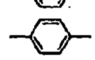 | $CH_2O$ | 3-Cl-5-$CF_3$-pyridin-2-yl | |
| P-209 | CN | 2,6-$Cl_2$-$C_6H_3$ | 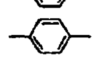 | — | 3-Cl-5-$CF_3$-pyridin-2-yl | |
| P-210 | CN | 2,6-$Cl_2$-$C_6H_3$ | 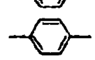 | O | 3-Cl-5-$CF_3$-pyridin-2-yl | |
| P-211 | CN | 2,6-$Cl_2$-$C_6H_3$ | 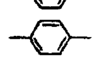 | $CH_2O$ | 3-Cl-5-$CF_3$-pyridin-2-yl | |
| P-212 | $CF_3$ | 2,6-$Cl_2$-$C_6H_3$ | 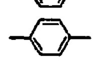 | — | 3-Cl-5-$CF_3$-pyridin-2-yl | |
| P-213 | $CF_3$ | 2,6-$Cl_2$-$C_6H_3$ | 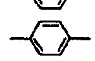 | O | 3-Cl-5-$CF_3$-pyridin-2-yl | |
| P-214 | $CF_3$ | 2,6-$Cl_2$-$C_6H_3$ | 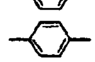 | $CH_2O$ | 3-Cl-5-$CF_3$-pyridin-2-yl | |
| P-215 | $CH_3$ | 2,6-$F_2$-$C_6H_3$ | 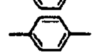 | — | 3-Cl-5-$CF_3$-pyridin-2-yl | |

Table 25

| No. | $R^1$ | $Ar^1$ | $Ar^2$ | A | $Ar^3$ | mp. |
|---|---|---|---|---|---|---|
| P-216 | $CH_3$ | 2,6-$F_2$-$C_6H_3$ | 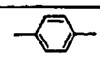 | O | 3-Cl-5-$CF_3$-pyridin-2-yl | |
| P-217 | $CH_3$ | 2,6-$F_2$-$C_6H_3$ | 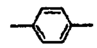 | $CH_2O$ | 3-Cl-5-$CF_3$-pyridin-2-yl | |
| P-218 | H | 2,6-$F_2$-$C_6H_3$ | 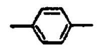 | — | 3-Cl-5-$CF_3$-pyridin-2-yl | |
| P-219 | H | 2,6-$F_2$-$C_6H_3$ | 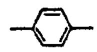 | O | 3-Cl-5-$CF_3$-pyridin-2-yl | |
| P-220 | H | 2,6-$F_2$-$C_6H_3$ | 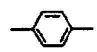 | $CH_2O$ | 3-Cl-5-$CF_3$-pyridin-2-yl | |
| P-221 | Cl | 2,6-$F_2$-$C_6H_3$ | 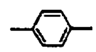 | — | 3-Cl-5-$CF_3$-pyridin-2-yl | |
| P-222 | Cl | 2,6-$F_2$-$C_6H_3$ | 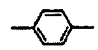 | O | 3-Cl-5-$CF_3$-pyridin-2-yl | |
| P-223 | Cl | 2,6-$F_2$-$C_6H_3$ | 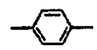 | $CH_2O$ | 3-Cl-5-$CF_3$-pyridin-2-yl | |
| P-224 | CN | 2,6-$F_2$-$C_6H_3$ | 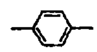 | — | 3-Cl-5-$CF_3$-pyridin-2-yl | |
| P-225 | CN | 2,6-$F_2$-$C_6H_3$ | 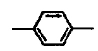 | O | 3-Cl-5-$CF_3$-pyridin-2-yl | |
| P-226 | CN | 2,6-$F_2$-$C_6H_3$ | 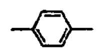 | $CH_2O$ | 3-Cl-5-$CF_3$-pyridin-2-yl | |
| P-227 | $CF_3$ | 2,6-$F_2$-$C_6H_3$ | 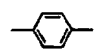 | — | 3-Cl-5-$CF_3$-pyridin-2-yl | |
| P-228 | $CF_3$ | 2,6-$F_2$-$C_6H_3$ | 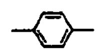 | O | 3-Cl-5-$CF_3$-pyridin-2-yl | |
| P-229 | $CF_3$ | 2,6-$F_2$-$C_6H_3$ | 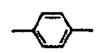 | $CH_2O$ | 3-Cl-5-$CF_3$-pyridin-2-yl | |
| P-230 | $CH_3$ | 2,6-$F_2$-$C_6H_3$ | 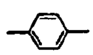 | | 3,5-$Cl_2$-pyridin-2-yl | |
| P-231 | $CH_3$ | 2,6-$F_2$-$C_6H_3$ | 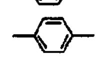 | O | 3,5-$Cl_2$-pyridin-2-yl | |
| P-232 | $CH_3$ | 2,6-$F_2$-$C_6H_3$ | 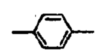 | $CH_2O$ | 3,5-$Cl_2$-pyridin-2-yl | |
| P-233 | H | 2,6-$F_2$-$C_6H_3$ | 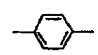 | — | 3,5-$Cl_2$-pyridin-2-yl | |
| P-234 | H | 2,6-$F_2$-$C_6H_3$ | 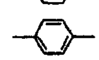 | O | 3,5-$Cl_2$-pyridin-2-yl | |
| P-235 | H | 2,6-$F_2$-$C_6H_3$ | 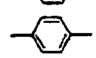 | $CH_2O$ | 3,5-$Cl_2$-pyridin-2-yl | |
| P-236 | Cl | 2,6-$F_2$-$C_6H_3$ | 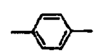 | — | 3,5-$Cl_2$-pyridin-2-yl | |
| P-237 | Cl | 2,6-$F_2$-$C_6H_3$ | 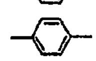 | O | 3,5-$Cl_2$-pyridin-2-yl | |
| P-238 | Cl | 2,6-$F_2$-$C_6H_3$ | 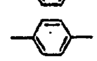 | $CH_2O$ | 3,5-$Cl_2$-pyridin-2-yl | |
| P-239 | CN | 2,6-$F_2$-$C_6H_3$ | 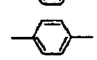 | — | 3,5-$Cl_2$-pyridin-2-yl | |
| P-240 | CN | 2,6-$F_2$-$C_6H_3$ | 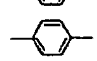 | O | 3,5-$Cl_2$-pyridin-2-yl | |
| P-241 | CN | 2,6-$F_2$-$C_6H_3$ | 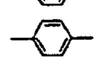 | $CH_2O$ | 3,5-$Cl_2$-pyridin-2-yl | |
| P-242 | $CF_3$ | 2,6-$F_2$-$C_6H_3$ | 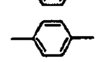 | — | 3,5-$Cl_2$-pyridin-2-yl | |
| P-243 | $CF_3$ | 2,6-$F_2$-$C_6H_3$ | 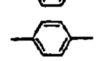 | O | 3,5-$Cl_2$-pyridin-2-yl | |
| P-244 | $CF_3$ | 2,6-$F_2$-$C_6H_3$ | 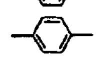 | $CH_2O$ | 3,5-$Cl_2$-pyridin-2-yl | |

Table 26

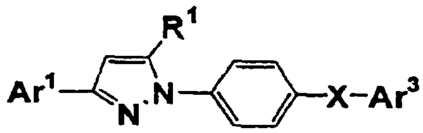

| No. | $R^1$ | $Ar^1$ | X | $Ar^3$ | mp |
|---|---|---|---|---|---|
| P-245 | $CH_3$ | 2-Cl-6-F-$C_6H_3$ | $CH_2S$ | 4-Cl-$C_6H_4$ | 124-125 |
| P-246 | $CH_3$ | 2-Cl-6-F-$C_6H_3$ | $CH_2NH$ | 4-Cl-$C_6H_4$ | |
| P-247 | $CH_3$ | 2-Cl-6-F-$C_6H_3$ | $OCH_2$ | 4-Cl-$C_6H_4$ | |
| P-248 | $CH_3$ | 2-Cl-6-F-$C_6H_3$ | $OCH_2$ | 2,4,6-$Cl_3$-$C_6H_2$ | |
| P-249 | $CH_3$ | 2-Cl-6-F-$C_6H_3$ | O | 3-Cl-5-$CF_3$-pyridin-2-yl | |
| P-250 | $CH_3$ | 2-Cl-6-F-$C_6H_3$ | NH | 5-$CF_3$-pyridin-2-yl | |
| P-251 | $CH_3$ | 2-Cl-6-F-$C_6H_3$ | — | 4-$CF_3O$-$C_6H_4$ | |
| P-252 | $CH_3$ | 2-Cl-6-F-$C_6H_3$ | $CH(CH_3)O$ | 3-Cl-5-$CF_3$-pyridin-2-yl | |
| P-253 | $CH_3$ | 2-Cl-6-F-$C_6H_3$ | $CH(CH_3)O$ | 3,5-$Cl_2$-pyridin-2-yl | |
| P-254 | $CH_3$ | 2-Cl-6-F-$C_6H_3$ | $OCH_2$ | 3-Cl-5-$CF_3$-pyridin-2-yl | |
| P-255 | $CH_3$ | 2-Cl-6-F-$C_6H_3$ | $OCH(CH_3)$ | 3-Cl-5-$CF_3$-pyridin-2-yl | |
| P-256 | $CH_3$ | 2-Cl-6-F-$C_6H_3$ | CH(CN)O | 3-Cl-5-$CF_3$-pyridin-2-yl | |
| P-257 | $CH_3$ | 2-Cl-6-F-$C_6H_3$ | — | 3-Cl-5-$CF_3$-pyridin-2-yl | |
| P-258 | Cl | 2-Cl-6-F-$C_6H_3$ | $CH_2S$ | 4-Cl-$C_6H_4$ | |
| P-259 | Cl | 2-Cl-6-F-$C_6H_3$ | $CH_2NH$ | 4-Cl-$C_6H_4$ | |
| P-260 | Cl | 2-Cl-6-F-$C_6H_3$ | $OCH_2$ | 4-Cl-$C_6H_4$ | |
| P-261 | Cl | 2-Cl-6-F-$C_6H_3$ | $OCH_2$ | 2,4,6-$Cl_3$-$C_6H_2$ | |
| P-262 | Cl | 2-Cl-6-F-$C_6H_3$ | O | 3-Cl-5-$CF_3$-pyridin-2-yl | |
| P-263 | Cl | 2-Cl-6-F-$C_6H_3$ | NH | 5-$CF_3$-pyridin-2-yl | |
| P-264 | Cl | 2-Cl-6-F-$C_6H_3$ | — | 4-$CF_3O$-$C_6H_4$ | |
| P-265 | Cl | 2-Cl-6-F-$C_6H_3$ | $CH(CH_3)O$ | 3-Cl-5-$CF_3$-pyridin-2-yl | |
| P-266 | Cl | 2-Cl-6-F-$C_6H_3$ | $CH(CH_3)O$ | 3,5-$Cl_2$-pyridin-2-yl | |
| P-267 | Cl | 2-Cl-6-F-$C_6H_3$ | $OCH_2$ | 3-Cl-5-$CF_3$-pyridin-2-yl | |
| P-268 | Cl | 2-Cl-6-F-$C_6H_3$ | $OCH(CH_3)$ | 3-Cl-5-$CF_3$-pyridin-2-yl | |
| P-269 | Cl | 2-Cl-6-F-$C_6H_3$ | CH(CN)O | 3-Cl-5-$CF_3$-pyridin-2-yl | |
| P-270 | Cl | 2-Cl-6-F-$C_6H_3$ | — | 3-Cl-5-$CF_3$-pyridin-2-yl | |

Table 27

| No. | $R^1$ | $Ar^1$ | X | $Ar^3$ | mp |
|---|---|---|---|---|---|
| P-271 | H | 2-Cl-6-F-$C_6H_3$ | $CH_2S$ | 4-Cl-$C_6H_4$ | |
| P-272 | H | 2-Cl-6-F-$C_6H_3$ | $CH_2NH$ | 4-Cl-$C_6H_4$ | |
| P-273 | H | 2-Cl-6-F-$C_6H_3$ | $OCH_2$ | 4-Cl-$C_6H_4$ | |
| P-274 | H | 2-Cl-6-F-$C_6H_3$ | $OCH_2$ | 2,4,6-$Cl_3$-$C_6H_2$ | |
| P-275 | H | 2-Cl-6-F-$C_6H_3$ | O | 3-Cl-5-$CF_3$-pyridin-2-yl | |
| P-276 | H | 2-Cl-6-F-$C_6H_3$ | NH | 5-$CF_3$-pyridin-2-yl | |
| P-277 | H | 2-Cl-6-F-$C_6H_3$ | — | 4-$CF_3O$-$C_6H_4$ | |
| P-278 | H | 2-Cl-6-F-$C_6H_3$ | $CH(CH_3)O$ | 3-Cl-5-$CF_3$-pyridin-2-yl | |
| P-279 | H | 2-Cl-6-F-$C_6H_3$ | $CH(CH_3)O$ | 3,5-$Cl_2$-pyridin-2-yl | |
| P-280 | H | 2-Cl-6-F-$C_6H_3$ | $OCH_2$ | 3-Cl-5-$CF_3$-pyridin-2-yl | |
| P-281 | H | 2-Cl-6-F-$C_6H_3$ | $OCH(CH_3)$ | 3-Cl-5-$CF_3$-pyridin-2-yl | |
| P-282 | H | 2-Cl-6-F-$C_6H_3$ | CH(CN)O | 3-Cl-5-$CF_3$-pyridin-2-yl | |
| P-283 | H | 2-Cl-6-F-$C_6H_3$ | — | 3-Cl-5-$CF_3$-pyridin-2-yl | |
| P-284 | CN | 2-Cl-6-F-$C_6H_3$ | $CH_2S$ | 4-Cl-$C_6H_4$ | |
| P-285 | CN | 2-Cl-6-F-$C_6H_3$ | $CH_2NH$ | 4-Cl-$C_6H_4$ | |
| P-286 | CN | 2-Cl-6-F-$C_6H_3$ | $OCH_2$ | 4-Cl-$C_6H_4$ | |
| P-287 | CN | 2-Cl-6-F-$C_6H_3$ | $OCH_2$ | 2,4,6-$Cl_3$-$C_6H_2$ | |
| P-288 | CN | 2-Cl-6-F-$C_6H_3$ | O | 3-Cl-5-$CF_3$-pyridin-2-yl | |
| P-289 | CN | 2-Cl-6-F-$C_6H_3$ | NH | 5-$CF_3$-pyridin-2-yl | |
| P-290 | CN | 2-Cl-6-F-$C_6H_3$ | — | 4-$CF_3O$-$C_6H_4$ | |
| P-291 | CN | 2-Cl-6-F-$C_6H_3$ | $CH(CH_3)O$ | 3-Cl-5-$CF_3$-pyridin-2-yl | |
| P-292 | CN | 2-Cl-6-F-$C_6H_3$ | $CH(CH_3)O$ | 3,5-$Cl_2$-pyridin-2-yl | |
| P-293 | CN | 2-Cl-6-F-$C_6H_3$ | $OCH_2$ | 3-Cl-5-$CF_3$-pyridin-2-yl | |
| P-294 | CN | 2-Cl-6-F-$C_6H_3$ | $OCH(CH_3)$ | 3-Cl-5-$CF_3$-pyridin-2-yl | |
| P-295 | CN | 2-Cl-6-F-$C_6H_3$ | CH(CN)O | 3-Cl-5-$CF_3$-pyridin-2-yl | |
| P-296 | CN | 2-Cl-6-F-$C_6H_3$ | — | 3-Cl-5-$CF_3$-pyridin-2-yl | |
| P-297 | $CF_3$ | 2-Cl-6-F-$C_6H_3$ | $CH_2S$ | 4-Cl-$C_6H_4$ | |
| P-298 | $CF_3$ | 2-Cl-6-F-$C_6H_3$ | $CH_2NH$ | 4-Cl-$C_6H_4$ | |
| P-299 | $CF_3$ | 2-Cl-6-F-$C_6H_3$ | $OCH_2$ | 4-Cl-$C_6H_4$ | |
| P-300 | $CF_3$ | 2-Cl-6-F-$C_6H_3$ | $OCH_2$ | 2,4,6-$Cl_3$-$C_6H_2$ | |
| P-301 | $CF_3$ | 2-Cl-6-F-$C_6H_3$ | O | 3-Cl-5-$CF_3$-pyridin-2-yl | |
| P-302 | $CF_3$ | 2-Cl-6-F-$C_6H_3$ | NH | 5-$CF_3$-pyridin-2-yl | |
| P-303 | $CF_3$ | 2-Cl-6-F-$C_6H_3$ | — | 4-$CF_3O$-$C_6H_4$ | |
| P-304 | $CF_3$ | 2-Cl-6-F-$C_6H_3$ | $CH(CH_3)O$ | 3-Cl-5-$CF_3$-pyridin-2-yl | |
| P-305 | $CF_3$ | 2-Cl-6-F-$C_6H_3$ | $CH(CH_3)O$ | 3,5-$Cl_2$-pyridin-2-yl | |
| P-306 | $CF_3$ | 2-Cl-6-F-$C_6H_3$ | $OCH_2$ | 3-Cl-5-$CF_3$-pyridin-2-yl | |
| P-307 | $CF_3$ | 2-Cl-6-F-$C_6H_3$ | $OCH(CH_3)$ | 3-Cl-5-$CF_3$-pyridin-2-yl | |
| P-308 | $CF_3$ | 2-Cl-6-F-$C_6H_3$ | CH(CN)O | 3-Cl-5-$CF_3$-pyridin-2-yl | |
| P-309 | $CF_3$ | 2-Cl-6-F-$C_6H_3$ | — | 3-Cl-5-$CF_3$-pyridin-2-yl | |

Table 28

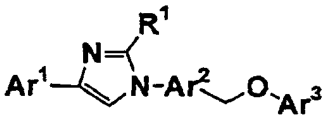

| No. | $R^1$ | $Ar^1$ | $Ar^2$ | $Ar^3$ | mp. |
|---|---|---|---|---|---|
| I-1 | $CH_3$ | 2-Cl-6-F-$C_6H_3$ | 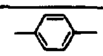 | 2-Cl-$C_6H_4$ | |
| I-2 | $CH_3$ | 2-Cl-6-F-$C_6H_3$ | 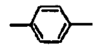 | 3-Cl-$C_6H_4$ | |
| I-3 | $CH_3$ | 2-Cl-6-F-$C_6H_3$ | 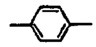 | 4-Cl-$C_6H_4$ | |
| I-4 | $CH_3$ | 2-Cl-6-F-$C_6H_3$ | 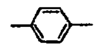 | 4-F-$C_6H_4$ | |
| I-5 | $CH_3$ | 2-Cl-6-F-$C_6H_3$ | 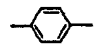 | 4-$CF_3$-$C_6H_4$ | |
| I-6 | $CH_3$ | 2-Cl-6-F-$C_6H_3$ | 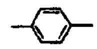 | 4-CN-$C_6H_4$ | |
| I-7 | $CH_3$ | 2-Cl-6-F-$C_6H_3$ | 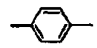 | 4-MeO-$C_6H_4$ | |
| I-8 | $CH_3$ | 2-Cl-6-F-$C_6H_3$ | 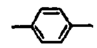 | 4-$NO_2$-$C_6H_4$ | |
| I-9 | $CH_3$ | 2-Cl-6-F-$C_6H_3$ | 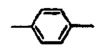 | 2,4-$Cl_2$-$C_6H_3$ | |
| I-10 | $CH_3$ | 2-Cl-6-F-$C_6H_3$ | 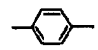 | 3,4-$Cl_2$-$C_6H_3$ | |
| I-11 | $CH_3$ | 2-Cl-6-F-$C_6H_3$ | 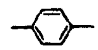 | 2,6-$Cl_2$-$C_6H_3$ | |
| I-12 | $CH_3$ | 2-Cl-6-F-$C_6H_3$ | 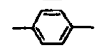 | 2-Cl-4-$CF_3$-$C_6H_3$ | |
| I-13 | $CH_3$ | 2-Cl-6-F-$C_6H_3$ | 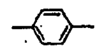 | 2-Cl-4-F-$C_6H_3$ | |
| I-14 | $CH_3$ | 2-Cl-6-F-$C_6H_3$ | 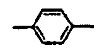 | 2,4,6-$Cl_3$-$C_6H_2$ | |
| I-15 | $CH_3$ | 2-Cl-6-F-$C_6H_3$ | 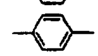 | 2,6-$Cl_2$-4-$CF_3$-$C_6H_2$ | |
| I-16 | $CH_3$ | 2-Cl-6-F-$C_6H_3$ | 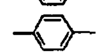 | 3-$CF_3$-pyridin-2-yl | |
| I-17 | $CH_3$ | 2-Cl-6-F-$C_6H_3$ | 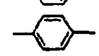 | 5-$CF_3$-pyridin-2-yl | |
| I-18 | $CH_3$ | 2-Cl-6-F-$C_6H_3$ | 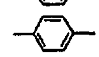 | 5-Cl-pyridin-2-yl | |
| I-19 | $CH_3$ | 2-Cl-6-F-$C_6H_3$ | 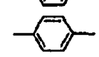 | 5-CN-pyridin-2-yl | |
| I-20 | $CH_3$ | 2-Cl-6-F-$C_6H_3$ | 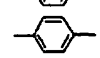 | 3,5-$Cl_2$-pyridin-2-yl | |
| I-21 | $CH_3$ | 2-Cl-6-F-$C_6H_3$ | 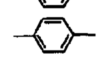 | 3-Cl-5-$CF_3$-pyridin-2-yl | |
| I-22 | $CH_3$ | 2-Cl-6-F-$C_6H_3$ | 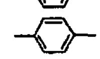 | 6-Cl-pyridazin-3-yl | |
| I-23 | $CH_3$ | 2-Cl-6-F-$C_6H_3$ | 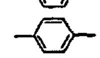 | 6-CN-pyridazin-3-yl | |
| I-24 | $CH_3$ | 2-Cl-6-F-$C_6H_3$ | 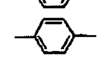 | 5-Cl-pyrimidin-2-yl | |
| I-25 | $CH_3$ | 2-Cl-6-F-$C_6H_3$ | 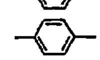 | 5-$CF_3$-1,3,4-thiadiazol-2-yl | |
| I-26 | $CH_3$ | 2-Cl-6-F-$C_6H_3$ | 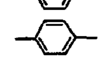 | 4`-Cl-biphenyl | |
| I-27 | $CH_3$ | 2-Cl-6-F-$C_6H_3$ | 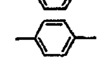 | 4-$Cl_2C$=CH-$C_6H_4$ | |
| I-28 | $CH_3$ | 2-Cl-6-F-$C_6H_3$ | 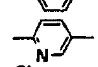 | 3-Cl-5-$CF_3$-pyridin-2-yl | |
| I-29 | $CH_3$ | 2-Cl-6-F-$C_6H_3$ | 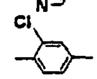 | 2,4,6-$Cl_3$-$C_6H_2$ | |

Table 29

| No. | $R^1$ | $Ar^1$ | $Ar^2$ | $Ar^3$ | mp. |
|---|---|---|---|---|---|
| I-30 | $CH_3$ | 2-Cl-6-F-$C_6H_3$ | 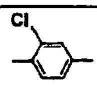 | 2,4,6-$Cl_3$-$C_6H_2$ | |
| I-31 | $CH_3$ | 2-Cl-6-F-$C_6H_3$ | 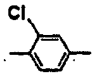 | 3-Cl-5-$CF_3$-pyridin-2-yl | |
| I-32 | $CH_3$ | 2-Cl-6-F-$C_6H_3$ | 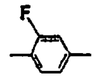 | 3-Cl-5-$CF_3$-pyridin-2-yl | |
| I-33 | $CH_3$ | 2-Cl-6-F-$C_6H_3$ | 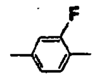 | 3-Cl-5-$CF_3$-pyridin-2-yl | |
| I-34 | $CH_3$ | 2-Cl-6-F-$C_6H_3$ | 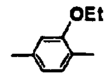 | 3-Cl-5-$CF_3$-pyridin-2-yl | |
| I-35 | $CH_3$ | 2-Cl-6-F-$C_6H_3$ | 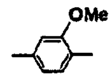 | 3-Cl-5-$CF_3$-pyridin-2-yl | |
| I-36 | $CH_3$ | 2-Cl-6-F-$C_6H_3$ | 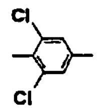 | 2,4,6-$Cl_3$-$C_6H_2$ | |
| I-37 | $CH_3$ | 2-Cl-6-F-$C_6H_3$ | 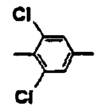 | 3-Cl-5-$CF_3$-pyridin-2-yl | |
| I-38 | H | 2-Cl-6-F-$C_6H_3$ | 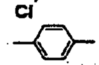 | 4-Cl-$C_6H_4$ | |
| I-39 | H | 2-Cl-6-F-$C_6H_3$ | 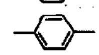 | 3-Cl-5-$CF_3$-pyridin-2-yl | |
| I-40 | $NH_2$ | 2-Cl-6-F-$C_6H_3$ | 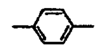 | 4-Cl-$C_6H_4$ | |
| I-41 | $NH_2$ | 2-Cl-6-F-$C_6H_3$ | 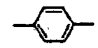 | 3-Cl-5-$CF_3$-pyridin-2-yl | |
| I-42 | $NMe_2$ | 2-Cl-6-F-$C_6H_3$ | 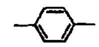 | 3-Cl-5-$CF_3$-pyridin-2-yl | |
| I-43 | Cl | 2-Cl-6-F-$C_6H_3$ | 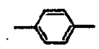 | 4-Cl-$C_6H_4$ | |
| I-44 | Cl | 2-Cl-6-F-$C_6H_3$ | 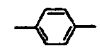 | 3-Cl-5-$CF_3$-pyridin-2-yl | |
| I-45 | F | 2-Cl-6-F-$C_6H_3$ | 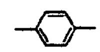 | 3-Cl-5-$CF_3$-pyridin-2-yl | |
| I-46 | MeO | 2-Cl-6-F-$C_6H_3$ | 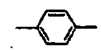 | 4-Cl-$C_6H_4$ | |
| I-47 | MeO | 2-Cl-6-F-$C_6H_3$ | 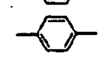 | 3-Cl-5-$CF_3$-pyridin-2-yl | |
| I-48 | $CF_3$ | 2-Cl-6-F-$C_6H_3$ | 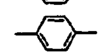 | 3-Cl-5-$CF_3$-pyridin-2-yl | |
| I-49 | Et | 2-Cl-6-F-$C_6H_3$ | 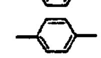 | 3-Cl-5-$CF_3$-pyridin-2-yl | |
| I-50 | MeS | 2-Cl-6-F-$C_6H_3$ | 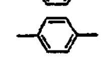 | 3-Cl-5-$CF_3$-pyridin-2-yl | |
| I-51 | MeSO | 2-Cl-6-F-$C_6H_3$ | 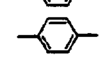 | 3-Cl-5-$CF_3$-pyridin-2-yl | |
| I-52 | $MeSO_2$ | 2-Cl-6-F-$C_6H_3$ | 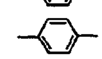 | 3-Cl-5-$CF_3$-pyridin-2-yl | |
| I-53 | CN | 2-Cl-6-F-$C_6H_3$ | 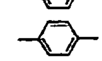 | 3-Cl-5-$CF_3$-pyridin-2-yl | |
| I-54 | $CH_2Cl$ | 2-Cl-6-F-$C_6H_3$ | 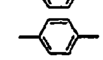 | 3-Cl-5-$CF_3$-pyridin-2-yl | |

Table 30

| No. | $R^1$ | $Ar^1$ | $Ar^2$ | $Ar^3$ | mp. |
|---|---|---|---|---|---|
| I-55 | $CH_2F$ | 2-Cl-6-F-$C_6H_3$ | 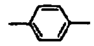 | 3-Cl-5-$CF_3$-pyridin-2-yl | |
| I-56 | $CH_2OH$ | 2-Cl-6-F-$C_6H_3$ | 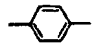 | 3-Cl-5-$CF_3$-pyridin-2-yl | |
| I-57 | $CH_2OCH_3$ | 2-Cl-6-F-$C_6H_3$ | 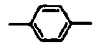 | 3-Cl-5-$CF_3$-pyridin-2-yl | |
| I-58 | $CH_2OCH_2OCH_3$ | 2-Cl-6-F-$C_6H_3$ | 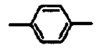 | 3-Cl-5-$CF_3$-pyridin-2-yl | |
| I-59 | $CH_2N(CH_3)_2$ | 2-Cl-6-F-$C_6H_3$ | 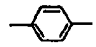 | 3-Cl-5-$CF_3$-pyridin-2-yl | |
| I-60 | $CH_2SCH_3$ | 2-Cl-6-F-$C_6H_3$ | 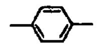 | 3-Cl-5-$CF_3$-pyridin-2-yl | |
| I-61 | $CH_2SO_2CH_3$ | 2-Cl-6-F-$C_6H_3$ | 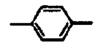 | 3-Cl-5-$CF_3$-pyridin-2-yl | |
| I-62 | $CH_2CN$ | 2-Cl-6-F-$C_6H_3$ | 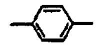 | 3-Cl-5-$CF_3$-pyridin-2-yl | |
| I-63 | $CH_3$ | 2-F-$C_6H_4$ | 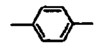 | 3-Cl-5-$CF_3$-pyridin-2-yl | 109 |
| I-64 | $CH_2OCH_3$ | 2-F-$C_6H_4$ | 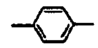 | 3-Cl-5-$CF_3$-pyridin-2-yl | 77-78 |
| I-65 | $CH_3$ | 2-Cl-$C_6H_4$ | 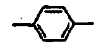 | 3-Cl-5-$CF_3$-pyridin-2-yl | 124-125 |
| I-66 | $CH_3$ | 2,6-$F_2$-$C_6H_4$ | 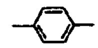 | 4-Cl-$C_6H_4$ | |
| I-67 | $CH_3$ | 2,6-$F_2$-$C_6H_4$ | 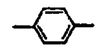 | 5-$CF_3$-pyridin-2-yl | |
| I-68 | $CH_3$ | 2,6-$F_2$-$C_6H_4$ | 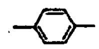 | 3-Cl-5-$CF_3$-pyridin-2-yl | |
| I-69 | $NH_2$ | 2,6-$F_2$-$C_6H_4$ | 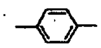 | 4-Cl-$C_6H_4$ | |
| I-70 | Cl | 2,6-$F_2$-$C_6H_4$ | 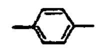 | 4-Cl-$C_6H_4$ | |
| I-71 | $CH_3$ | 2,6-$Cl_2$-$C_6H_4$ | 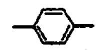 | 4-Cl-$C_6H_4$ | |
| I-72 | $CH_3$ | 2,6-$Cl_2C_6H_4$ | 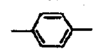 | 2,4-$Cl_2$-$C_6H_3$ | |
| I-73 | $CH_3$ | 2,6-$Cl_2C_6H_4$ | 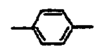 | 5-$CF_3$-pyridin-2-yl | |
| I-74 | $CH_3$ | 2,6-$Cl_2C_6H_4$ | 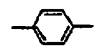 | 3-Cl-5-$CF_3$-pyridin-2-yl | |
| I-75 | $CH_3$ | 2,6-$Me_2$-$C_6H_4$ | 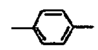 | 3-Cl-5-$CF_3$-pyridin-2-yl | |
| I-76 | H | 2,6-$Cl_2$-pyridin-4-yl | 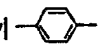 | 3-Cl-5-$CF_3$-pyridin-2-yl | |
| I-77 | Cl | 2-F-$C_6H_4$ | 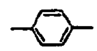 | 3-Cl-5-$CF_3$-pyridin-2-yl | 73-76 |
| I-78 | $CH_2OCH_3$ | 2-F-$C_6H_4$ | 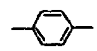 | 3,5-$Cl_2$-pyridin-2-yl | 148-149 |
| I-79 | Cl | 2-F-$C_6H_4$ | 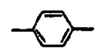 | 3,5-$Cl_2$-pyridin-2-yl | 147-149 |
| I-80 | $CH_2OCH_2OCH_3$ | 2-F-$C_6H_4$ | 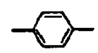 | 3-Cl-5-$CF_3$-pyridin-2-yl | 128-129 |
| I-81 | CN | 2-F-$C_6H_4$ | 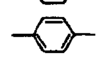 | 3-Cl-5-$CF_3$-pyridin-2-yl | 162-163 |
| I-82 | CHO | 2-F-$C_6H_4$ | 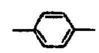 | 3-Cl-5-$CF_3$-pyridin-2-yl | 163-164 |
| I-83 | $CH_2OH$ | 2-F-$C_6H_4$ | 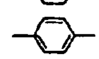 | 3-Cl-5-$CF_3$-pyridin-2-yl | 148-150 |
| I-84 | $CH_3$ | 2-Cl-$C_6H_4$ | 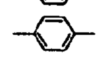 | 3,5-$Cl_2$-pyridin-2-yl | 165-166 |

Table 31

| No. | $R^1$ | $Ar^1$ | $Ar^2$ | $Ar^3$ | mp. |
|---|---|---|---|---|---|
| I-85 | H | 2,6-$F_2$-$C_6H_3$ | 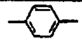 | 3-Cl-5-$CF_3$-pyridin-2-yl | |
| I-86 | $CF_3$ | 2,6-$F_2$-$C_6H_3$ | 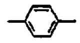 | 3-Cl-5-$CF_3$-pyridin-2-yl | |
| I-87 | Cl | 2,6-$F_2$-$C_6H_3$ | 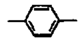 | 3-Cl-5-$CF_3$-pyridin-2-yl | |
| I-88 | CN | 2,6-$F_2$-$C_6H_3$ | 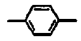 | 3-Cl-5-$CF_3$-pyridin-2-yl | |
| I-89 | $CH_3$ | 2-Cl-6-F-$C_6H_3$ | 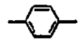 | 3,4,5,6-$Cl_4$-pyridin-2-yl | |
| I-90 | $CH_3$ | 2-Cl-6-F-$C_6H_3$ | 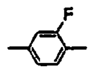 | 3,5-$Cl_2$-pyridin-2-yl | |
| I-91 | $CH_3$ | 2,6-$Cl_2$-$C_6H_3$ | 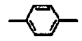 | 3,5-$Cl_2$-pyridin-2-yl | |
| I-92 | H | 2,6-$F_2$-$C_6H_3$ | 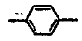 | 3,5-$Cl_2$-pyridin-2-yl | |
| I-93 | H | 2,6-$F_2$-$C_6H_3$ | 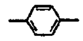 | 3-Cl-5-$CF_3$-pyridin-2-yl | |
| I-94 | $CH_3$ | 2,6-$F_2$-$C_6H_3$ | 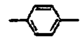 | 3,5-$Cl_2$-pyridin-2-yl | |
| I-95 | $CH_3$ | 2,6-$F_2$-$C_6H_3$ | 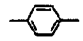 | 2-Cl-4-$CF_3$-$C_6H_3$ | |
| I-96 | $C_2H_5$ | 2,6-$F_2$-$C_6H_3$ | 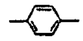 | 3,5-$Cl_2$-pyridin-2-yl | |
| I-97 | $C_2H_5$ | 2,6-$F_2$-$C_6H_3$ | 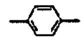 | 3-Cl-5-$CF_3$-pyridin-2-yl | |
| I-98 | $C_2H_5$ | 2,6-$F_2$-$C_6H_3$ | 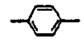 | 2-Cl-4-$CF_3$-$C_6H_3$ | |
| I-99 | $CF_3$ | 2,6-$F_2$-$C_6H_3$ | 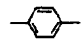 | 3,5-$Cl_2$-pyridin-2-yl | |
| I-100 | $CF_3$ | 2,6-$F_2$-$C_6H_4$ | 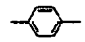 | 2-Cl-4-$CF_3$-$C_6H_3$ | |
| I-101 | *i*-$C_3H_7$ | 2-Cl-6-F-$C_6H_3$ | 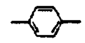 | 3-Cl-5-$CF_3$-pyridin-2-yl | |
| I-102 | *c*-$C_3H_5$ | 2-Cl-6-F-$C_6H_3$ | 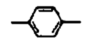 | 3-Cl-5-$CF_3$-pyridin-2-yl | |
| I-103 | $CHCl_2$ | 2-Cl-6-F-$C_6H_3$ | 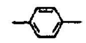 | 3-Cl-5-$CF_3$-pyridin-2-yl | |
| I-104 | $C_2H_5$ | 2-Cl-6-F-$C_6H_3$ | 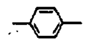 | 2-Cl-4-$CF_3$-$C_6H_3$ | |
| I-105 | $C_2F_5$ | 2-Cl-6-F-$C_6H_3$ | 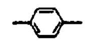 | 3-Cl-5-$CF_3$-pyridin-2-yl | |
| I-106 | Cl | 2,6-$F_2$-$C_6H_4$ | 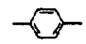 | 2-Cl-4-$CF_3$-$C_6H_3$ | |
| I-107 | Cl | 2,6-$F_2$-$C_6H_4$ | 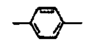 | 3,5-$Cl_2$-pyridin-2-yl | |
| I-108 | CN | 2,6-$F_2$-$C_6H_3$ | 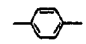 | 2-Cl-4-$CF_3$-$C_6H_3$ | |
| I-109 | $CF_3$ | 2,6-$F_2$-$C_6H_3$ | 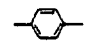 | 3,5-$Cl_2$-pyridin-2-yl | |
| I-110 | H | 2,6-$Cl_2$-$C_6H_3$ | 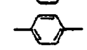 | 2-Cl-4-$CF_3$-$C_6H_3$ | |
| I-111 | H | 2,6-$Cl_2$-$C_6H_3$ | 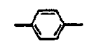 | 3,5-$Cl_2$-pyridin-2-yl | |
| I-112 | $CH_3$ | 2,6-$Cl_2$-$C_6H_3$ | 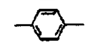 | 2-Cl-4-$CF_3$-$C_6H_3$ | |
| I-113 | $C_2H_5$ | 2,6-$Cl_2$-$C_6H_3$ | 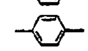 | 3,5-$Cl_2$-pyridin-2-yl | |
| I-114 | $C_2H_5$ | 2,6-$Cl_2$-$C_6H_3$ | 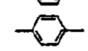 | 3-Cl-5-$CF_3$-pyridin-2-yl | |
| I-115 | $H_2C$=CH | 2-Cl-6-F-$C_6H_3$ | 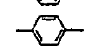 | 3-Cl-5-$CF_3$-pyridin-2-yl | |
| I-116 | CHO | 2-Cl-6-F-$C_6H_3$ | 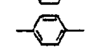 | 3-Cl-5-$CF_3$-pyridin-2-yl | |
| I-117 | $CH_3$ | 2-Cl-6-F-$C_6H_3$ | 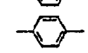 | 5-Cl-6-$CF_3$-pyrimidin-4-yl | |
| I-118 | $CH_3$ | 2-Cl-6-F-$C_6H_3$ | 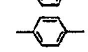 | 4-$CF_3$-pyrimidin-2-yl | |
| I-119 | $CH_3$ | 2-Cl-6-F-$C_6H_3$ | 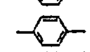 | 5-Cl-4-$CHF_2$-pyrimidin-6-yl | |

Table 32

| No. | $R^1$ | $Ar^1$ | $Ar^2$ | $Ar^3$ | mp. |
|---|---|---|---|---|---|
| I-120 | $C_2H_5$ | 2,6-$Cl_2$-$C_6H_3$ | 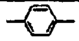 | 2-Cl-4-$CF_3$-$C_6H_3$ | |
| I-121 | $CF_3$ | 2,6-$Cl_2$-$C_6H_3$ | 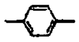 | 3-Cl-5-$CF_3$-pyridin-2-yl | |
| I-122 | $CF_3$ | 2,6-$Cl_2$-$C_6H_3$ | 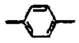 | 3,5-$Cl_2$-pyridin-2-yl | |
| I-123 | Cl | 2,6-$Cl_2$-$C_6H_3$ | 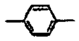 | 3,5-$Cl_2$-pyridin-2-yl | |
| I-124 | Cl | 2,6-$Cl_2$-$C_6H_3$ | 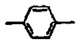 | 2-Cl-4-$CF_3$-$C_6H_3$ | |
| I-125 | CN | 2,6-$Cl_2$-$C_6H_3$ | 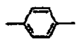 | 3,5-$Cl_2$-pyridin-2-yl | |
| I-126 | CN | 2,6-$Cl_2$-$C_6H_3$ | 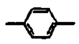 | 2-Cl-4-$CF_3$-$C_6H_3$ | |
| I-127 | $CF_3$ | 2,6-$Cl_2$-$C_6H_3$ | 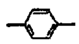 | 2-Cl-4-$CF_3$-$C_6H_3$ | |
| I-128 | $CH_3$ | 3-Cl-pyridin-2-yl | 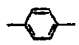 | 3-Cl-5-$CF_3$-pyridin-2-yl | |
| I-129 | $CHF_2$ | 2-Cl-6-F-$C_6H_3$ | 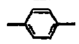 | 3-Cl-5-$CF_3$-pyridin-2-yl | |
| I-130 | H | 2-Cl-6-F-$C_6H_3$ | 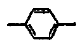 | 3,5-$Cl_2$-pyridin-2-yl | |
| I-131 | Cl | 2-Cl-6-F-$C_6H_3$ | 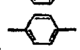 | 3,5-$Cl_2$-pyridin-2-yl | |
| I-132 | CN | 2-Cl-6-F-$C_6H_3$ | 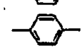 | 3,5-$Cl_2$-pyridin-2-yl | |
| I-133 | $CF_3$ | 2-Cl-6-F-$C_6H_3$ | 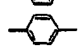 | 3,5-$Cl_2$-pyridin-2-yl | |
| I-134 | H | 2-Cl-6-F-$C_6H_3$ | 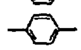 | 2-Cl-4-$CF_3$-$C_6H_3$ | |
| I-135 | Cl | 2-Cl-6-F-$C_6H_3$ | 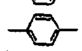 | 2-Cl-4-$CF_3$-$C_6H_3$ | |
| I-136 | CN | 2-Cl-6-F-$C_6H_3$ | 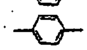 | 2-Cl-4-$CF_3$-$C_6H_3$ | |
| I-137 | $CF_3$ | 2-Cl-6-F-$C_6H_3$ | 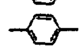 | 2-Cl-4-$CF_3$-$C_6H_3$ | |
| I-138 | $CH_3$ | 2-$CF_3$-$C_6H_4$ | 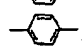 | 3-Cl-5-$CF_3$-pyridin-2-yl | |
| I-139 | $CH_3$ | 2-Cl-pyridin-3-yl | 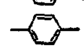 | 3-Cl-5-$CF_3$-pyridin-2-yl | |
| I-140 | $CH_3$ | 2,4,6-$Cl_3$-$C_6H_2$ | 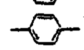 | 3-Cl-5-$CF_3$-pyridin-2-yl | |
| I-141 | HC≡C | 2-Cl-6-F-$C_6H_3$ | 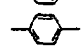 | 3-Cl-5-$CF_3$-pyridin-2-yl | |
| I-142 | $CH_3$ | 2-F-6-$CF_3$-$C_6H_3$ | 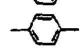 | 3-Cl-5-$CF_3$-pyridin-2-yl | |
| I-143 | $CH_3$ | 2,3,5,6-$F_4$-$C_6H_1$ | 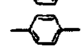 | 3-Cl-5-$CF_3$-pyridin-2-yl | |
| I-144 | $CH_3$ | 2,4,6-$F_3$-$C_6H_2$ | 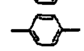 | 3-Cl-5-$CF_3$-pyridin-2-yl | |
| I-145 | $CH_3$ | 2-$OCF_3$-$C_6H_4$ | 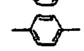 | 3-Cl-5-$CF_3$-pyridin-2-yl | |
| I-146 | $CH_3$ | 3-Cl-2,6-$F_2$-$C_6H_2$ | 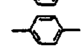 | 3-Cl-5-$CF_3$-pyridin-2-yl | |
| I-147 | $CH_3$ | 2-Cl-3,6-$F_2$-$C_6H_2$ | 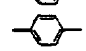 | 3-Cl-5-$CF_3$-pyridin-2-yl | |
| I-148 | $CH_3$ | 2-Cl-6-F-$C_6H_3$ | 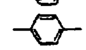 | 5-$CF_3$-pyrimidin-2-yl | |
| I-149 | $CH_3$ | 2,6-$(OCH_3)_2C_6H_3$ | 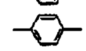 | 3-Cl-5-$CF_3$-pyridin-2-yl | |
| I-150 | $CH_3$ | 2-Cl-6-F-$C_6H_3$ | 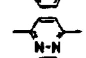 | 3-Cl-5-$CF_3$-pyridin-2-yl | |
| I-151 | $CH_3$ | 2,6-$(CF_3)_2C_6H_3$ | 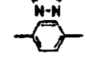 | 3-Cl-5-$CF_3$-pyridin-2-yl | |
| I-152 | $COOCH_3$ | 2-Cl-6-F-$C_6H_3$ | 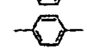 | 3-Cl-5-$CF_3$-pyridin-2-yl | |
| I-153 | $CH_3$ | 3-Cl-pyridin-2-yl | 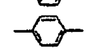 | 3,5-$Cl_2$-pyridin-2-yl | |
| I-154 | H | 3-Cl-pyridin-2-yl | 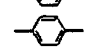 | 3,5-$Cl_2$-pyridin-2-yl | |
| I-155 | Cl | 3-Cl-pyridin-2-yl | 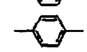 | 3,5-$Cl_2$-pyridin-2-yl | |
| I-156 | CN | 3-Cl-pyridin-2-yl | 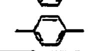 | 3,5-$Cl_2$-pyridin-2-yl | |

Table 33

| No. | $R^1$ | $Ar^1$ | $Ar^2$ | $Ar^3$ | mp. |
|---|---|---|---|---|---|
| I-157 | $CF_3$ | 3-Cl-pyridin-2-yl | 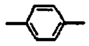 | 3,5-$Cl_2$-pyridin-2-yl | |
| I-158 | H | 3-Cl-pyridin-2-yl | 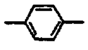 | 3-Cl-5-$CF_3$-pyridin-2-yl | |
| I-159 | Cl | 3-Cl-pyridin-2-yl | 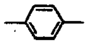 | 3-Cl-5-$CF_3$-pyridin-2-yl | |
| I-160 | CN | 3-Cl-pyridin-2-yl | 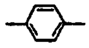 | 3-Cl-5-$CF_3$-pyridin-2-yl | |
| I-161 | $CF_3$ | 3-Cl-pyridin-2-yl | 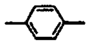 | 3-Cl-5-$CF_3$-pyridin-2-yl | |

Table 34

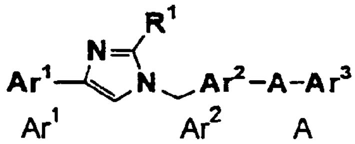

| No. | $R^1$ | $Ar^1$ | $Ar^2$ | A | $Ar^3$ | mp. |
|---|---|---|---|---|---|---|
| I-162 | $CH_3$ | 2-Cl-6-F-$C_6H_3$ | 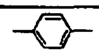 | — | 3-Cl-5-$CF_3$-pyridin-2-yl | |
| I-163 | $CH_3$ | 2-Cl-6-F-$C_6H_3$ | 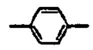 | O | 3-Cl-5-$CF_3$-pyridin-2-yl | |
| I-164 | $CH_3$ | 2-Cl-6-F-$C_6H_3$ | 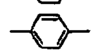 | $CH_2O$ | 3-Cl-5-$CF_3$-pyridin-2-yl | |
| I-165 | H | 2-Cl-6-F-$C_6H_3$ | 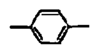 | — | 3-Cl-5-$CF_3$-pyridin-2-yl | |
| I-166 | H | 2-Cl-6-F-$C_6H_3$ | 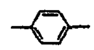 | O | 3-Cl-5-$CF_3$-pyridin-2-yl | |
| I-167 | H | 2-Cl-6-F-$C_6H_3$ | 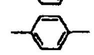 | $CH_2O$ | 3-Cl-5-$CF_3$-pyridin-2-yl | |
| I-168 | Cl | 2-Cl-6-F-$C_6H_3$ | 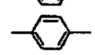 | — | 3-Cl-5-$CF_3$-pyridin-2-yl | |
| I-169 | Cl | 2-Cl-6-F-$C_6H_3$ | 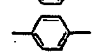 | O | 3-Cl-5-$CF_3$-pyridin-2-yl | |
| I-170 | Cl | 2-Cl-6-F-$C_6H_3$ | 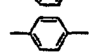 | $CH_2O$ | 3-Cl-5-$CF_3$-pyridin-2-yl | |
| I-171 | CN | 2-Cl-6-F-$C_6H_3$ | 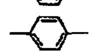 | — | 3-Cl-5-$CF_3$-pyridin-2-yl | |
| I-172 | CN | 2-Cl-6-F-$C_6H_3$ | 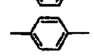 | O | 3-Cl-5-$CF_3$-pyridin-2-yl | |
| I-173 | CN | 2-Cl-6-F-$C_6H_3$ | 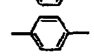 | $CH_2O$ | 3-Cl-5-$CF_3$-pyridin-2-yl | |
| I-174 | $CF_3$ | 2-Cl-6-F-$C_6H_3$ | 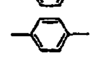 | — | 3-Cl-5-$CF_3$-pyridin-2-yl | |
| I-175 | $CF_3$ | 2-Cl-6-F-$C_6H_3$ | 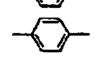 | O | 3-Cl-5-$CF_3$-pyridin-2-yl | |
| I-176 | $CF_3$ | 2-Cl-6-F-$C_6H_3$ | 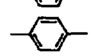 | $CH_2O$ | 3-Cl-5-$CF_3$-pyridin-2-yl | |
| I-177 | $CH_3$ | 2-Cl-6-F-$C_6H_3$ | 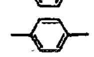 | — | 3,5-$Cl_2$-pyridin-2-yl | |
| I-178 | $CH_3$ | 2-Cl-6-F-$C_6H_3$ | 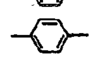 | O | 3,5-$Cl_2$-pyridin-2-yl | |
| I-179 | $CH_3$ | 2-Cl-6-F-$C_6H_3$ | 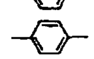 | $CH_2O$ | 3,5-$Cl_2$-pyridin-2-yl | |
| I-180 | H | 2-Cl-6-F-$C_6H_3$ | 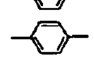 | — | 3,5-$Cl_2$-pyridin-2-yl | |
| I-181 | H | 2-Cl-6-F-$C_6H_3$ | 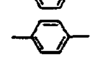 | O | 3,5-$Cl_2$-pyridin-2-yl | |
| I-182 | H | 2-Cl-6-F-$C_6H_3$ | 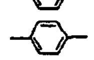 | $CH_2O$ | 3,5-$Cl_2$-pyridin-2-yl | |
| I-183 | Cl | 2-Cl-6-F-$C_6H_3$ | 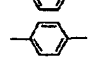 | — | 3,5-$Cl_2$-pyridin-2-yl | |
| I-184 | Cl | 2-Cl-6-F-$C_6H_3$ | 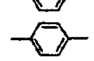 | O | 3,5-$Cl_2$-pyridin-2-yl | |
| I-185 | Cl | 2-Cl-6-F-$C_6H_3$ | 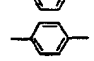 | $CH_2O$ | 3,5-$Cl_2$-pyridin-2-yl | |
| I-186 | CN | 2-Cl-6-F-$C_6H_3$ | 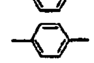 | — | 3,5-$Cl_2$-pyridin-2-yl | |
| I-187 | CN | 2-Cl-6-F-$C_6H_3$ | 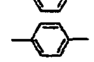 | O | 3,5-$Cl_2$-pyridin-2-yl | |
| I-188 | CN | 2-Cl-6-F-$C_6H_3$ | 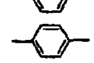 | $CH_2O$ | 3,5-$Cl_2$-pyridin-2-yl | |
| I-189 | $CF_3$ | 2-Cl-6-F-$C_6H_3$ | 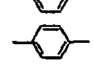 | — | 3,5-$Cl_2$-pyridin-2-yl | |
| I-190 | $CF_3$ | 2-Cl-6-F-$C_6H_3$ | 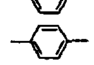 | O | 3,5-$Cl_2$-pyridin-2-yl | |
| I-191 | $CF_3$ | 2-Cl-6-F-$C_6H_3$ | 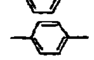 | $CH_2O$ | 3,5-$Cl_2$-pyridin-2-yl | |

Table 35

| No. | $R^1$ | $Ar^1$ | $Ar^2$ | A | $Ar^3$ | mp. |
|---|---|---|---|---|---|---|
| I-192 | $CH_3$ | 2,6-$Cl_2$-$C_6H_3$ | 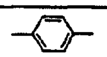 | — | 3,5-$Cl_2$-pyridin-2-yl | |
| I-193 | $CH_3$ | 2,6-$Cl_2$-$C_6H_3$ | 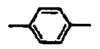 | O | 3,5-$Cl_2$-pyridin-2-yl | |
| I-194 | $CH_3$ | 2,6-$Cl_2$-$C_6H_3$ | 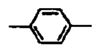 | $CH_2O$ | 3,5-$Cl_2$-pyridin-2-yl | |
| I-195 | H | 2,6-$Cl_2$-$C_6H_3$ | 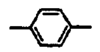 | — | 3,5-$Cl_2$-pyridin-2-yl | |
| I-196 | H | 2,6-$Cl_2$-$C_6H_3$ | 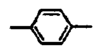 | O | 3,5-$Cl_2$-pyridin-2-yl | |
| I-197 | H | 2,6-$Cl_2$-$C_6H_3$ | 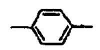 | $CH_2O$ | 3,5-$Cl_2$-pyridin-2-yl | |
| I-198 | Cl | 2,6-$Cl_2$-$C_6H_3$ | 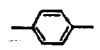 | — | 3,5-$Cl_2$-pyridin-2-yl | |
| I-199 | Cl | 2,6-$Cl_2$-$C_6H_3$ | 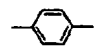 | O | 3,5-$Cl_2$-pyridin-2-yl | |
| I-200 | Cl | 2,6-$Cl_2$-$C_6H_3$ | 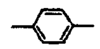 | $CH_2O$ | 3,5-$Cl_2$-pyridin-2-yl | |
| I-201 | CN | 2,6-$Cl_2$-$C_6H_3$ | 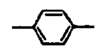 | — | 3,5-$Cl_2$-pyridin-2-yl | |
| I-202 | CN | 2,6-$Cl_2$-$C_6H_3$ | 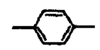 | O | 3,5-$Cl_2$-pyridin-2-yl | |
| I-203 | CN | 2,6-$Cl_2$-$C_6H_3$ | 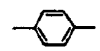 | $CH_2O$ | 3,5-$Cl_2$-pyridin-2-yl | |
| I-204 | $CF_3$ | 2,6-$Cl_2$-$C_6H_3$ | 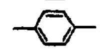 | — | 3,5-$Cl_2$-pyridin-2-yl | |
| I-205 | $CF_3$ | 2,6-$Cl_2$-$C_6H_3$ | 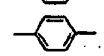 | O | 3,5-$Cl_2$-pyridin-2-yl | |
| I-206 | $CF_3$ | 2,6-$Cl_2$-$C_6H_3$ | 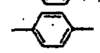 | $CH_2O$ | 3,5-$Cl_2$-pyridin-2-yl | |
| I-207 | $CH_3$ | 2,6-$Cl_2$-$C_6H_3$ | 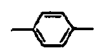 | — | 3-Cl-5-$CF_3$-pyridin-2-yl | |
| I-208 | $CH_3$ | 2,6-$Cl_2$-$C_6H_3$ | 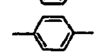 | O | 3-Cl-5-$CF_3$-pyridin-2-yl | |
| I-209 | $CH_3$ | 2,6-$Cl_2$-$C_6H_3$ | 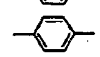 | $CH_2O$ | 3-Cl-5-$CF_3$-pyridin-2-yl | |
| I-210 | H | 2,6-$Cl_2$-$C_6H_3$ | 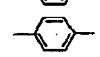 | — | 3-Cl-5-$CF_3$-pyridin-2-yl | |
| I-211 | H | 2,6-$Cl_2$-$C_6H_3$ | 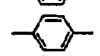 | O | 3-Cl-5-$CF_3$-pyridin-2-yl | |
| I-212 | H | 2,6-$Cl_2$-$C_6H_3$ | 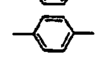 | $CH_2O$ | 3-Cl-5-$CF_3$-pyridin-2-yl | |
| I-213 | Cl | 2,6-$Cl_2$-$C_6H_3$ | 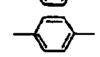 | — | 3-Cl-5-$CF_3$-pyridin-2-yl | |
| I-214 | Cl | 2,6-$Cl_2$-$C_6H_3$ | 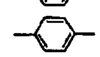 | O | 3-Cl-5-$CF_3$-pyridin-2-yl | |
| I-215 | Cl | 2,6-$Cl_2$-$C_6H_3$ | 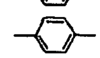 | $CH_2O$ | 3-Cl-5-$CF_3$-pyridin-2-yl | |
| I-216 | CN | 2,6-$Cl_2$-$C_6H_3$ | 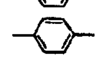 | — | 3-Cl-5-$CF_3$-pyridin-2-yl | |
| I-217 | CN | 2,6-$Cl_2$-$C_6H_3$ | 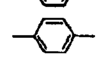 | O | 3-Cl-5-$CF_3$-pyridin-2-yl | |
| I-218 | CN | 2,6-$Cl_2$-$C_6H_3$ | 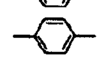 | $CH_2O$ | 3-Cl-5-$CF_3$-pyridin-2-yl | |
| I-219 | $CF_3$ | 2,6-$Cl_2$-$C_6H_3$ | 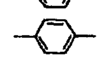 | — | 3-Cl-5-$CF_3$-pyridin-2-yl | |
| I-220 | $CF_3$ | 2,6-$Cl_2$-$C_6H_3$ | 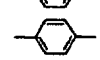 | O | 3-Cl-5-$CF_3$-pyridin-2-yl | |
| I-221 | $CF_3$ | 2,6-$Cl_2$-$C_6H_3$ | 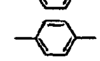 | $CH_2O$ | 3-Cl-5-$CF_3$-pyridin-2-yl | |
| I-222 | $CH_3$ | 2,6-$F_2$-$C_6H_3$ | 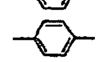 | — | 3-Cl-5-$CF_3$-pyridin-2-yl | |
| I-223 | $CH_3$ | 2,6-$F_2$-$C_6H_3$ | 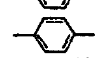 | O | 3-Cl-5-$CF_3$-pyridin-2-yl | |

Table 36

| No. | $R^1$ | $Ar^1$ | $Ar^2$ | A | $Ar^3$ | mp. |
|---|---|---|---|---|---|---|
| I-224 | $CH_3$ | 2,6-$F_2$-$C_6H_3$ | 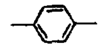 | $CH_2O$ | 3-Cl-5-$CF_3$-pyridin-2-yl | |
| I-225 | H | 2,6-$F_2$-$C_6H_3$ | 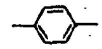 | — | 3-Cl-5-$CF_3$-pyridin-2-yl | |
| I-226 | H | 2,6-$F_2$-$C_6H_3$ | 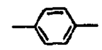 | O | 3-Cl-5-$CF_3$-pyridin-2-yl | |
| I-227 | H | 2,6-$F_2$-$C_6H_3$ | 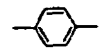 | $CH_2O$ | 3-Cl-5-$CF_3$-pyridin-2-yl | |
| I-228 | Cl | 2,6-$F_2$-$C_6H_3$ | 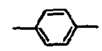 | — | 3-Cl-5-$CF_3$-pyridin-2-yl | |
| I-229 | Cl | 2,6-$F_2$-$C_6H_3$ | 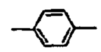 | O | 3-Cl-5-$CF_3$-pyridin-2-yl | |
| I-230 | Cl | 2,6-$F_2$-$C_6H_3$ | 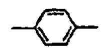 | $CH_2O$ | 3-Cl-5-$CF_3$-pyridin-2-yl | |
| I-231 | CN | 2,6-$F_2$-$C_6H_3$ | 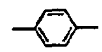 | — | 3-Cl-5-$CF_3$-pyridin-2-yl | |
| I-232 | CN | 2,6-$F_2$-$C_6H_3$ | 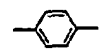 | O | 3-Cl-5-$CF_3$-pyridin-2-yl | |
| I-233 | CN | 2,6-$F_2$-$C_6H_3$ | 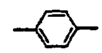 | $CH_2O$ | 3-Cl-5-$CF_3$-pyridin-2-yl | |
| I-234 | $CF_3$ | 2,6-$F_2$-$C_6H_3$ | 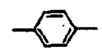 | — | 3-Cl-5-$CF_3$-pyridin-2-yl | |
| I-235 | $CF_3$ | 2,6-$F_2$-$C_6H_3$ | 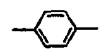 | O | 3-Cl-5-$CF_3$-pyridin-2-yl | |
| I-236 | $CF_3$ | 2,6-$F_2$-$C_6H_3$ | 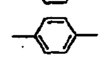 | $CH_2O$ | 3-Cl-5-$CF_3$-pyridin-2-yl | |
| I-237 | $CH_3$ | 2,6-$F_2$-$C_6H_3$ | 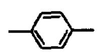 | | 3,5-$Cl_2$-pyridin-2-yl | |
| I-238 | $CH_3$ | 2,6-$F_2$-$C_6H_3$ | 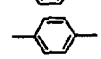 | O | 3,5-$Cl_2$-pyridin-2-yl | |
| I-239 | $CH_3$ | 2,6-$F_2$-$C_6H_3$ | 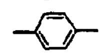 | $CH_2O$ | 3,5-$Cl_2$-pyridin-2-yl | |
| I-240 | H | 2,6-$F_2$-$C_6H_3$ | 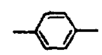 | — | 3,5-$Cl_2$-pyridin-2-yl | |
| I-241 | H | 2,6-$F_2$-$C_6H_3$ | 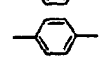 | O | 3,5-$Cl_2$-pyridin-2-yl | |
| I-242 | H | 2,6-$F_2$-$C_6H_3$ | 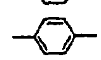 | $CH_2O$ | 3,5-$Cl_2$-pyridin-2-yl | |
| I-243 | Cl | 2,6-$F_2$-$C_6H_3$ | 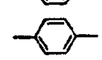 | — | 3,5-$Cl_2$-pyridin-2-yl | |
| I-244 | Cl | 2,6-$F_2$-$C_6H_3$ | 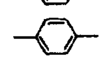 | O | 3,5-$Cl_2$-pyridin-2-yl | |
| I-245 | Cl | 2,6-$F_2$-$C_6H_3$ | 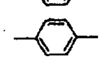 | $CH_2O$ | 3,5-$Cl_2$-pyridin-2-yl | |
| I-246 | CN | 2,6-$F_2$-$C_6H_3$ | 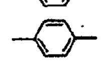 | — | 3,5-$Cl_2$-pyridin-2-yl | |
| I-247 | CN | 2,6-$F_2$-$C_6H_3$ | 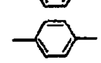 | O | 3,5-$Cl_2$-pyridin-2-yl | |
| I-248 | CN | 2,6-$F_2$-$C_6H_3$ | 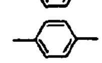 | $CH_2O$ | 3,5-$Cl_2$-pyridin-2-yl | |
| I-249 | $CF_3$ | 2,6-$F_2$-$C_6H_3$ | 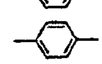 | — | 3,5-$Cl_2$-pyridin-2-yl | |
| I-250 | $CF_3$ | 2,6-$F_2$-$C_6H_3$ | 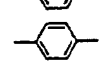 | O | 3,5-$Cl_2$-pyridin-2-yl | |
| I-251 | $CF_3$ | 2,6-$F_2$-$C_6H_3$ | 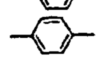 | $CH_2O$ | 3,5-$Cl_2$-pyridin-2-yl | |

Table 37

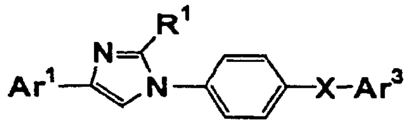

| No. | $R^1$ | $Ar^1$ | X | $Ar^3$ | mp |
|---|---|---|---|---|---|
| I-252 | $CH_3$ | 2-Cl-6-F-$C_6H_3$ | $CH_2S$ | 4-Cl-$C_6H_4$ | |
| I-253 | $CH_3$ | 2-Cl-6-F-$C_6H_3$ | $CH_2NH$ | 4-Cl-$C_6H_4$ | |
| I-254 | $CH_3$ | 2-Cl-6-F-$C_6H_3$ | $OCH_2$ | 4-Cl-$C_6H_4$ | |
| I-255 | $CH_3$ | 2-Cl-6-F-$C_6H_3$ | $OCH_2$ | 2,4,6-$Cl_3$-$C_6H_2$ | |
| I-256 | $CH_3$ | 2-Cl-6-F-$C_6H_3$ | O | 3-Cl-5-$CF_3$-pyridin-2-yl | |
| I-257 | $CH_3$ | 2-Cl-6-F-$C_6H_3$ | NH | 5-$CF_3$-pyridin-2-yl | |
| I-258 | $CH_3$ | 2-Cl-6-F-$C_6H_3$ | — | 4-$CF_3O$-$C_6H_4$ | |
| I-259 | $CH_3$ | 2-Cl-6-F-$C_6H_3$ | $CH(CH_3)O$ | 3-Cl-5-$CF_3$-pyridin-2-yl | |
| I-260 | $CH_3$ | 2-Cl-6-F-$C_6H_3$ | $CH(CH_3)O$ | 3,5-$Cl_2$-pyridin-2-yl | |
| I-261 | $CH_3$ | 2-Cl-6-F-$C_6H_3$ | $OCH_2$ | 3-Cl-5-$CF_3$-pyridin-2-yl | |
| I-262 | $CH_3$ | 2-Cl-6-F-$C_6H_3$ | $OCH(CH_3)$ | 3-Cl-5-$CF_3$-pyridin-2-yl | |
| I-263 | $CH_3$ | 2-Cl-6-F-$C_6H_3$ | CH(CN)O | 3-Cl-5-$CF_3$-pyridin-2-yl | |
| I-264 | $CH_3$ | 2-Cl-6-F-$C_6H_3$ | — | 3-Cl-5-$CF_3$-pyridin-2-yl | |
| I-265 | Cl | 2-Cl-6-F-$C_6H_3$ | $CH_2S$ | 4-Cl-$C_6H_4$ | |
| I-266 | Cl | 2-Cl-6-F-$C_6H_3$ | $CH_2NH$ | 4-Cl-$C_6H_4$ | |
| I-267 | Cl | 2-Cl-6-F-$C_6H_3$ | $OCH_2$ | 4-Cl-$C_6H_4$ | |
| I-268 | Cl | 2-Cl-6-F-$C_6H_3$ | $OCH_2$ | 2,4,6-$Cl_3$-$C_6H_2$ | |
| I-269 | Cl | 2-Cl-6-F-$C_6H_3$ | O | 3-Cl-5-$CF_3$-pyridin-2-yl | |
| I-270 | Cl | 2-Cl-6-F-$C_6H_3$ | NH | 5-$CF_3$-pyridin-2-yl | |
| I-271 | Cl | 2-Cl-6-F-$C_6H_3$ | — | 4-$CF_3O$-$C_6H_4$ | |
| I-272 | Cl | 2-Cl-6-F-$C_6H_3$ | $CH(CH_3)O$ | 3-Cl-5-$CF_3$-pyridin-2-yl | |
| I-273 | Cl | 2-Cl-6-F-$C_6H_3$ | $CH(CH_3)O$ | 3,5-$Cl_2$-pyridin-2-yl | |
| I-274 | Cl | 2-Cl-6-F-$C_6H_3$ | $OCH_2$ | 3-Cl-5-$CF_3$-pyridin-2-yl | |
| I-275 | Cl | 2-Cl-6-F-$C_6H_3$ | $OCH(CH_3)$ | 3-Cl-5-$CF_3$-pyridin-2-yl | |
| I-276 | Cl | 2-Cl-6-F-$C_6H_3$ | CH(CN)O | 3-Cl-5-$CF_3$-pyridin-2-yl | |
| I-277 | Cl | 2-Cl-6-F-$C_6H_3$ | — | 3-Cl-5-$CF_3$-pyridin-2-yl | |

Table 38

| No. | $R^1$ | $Ar^1$ | X | $Ar^3$ | mp |
|---|---|---|---|---|---|
| I-278 | H | 2-Cl-6-F-$C_6H_3$ | $CH_2S$ | 4-Cl-$C_6H_4$ | |
| I-279 | H | 2-Cl-6-F-$C_6H_3$ | $CH_2NH$ | 4-Cl-$C_6H_4$ | |
| I-280 | H | 2-Cl-6-F-$C_6H_3$ | $OCH_2$ | 4-Cl-$C_6H_4$ | |
| I-281 | H | 2-Cl-6-F-$C_6H_3$ | $OCH_2$ | 2,4,6-$Cl_3$-$C_6H_2$ | |
| I-282 | H | 2-Cl-6-F-$C_6H_3$ | O | 3-Cl-5-$CF_3$-pyridin-2-yl | |
| I-283 | H | 2-Cl-6-F-$C_6H_3$ | NH | 5-$CF_3$-pyridin-2-yl | |
| I-284 | H | 2-Cl-6-F-$C_6H_3$ | — | 4-$CF_3O$-$C_6H_4$ | |
| I-285 | H | 2-Cl-6-F-$C_6H_3$ | $CH(CH_3)O$ | 3-Cl-5-$CF_3$-pyridin-2-yl | |
| I-286 | H | 2-Cl-6-F-$C_6H_3$ | $CH(CH_3)O$ | 3,5-$Cl_2$-pyridin-2-yl | |
| I-287 | H | 2-Cl-6-F-$C_6H_3$ | $OCH_2$ | 3-Cl-5-$CF_3$-pyridin-2-yl | |
| I-288 | H | 2-Cl-6-F-$C_6H_3$ | $OCH(CH_3)$ | 3-Cl-5-$CF_3$-pyridin-2-yl | |
| I-289 | H | 2-Cl-6-F-$C_6H_3$ | CH(CN)O | 3-Cl-5-$CF_3$-pyridin-2-yl | |
| I-290 | H | 2-Cl-6-F-$C_6H_3$ | — | 3-Cl-5-$CF_3$-pyridin-2-yl | |
| I-291 | CN | 2-Cl-6-F-$C_6H_3$ | $CH_2S$ | 4-Cl-$C_6H_4$ | |
| I-292 | CN | 2-Cl-6-F-$C_6H_3$ | $CH_2NH$ | 4-Cl-$C_6H_4$ | |
| I-293 | CN | 2-Cl-6-F-$C_6H_3$ | $OCH_2$ | 4-Cl-$C_6H_4$ | |
| I-294 | CN | 2-Cl-6-F-$C_6H_3$ | $OCH_2$ | 2,4,6-$Cl_3$-$C_6H_2$ | |
| I-295 | CN | 2-Cl-6-F-$C_6H_3$ | O | 3-Cl-5-$CF_3$-pyridin-2-yl | |
| I-296 | CN | 2-Cl-6-F-$C_6H_3$ | NH | 5-$CF_3$-pyridin-2-yl | |
| I-297 | CN | 2-Cl-6-F-$C_6H_3$ | — | 4-$CF_3O$-$C_6H_4$ | |
| I-298 | CN | 2-Cl-6-F-$C_6H_3$ | $CH(CH_3)O$ | 3-Cl-5-$CF_3$-pyridin-2-yl | |
| I-299 | CN | 2-Cl-6-F-$C_6H_3$ | $CH(CH_3)O$ | 3,5-$Cl_2$-pyridin-2-yl | |
| I-300 | CN | 2-Cl-6-F-$C_6H_3$ | $OCH_2$ | 3-Cl-5-$CF_3$-pyridin-2-yl | |
| I-301 | CN | 2-Cl-6-F-$C_6H_3$ | $OCH(CH_3)$ | 3-Cl-5-$CF_3$-pyridin-2-yl | |
| I-302 | CN | 2-Cl-6-F-$C_6H_3$ | CH(CN)O | 3-Cl-5-$CF_3$-pyridin-2-yl | |
| I-303 | CN | 2-Cl-6-F-$C_6H_3$ | — | 3-Cl-5-$CF_3$-pyridin-2-yl | |
| I-304 | $CF_3$ | 2-Cl-6-F-$C_6H_3$ | $CH_2S$ | 4-Cl-$C_6H_4$ | |
| I-305 | $CF_3$ | 2-Cl-6-F-$C_6H_3$ | $CH_2NH$ | 4-Cl-$C_6H_4$ | |
| I-306 | $CF_3$ | 2-Cl-6-F-$C_6H_3$ | $OCH_2$ | 4-Cl-$C_6H_4$ | |
| I-307 | $CF_3$ | 2-Cl-6-F-$C_6H_3$ | $OCH_2$ | 2,4,6-$Cl_3$-$C_6H_2$ | |
| I-308 | $CF_3$ | 2-Cl-6-F-$C_6H_3$ | O | 3-Cl-5-$CF_3$-pyridin-2-yl | |
| I-309 | $CF_3$ | 2-Cl-6-F-$C_6H_3$ | NH | 5-$CF_3$-pyridin-2-yl | |
| I-310 | $CF_3$ | 2-Cl-6-F-$C_6H_3$ | — | 4-$CF_3O$-$C_6H_4$ | |
| I-311 | $CF_3$ | 2-Cl-6-F-$C_6H_3$ | $CH(CH_3)O$ | 3-Cl-5-$CF_3$-pyridin-2-yl | |
| I-312 | $CF_3$ | 2-Cl-6-F-$C_6H_3$ | $CH(CH_3)O$ | 3,5-$Cl_2$-pyridin-2-yl | |
| I-313 | $CF_3$ | 2-Cl-6-F-$C_6H_3$ | $OCH_2$ | 3-Cl-5-$CF_3$-pyridin-2-yl | |
| I-314 | $CF_3$ | 2-Cl-6-F-$C_6H_3$ | $OCH(CH_3)$ | 3-Cl-5-$CF_3$-pyridin-2-yl | |
| I-315 | $CF_3$ | 2-Cl-6-F-$C_6H_3$ | CH(CN)O | 3-Cl-5-$CF_3$-pyridin-2-yl | |
| I-316 | $CF_3$ | 2-Cl-6-F-$C_6H_3$ | — | 3-Cl-5-$CF_3$-pyridin-2-yl | |

Formulation Example 1

Compound No. T-21 (20% by weight), xylene (75% by weight) and polyoxyethylene alkyl ether (Neugen ET-135 (trade name), 5% by weight) are mixed well to prepare an emulsion.

Formulation Example 2

Compound No. T-21 (30% by weight), sodium lignin sulfonate (5% by weight), polyoxyethylene glycol ether (Neugen ET-135 (trade name), 5% by weight), white carbon (30% by weight) and clay (30% by weight) are mixed well to prepare a wettable powder.

Formulation Example 3

Compound No. T-21 (3% by weight), white carbon (3% by weight) and clay (94% by weight) are mixed well to prepare a powder.

Formulation Example 4

Compound No. T-21 (10% by weight), polyoxyethylene polyoxypropylene block copolymer (Newpol PE-64 (trade name), 1% by weight), sodium lignin sulfonate (5% by weight) and clay (84% by weight) are pulverized and mixed well. To the resulting mixture is added water, followed by well kneading, granulating and drying, to prepare a granule.

Formulation Example 5

Compound No. T-21 (3% by weight), white carbon (3% by weight), liquid paraffin (Doriresu C (trade name), 1% by weight), Hartol fatty acid (0.5% by weight) and clay (92.5% by weight) are mixed well to prepare a powder DL.

Formulation Example 6

Compound No. T-21 (10% by weight), polyoxyalkylene allyl phenyl ether sulfate (New Cargen FS-7 (trade name), 3% by weight), ethylene glycol (8% by weight), colloidal hydrated aluminum silicate (Kunipia F (trade name), 1% by weight), silicone emulsion (Antifoam E-20 (trade name), 0.2% by weight), n-butyl p-hydroxybenzoate (0.1% by weight) and water (77.7% by weight) are mixed and milled in a wet system to prepare a flowable formulation.

Test Example

1) insecticidal effect for *Plutella xylostella*

One 7 to 8-leave stage foliage leaf of cabbage was cut off and dipped in a pesticidal solution for a few seconds, which was prepared by dissolving 5 mg of test compound (represented by the compound Nos. in the above Examples) in 0.5 ml of acetone containing Tween 20 (trade name) and then diluting it to the prescribed concentration (100 ppm) with Dain water diluted 5000-fold. After drying the pesticidal solution, the leaf was put into an ice cream cup (180 ml), and ten second-instar larvae of *Plutella xylostella* were set free. The cup was left in a temperature-controlled breeding room (24°C). On the fifth day after, the number of surviving *Plutella xylostella* was examined. The rate of dead pests was calculated by the following formula, and the result was shown in table 39.

$$\text{rate of dead pests (\%)} = (\text{number of dead pests/ number of test pests}) \times 100$$

Table 39

| Compound No. | Rate of dead pests (%) |
|---|---|
| T-3 | 100 |
| T-5 | 95 |
| T-9 | 100 |
| T-12 | 100 |
| T-17 | 100 |
| T-18 | 100 |
| T-20 | 100 |
| T-21 | 100 |
| T-28 | 100 |
| T-30 | 100 |
| T-32 | 100 |

Table 39 (continued)

| Compound No. | Rate of dead pests (%) |
|---|---|
| T-40 | 95 |
| T-43 | 100 |
| T-46 | 100 |
| T-47 | 100 |
| T-48 | 100 |
| T-52 | 100 |
| T-56 | 100 |
| T-66 | 95 |
| T-74 | 100 |
| T-78 | 100 |
| T-88 | 95 |
| T-106 | 100 |
| T-107 | 100 |
| T-120 | 100 |
| T-133 | 95 |
| T-267 | 100 |
| P-21 | 100 |
| P-43 | 100 |
| P-46 | 100 |

2) acaricidal effect for *Tetranychus urticae*

Ten female imagoes *Tetranychus urticae* were set free on a string bean wherein first leaves had just developed. On the following day, the pesticidal solution, which was prepared by dissolving 5 mg of test compound (represented by the compound Nos. in the above Examples) in 0.5 ml of acetone containing Tween 20 (trade name) and then diluting it to the prescribed concentration (500 ppm) with Dain water diluted 5000-fold, was sprayed with spray gun in the amounts wherein the pesticidal solution dripped. The string bean was left in a temperature-controlled breeding room (25°C). On the second and seventh day after treatment, the number of surviving *Tetranychus urticae* was examined. The rate of decrease was calculated by the following formula, and the result was shown in table 40.

$$\text{rate of decrease (\%)} = (1-(S_2/(5.59\times(10+(10+S_1)/2+S_1)))) \times 100$$

$S_1$; the number of surviving imagoes on the second day after
$S_2$; the number of surviving imagoes on the seventh day after

Table 40

| Compound No. | Rate of decrease(%) | Compound No. | Rate of decrease(%) |
|---|---|---|---|
| T-3 | 98 | T-106 | 97 |
| T-5 | 96 | T-107 | 100 |
| T-9 | 99 | T-120 | 99 |
| T-12 | 98 | T-130 | 98 |
| T-18 | 97 | T-131 | 100 |
| T-20 | 98 | T-132 | 100 |
| T-21 | 100 | T-133 | 99 |
| T-24 | 97 | T-134 | 100 |
| T-25 | 99 | T-135 | 96 |
| T-28 | 98 | T-136 | 99 |
| T-30 | 99 | T-137 | 99 |

Table 40 (continued)

| Compound No. | Rate of decrease(%) | Compound No. | Rate of decrease(%) |
|---|---|---|---|
| T-32 | 99 | T-138 | 99 |
| T-33 | 97 | T-139 | 97 |
| T-40 | 97 | T-140 | 99 |
| T-43 | 100 | T-141 | 96 |
| T-46 | 99 | T-143 | 98 |
| T-47 | 100 | T-144 | 98 |
| T-48 | 99 | T-171 | 99 |
| T-52 | 97 | T-172 | 98 |
| T-53 | 99 | T-264 | 98 |
| T-54 | 98 | T-267 | 99 |
| T-56 | 98 | P-21 | 99 |
| T-74 | 97 | P-38 | 98 |
| T-75 | 99 | P-43 | 99 |
| T-76 | 96 | P-46 | 98 |
| T-78 | 98 | P-52 | 100 |
| T-88 | 98 | P-77 | 97 |
| T-92 | 98 | I-77 | 95 |
| T-93 | 97 | I-82 | 99 |

Industrial Applicability

As described above, according to the present application, novel azole compounds useful as a pest controller are provided, and also a novel pest controller containing the azole compound as an active ingredient is provided.

**Claims**

**1.** A compound represented by the formula:

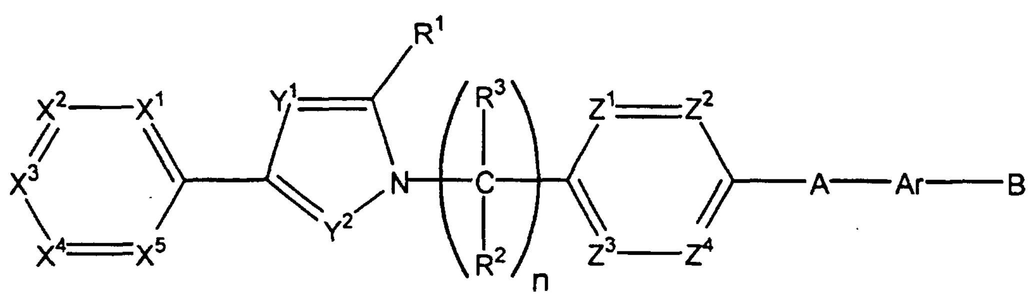

(I)

wherein $X^1$ represents N or C-$R^a$, $X^2$ represents N or C-$R^b$, $X^3$ represents N or C-$R^c$, $X^4$ represents N or C-$R^d$, $X^5$ represents N or C-$R^e$;

$Y^1$ and $Y^2$ are the same or different, and represent N or CH, respectively, provided that they are not CH at the same time;

$Z^1$ represents N or C-$R^f$, $Z^2$ represents N or C-$R^g$, $Z^3$ represents N or C-$R^h$, $Z^4$ represents N or C-$R^i$;

A represents a single bond, O, $CR^jR^kO$, $OCR^jR^k$, $SO_m$, $CR^jR^kSO_m$, $SO_mCR^jR^k$, $NR^l$, $CR^jR^kNR^l$ or $NR^lCR^jR^k$;

Ar represents an aromatic residue optionally having a substituent;

B represents a halogen, an optionally branched alkyl, a cycloalkyl, a haloalkyl, an alkenyl, a haloalkenyl, an alkoxy, a haloalkoxy, an alkyl$SO_m$, a haloalkyl$SO_m$, an amino optionally substituted with 1 or 2 substituents, an alkyl$SO_m$alkyl, a cyano, a nitro or an optionally substituted phenyl;

$R^1$ represents H, a halogen, an optionally substituted and optionally branched alkyl, an optionally substituted

cycloalkyl, an optionally substituted alkenyl, an optionally substituted alkynyl, an optionally substituted alkoxy, an optionally substituted alkyl$SO_m$, an amino optionally substituted with 1 or 2 substituents, a cyano, a nitro, a formyl, a hydroxy, an optionally substituted acyl or an optionally substituted alkoxycarbonyl;
$R^2$ and $R^3$ are the same or different, and represent H or an alkyl, respectively, or may form a ring together with the carbon atom which they bind to;
$R^a$ and $R^e$ are the same or different, and represent H, a halogen, an optionally substituted and optionally branched alkyl, an optionally substituted cycloalkyl, an optionally substituted alkoxy, an optionally substituted alkyl$SO_m$ or an amino optionally substituted with 1 or 2 substituents, respectively, provided that they are not H at the same time;
$R^b$ and $R^d$ are the same or different, and represent H, a halogen, an optionally substituted and optionally branched alkyl, an optionally substituted cycloalkyl, an optionally substituted alkenyl, an optionally substituted alkynyl, an optionally substituted alkoxy, an optionally substituted alkyl$SO_m$, an amino optionally substituted with 1 or 2 substituents, an optionally substituted acyl, an optionally substituted alkoxycarbonyl, a cyano, a nitro, a formyl or a hydroxy, respectively;
$R^c$ represents H, a halogen, an optionally substituted and optionally branched alkyl, an optionally substituted cycloalkyl, an optionally substituted alkenyl, an optionally substituted alkynyl, an optionally substituted alkoxy, an optionally substituted alkyl$SO_m$, an amino optionally substituted with 1 or 2 substituents, an optionally substituted acyl, an optionally substituted alkoxycarbonyl, a nitro, a formyl or a hydroxy;
$R^f$ and $R^h$ are the same or different, and represent H, a halogen, an optionally substituted and optionally branched alkyl, an optionally substituted cycloalkyl, an optionally substituted alkenyl, an optionally substituted alkynyl, an optionally substituted acyl, an optionally substituted alkoxycarbonyl, a cyano, a nitro, a formyl or a hydroxyl, respectively;
$R^g$ and $R^i$ are the same or different, and represent H, a halogen, an optionally substituted and optionally branched alkyl, an optionally substituted cycloalkyl, an optionally substituted alkenyl, an optionally substituted alkynyl, an optionally substituted alkoxy, an optionally substituted alkyl$SO_m$, an amino optionally substituted with 1 or 2 substituents, an optionally substituted acyl, an optionally substituted alkoxycarbonyl, a cyano, a nitro, a formyl or a hydroxyl, respectively;
$R^j$ and $R^k$ are the same or different, and represent H, a cyano or an alkyl, respectively, or may form a ring together with the carbon atom which they bind to;
$R^l$ represents H or an alkyl;
m represents 0, 1 or 2; n represents 0 or 1;
provided that when $Y^1$ and $Y^2$ are N at the same time, and n is 0, and one of $X^1$ to $X^5$ is N, and $R^1$ is a hydrogen, a lower alkyl, a lower alkenyl, a lower alkynyl, a haloalkyl, an alkoxyalkyl or an optionally substituted phenyl group, then Ar represents an aromatic heterocyclic residue optionally having a substituent,
when $Y^1$ and $Y^2$ are N at the same time, and n is 0, and $X^1$ to $X^5$ are not N, and A is O, S or $NR^l$, then $R^1$ represents a group other than halogen,
when $Y^1$ and $Y^2$ are N at the same time, and n is 0, and $X^1$ to $X^5$ are not N, and A is $OCR^jR^k$ or $SCR^jR^k$, then Ar represents an aromatic hydrocarbon residue optionally having a substituent or an aromatic heterocyclic residue other than an oxyazole ring and an thiazole ring which may be substituted and may be fused with other ring,
when $Y^1$ and $Y^2$ are N at the same time and n is 1, then A represents O, $CR^jR^kO$, $OCR^jR^k$, and $R^1$ represents a group other than alkyl$SO_m$,
when $Y^1$ and $Y^2$ are N at the same time, n is 0, and A is a single bond, then $X^1$ to $X^5$ are not N,
or a salt thereof.

**2.** The compound according to claim 1, wherein 0 to 2 of $X^1$ to $X^5$ are N,
0 to 3 of $Z^1$ to $Z^4$ are N,
Ar is a phenyl group optionally having a substituent or
an aromatic 5- or 6-membered heterocyclic group optionally having a substituent,
$R^1$ is a hydrogen, a halogen, an amino optionally substituted with 1 or 2 substituents, an optionally substituted alkyl$SO_m$, a cyano, an optionally substituted and optionally branched alkyl, an optionally substituted cycloalkyl, an optionally substituted alkenyl, an
optionally substituted alkynyl, an optionally substituted alkoxy, an optionally substituted alkoxycarbonyl or a formyl,
$R^a$, $R^b$, $R^c$, $R^d$ and $R^e$ are the same or different, and are a hydrogen, a halogen, an optionally substituted and optionally branched alkyl, an optionally substituted cycloalkyl, an optionally substituted alkoxy or an optionally substituted alkyl$SO_m$, wherein $R^a$ and $R^e$ are not a hydrogen at the same time,
$R^f$ and $R^h$ are a hydrogen, a halogen, an optionally substituted and optionally branched alkyl or an optionally substituted cycloalkyl,
$R^g$ and $R^i$ are a hydrogen, a halogen, an optionally substituted and optionally branched alkyl, an optionally substituted cycloalkyl or an optionally substituted alkoxy.

**3.** The compound according to claim 1, wherein 0 to 1 of $X^1$ to $X^5$ is N, 0 to 2 of $Z^1$ to $Z^4$ are N, Ar is a phenyl group optionally having a substituent or an aromatic 5-or 6-membered heterocyclic group optionally having a substituent, $R^1$ is a hydrogen, a halogen, an amino, a monoalkylamino, a dialkylamino, an alkyl$SO_m$, a cyano; an alkyl, a branched alkyl or a cycloalkyl, each of which may be substituted with a halogen, a hydroxy, a cyano, an alkyl$SO_m$, an alkoxy or mono- or di-alkylamino; an alkenyl, an alkynyl, an alkoxy, an alkoxycarbonyl or a formyl, $R^a$, $R^b$, $R^c$, $R^d$ and $R^e$ are the same or different and are a hydrogen, a halogen; an alkyl, branched alkyl or a cycloalkyl, each of which may be substituted with halogen; an alkoxy, a haloalkoxy, an alkylthio or a haloalkylthio, respectively, $R^a$ and $R^e$ are not a hydrogen at the same time, $R^f$ and $R^h$ are a hydrogen, a halogen, an alkyl, a branched alkyl or a cycloalkyl, each of which may be substituted with halogen, and $R^g$ and $R^i$ are a hydrogen, a halogen; an alkyl, a branched alkyl, a cycloalkyl or an alkoxy, each of which may be substituted with halogen.

**4.** The compound according to claim 1, wherein A is a single bond, O, $CH_2O$, $OCH_2$, $CH(CH_3)O$, CH(CN)O, OCH $(CH_3)$, $CH_2S$, NH or $CH_2NH$, Ar is an optionally halogenated phenyl or pyridyl, pyridazinyl, pyrimidinyl or thiadiazolyl, each of which may be substituted with a halogen or an alkylthio, B is a halogen, an alkyl, a haloalkyl, a haloalkenyl, an alkoxy, a haloalkoxy, a cyano, a nitro or a halophenyl, $R^1$ is H, a halogen, an alkyl, a branched alkyl, a cycloalkyl, a haloalkyl, an alkenyl, an alkynyl, a hydroxyalkyl, a cyanoalkyl, an alkoxy, an alkyl$SO_m$, an amino, a dialkylamino, an alkoxyalkyl, an alkyl$SO_m$alkyl, a dialkylaminoalkyl, a formyl, an alkoxycarbonyl or a cyano, $R^a$ and $R^e$ are the same or different, and H, a halogen, an alkyl, a haloalkyl, an alkoxy, a haloalkoxy or an alkylthio, respectively (provided that they are not a hydrogen at the same time), $R^b$, $R^d$ and $R^c$ are H or a halogen, $R^f$ and $R^h$ are the same or different and H or a halogen, respectively, $R^g$ and $R^i$ are the same or different and are a hydrogen, a halogen or an alkoxy, respectively, 0 to 1 of $X^1$ to $X^5$ is N, and 0 to 2 of $Z^1$ to $Z^4$ are N.

**5.** The compound according to claim 1, which is 3-(2-chloro-6-fluorophenyl)-1-[4-(3,5-dichloropyridin-2-yloxymethyl) phenyl]-5-methyl-1*H*-1,2,4-triazole, 3-(2-chloro-6-fluorophenyl)-1-[4-(3-chloro-5-trifluoromethylpyridin-2-yloxymethyl)phenyl]-5-methyl-1*H*-1,2,4-triazole or 3-(3-chloropyridin-2-yl)-1-[4-(3-chloro-5-trifluoromethylpyridin-2-yloxymethyl)phenyl]-5-methyl-1*H*-1,2,4-triazole.

**6.** A process for producing the compound according to claim 1, which comprises reacting a compound represented by the formula:

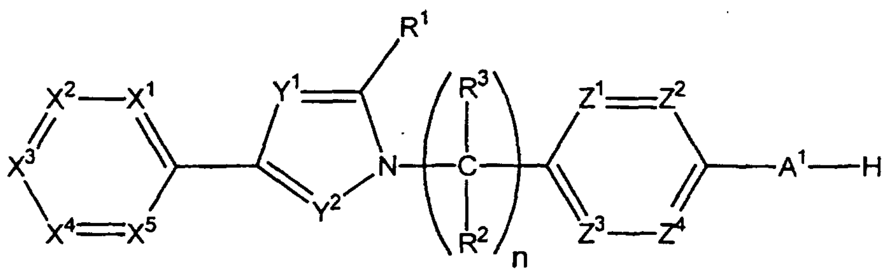

(II)

wherein $A^1$ represents O, $CR^jR^kO$, $SO_m$, $CR^jR^kSO_m$, $NR^l$, or $CR^jR^kNR^l$, and other symbols are as defined in claim the 1, with a compound represented by the formula:

$$L^1\text{-Ar-B} \qquad \text{(III)}$$

wherein $L^1$ represents a leaving group, and other symbols are as defined in claim 1.

**7.** A process for producing the compound according to claim 1, which comprises reacting a compound represented by the formula:

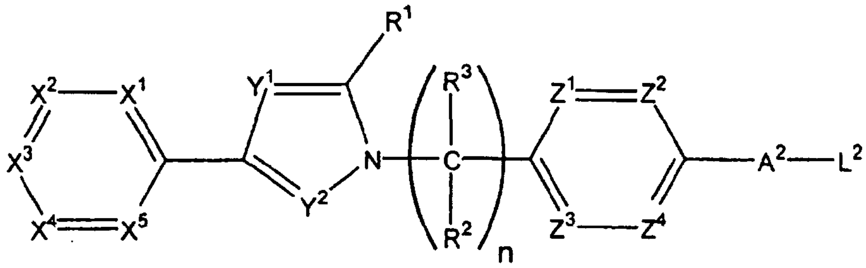

(IV)

wherein $A^2$ represents a single bond or $CR^jR^k$, $L^2$ represents a leaving group, and other symbols are as defined in claim 1, with a compound represented by the formula:

$$H\text{-}A^3\text{-}Ar\text{-}B \qquad (V)$$

wherein $A^3$ represents O, $SO_m$ or $NR^l$, and other symbols are as defined in claim 1.

**8.** A process for producing the compound according to claim 1, which comprises reacting a compound represented by the formula:

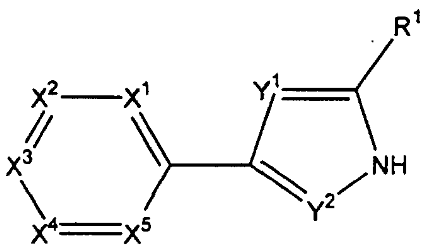

(VI)

wherein respective symbols are as defined in claim 1, with a compound represented by the formula:

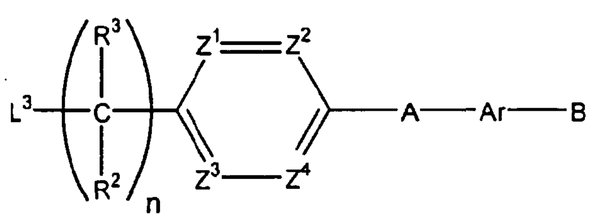

(VII)

wherein $L^3$ represents a leaving group, and other symbols are as defined in claim 1.

**9.** A process for producing the compound according to claim 1 wherein both $Y^1$ and $Y^2$ are N, which comprises:

(1) reacting a compound represented by the formula:

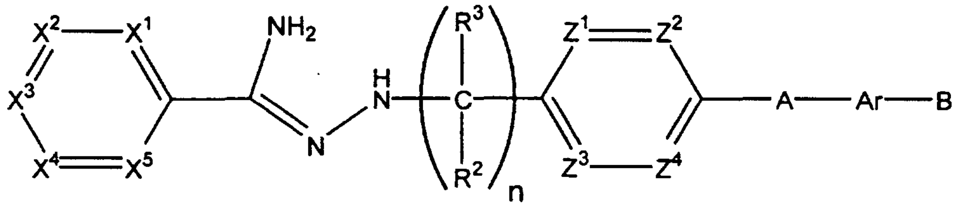

(VIII)

wherein respective symbols are as defined in claim 1, with a compound represented by the formula :

$$R^1\text{-C(=O)-O-C(=O)-}R^1 \qquad \text{(IX)}$$

or the formula:

$$R^1\text{-C(=O)-}L^4 \qquad \text{(X)}$$

or

$$R^1C(L^{4'})_3 \qquad \text{(XI)}$$

wherein $L^4$ and $L^{4'}$ represent a leaving group, and $R^1$ is as defined in claim 1, or

(2) reacting the compound represented by the above-mentioned formula (VIII) with a compound represented by the formula :

$$L^5\text{-C(=O)-}L^6 \qquad \text{(XII)}$$

wherein $L^5$ and $L^6$ represent a leaving group,
to obtain a compound represented by the formula:

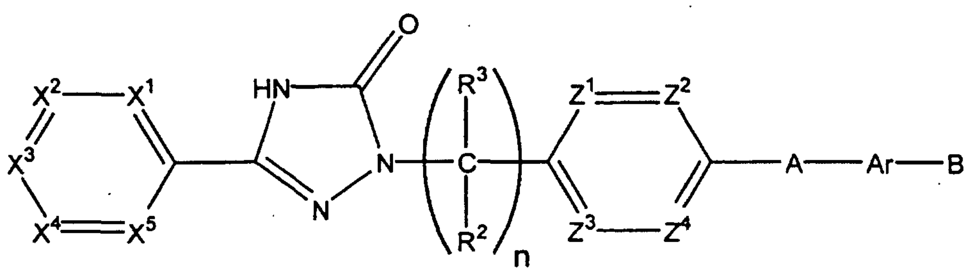

(XIII)

wherein respective symbols are as defined in claim 1, and reacting the resulting compound with a halogenating agent.

**10.** A process for producing the compound according to claim 1 wherein $Y^1$ is CH and $Y^2$ is N, which comprises:

(1) reacting a compound represented by the formula :

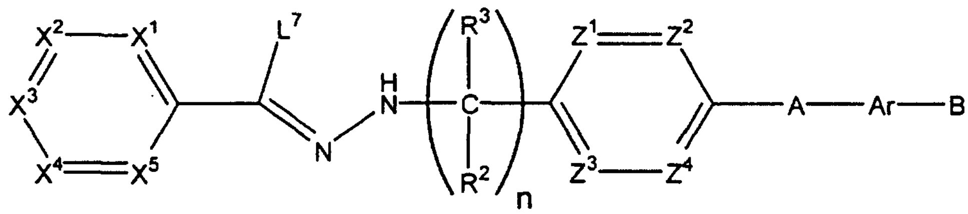

(XIV)

wherein $L^7$ represents a leaving group, with a compound represented by the formula:

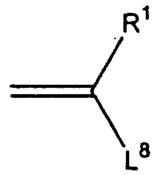

(XV)

wherein $L^8$ represents a leaving group, or
(2) reacting a compound represented by the aforementioned formula (XIV) with a compound represented by the formula:

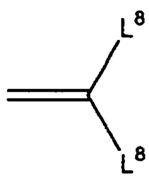

(XVI)

wherein $L^8$ is as defined above,
to obtain a compound represented by the formula:

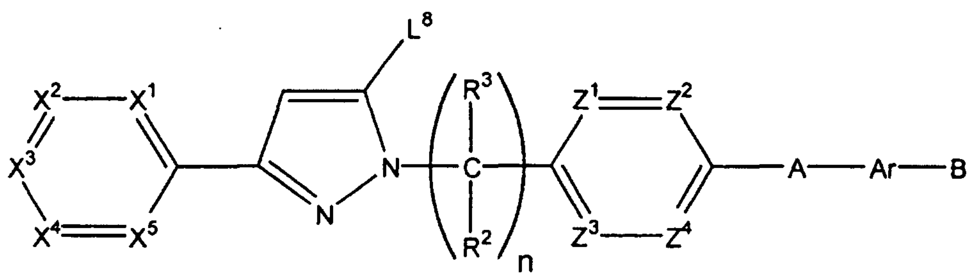

(XVII)

wherein $L^8$ is as defined above, and other symbols are as defined in claim 1,
and reacting the resulting compound with a compound represented by the formula:

$R^1$-H (XVIII)

wherein $R^1$ is as defined in claim 1.

**11.** A process for producing the compound according to claim 1 wherein $Y^1$ is N and $Y^2$ is N or CH, which comprises reacting a compound represented by the formula:

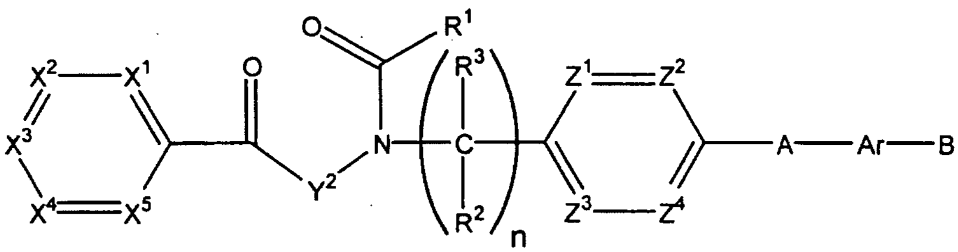

(XIX)

wherein respective symbols are as defined in claim 1, with a compound represented by the formula:

$Acy\text{-}OHNH_3$ (XX)

wherein Acy represents an acyl group.

**12.** A process for producing the compound according to claim 1 wherein $Y^1$ is N and $Y^2$ is CH or N, which comprises reacting a compound represented by the formula:

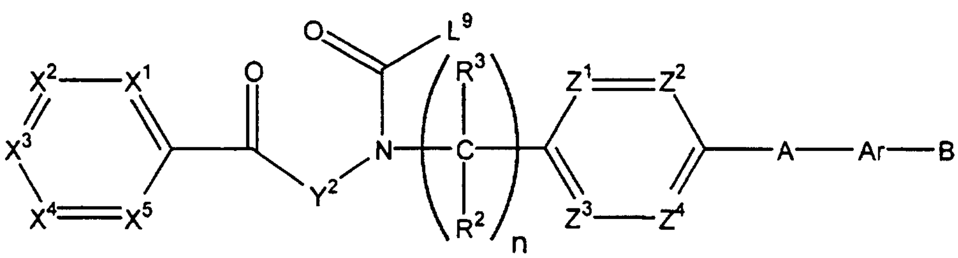

(XXI)

wherein $L^9$ represents a leaving group, with a compound represented by the formula:

$Acy\text{-}OHNH_3$ (XXII)

wherein Acy represents an acyl group,
to obtain a compound represented by the formula:

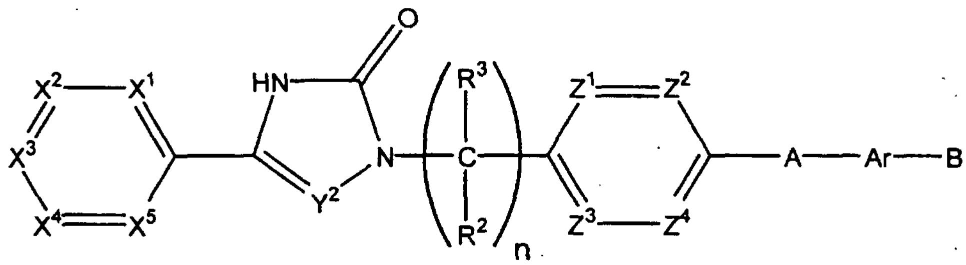

(XXIII)

wherein respective symbols are as defined in claim 1, and reacting the resulting compound with a halogenating agent.

**13.** A process for producing the compound according to claim 1, which comprises reacting a compound represented by the formula:

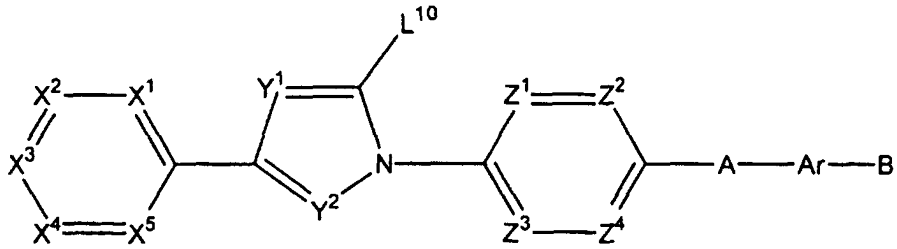

(XXIV)

wherein $L^{10}$ represents a leaving group, and other symbols are as defined in claim 1, with a compound represented by the formula:

$$R^1\text{-H} \qquad \text{(XVIII)}$$

wherein $R^1$ is as defined in claim 1.

**14.** A pest controller which comprises as an active ingredient a compound represented by the formula:

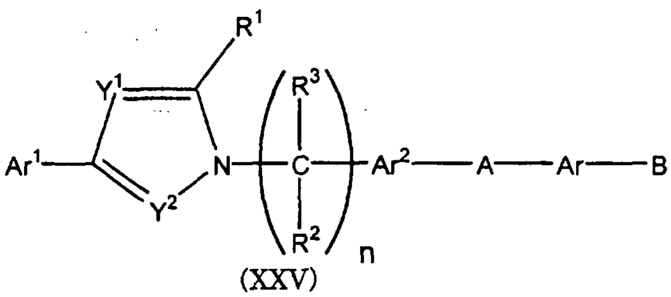

(XXV)

wherein $Ar^1$ represents a 6-membered aromatic hydrocarbon group or a 6-membered nitrogen-containing aromatic heterocyclic group, each of which has a substituent at an ortho position and may be further substituted;
$Ar^2$ represents an optionally substituted 6-membered aromatic hydrocarbon group or an optionally substituted 6-membered nitrogen-containing aromatic heterocyclic group;

$Y^1$ and $Y^2$ are the same or different, and represent N or CH, respectively, provided that they are not CH at the same time;
$R^1$ represents H, a halogen, an optionally substituted and optionally branched alkyl, an optionally substituted cycloalkyl, an optionally substituted alkenyl, an optionally substituted alkynyl, an optionally substituted alkoxy, an optionally substituted alkyl$SO_m$, an amino optionally substituted with 1 or 2 substituents, a cyano, a nitro, a formyl, a hydroxy, an optionally substituted acyl or an optionally substituted alkoxycarbonyl;
$R^2$ and $R^3$ are the same or different, and represent H or an alkyl, respectively, or may form a ring together with a carbon atom which they bind to;
A represents a single bond, O, $CR^jR^kO$, $OCR^jR^k$, $SO_m$, $CR^jR^kSO_m$, $SO_mCR^jR^k$, $NR^l$, $CR^jR^kNR^l$ or $NR^lCR^jR^k$;
Ar represents an aromatic residue optionally having a substituent;
B represents a halogen, an optionally branched alkyl, a cycloalkyl, a haloalkyl, an alkenyl, a haloalkenyl, an alkoxy, a haloalkoxy, an alkyl$SO_m$, a haloalkyl$SO_m$, an amino optionally substituted with 1 or 2 substituents, an alkyl$SO_m$alkyl, a cyano, a nitro or an optionally substituted phenyl;
$R^j$ and $R^k$ are the same or different, and represent H, a cyano or an alkyl, respectively, or may form a ring together with a carbon atom which they bind to;
$R^l$ represents H or an alkyl;
m represents 0, 1 or 2; n represents 0 or 1;
provided that, when $Y^1$ and $Y^2$ are N at the same time, and n is 0, and $Ar^1$ is pyridyl, and $R^1$ is hydrogen, a lower alkyl, a lower alkenyl, a lower alkynyl, a haloalkyl, an alkoxyalkyl or an optionally substituted phenyl group, then Ar represents an aromatic heterocyclic residue optionally having a substituent,
when $Y^1$ and $Y^2$ are N at the same time, and n is 0 and $Ar^1$ is a 6-membered aromatic hydrocarbon group, and A is O, S or $NR^l$, then $R^1$ represents a group other than a halogen, when $Y^1$ and $Y^2$ are N at the same time, n is 0, and $Ar^1$ is a 6-membered aromatic hydrocarbon group, and A is $OCR^jR^k$ or $SCR^jR^k$, then Ar represents an aromatic hydrocarbon residue optionally having a substituent or an aromatic heterocyclic residue other than an oxazole ring and a thiazole ring which may be substituted and may be fused with other ring,
when $Y^1$ and $Y^2$ are N at the same time, and n is 1, then A represents O, $CR^jR^kO$, or $OCR^jR^k$ and $R^1$ represents a group other than alkyl$SO_m$, when $Y^1$ and $Y^2$ are N at the same time, n is 0, and A is a single bond, then $Ar^1$ is a 6-membered aromatic hydrocarbon group, or a salt thereof.

**15.** The pest controller according to claim 14, wherein the compound of the formula (XXV) is a compound represented by the formula:

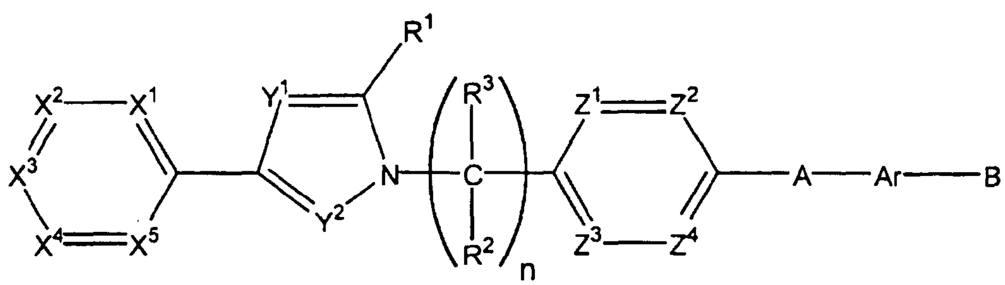

(I)

wherein $X^1$ represents N or C-$R^a$, $X^2$ represents N or C-$R^b$,
$X^3$ represents N or C-$R^c$, $X^4$ represents N or C-$R^d$ and $X^5$ represents N or C-$R^e$;
$Y^1$ and $Y^2$ are the same or different, and represent N or CH, respectively, provided that they are not CH at the same time;
$Z^1$ represents N or C-$R^f$, $Z^2$ represents N or C-$R^g$, $Z^3$ represents N or C-$R^h$ and $Z^4$ represents N or C-$R^i$;
A represents a single bond, O, $CR^jR^kO$, $OCR^jR^k$, $SO_m$, $CR^jR^kSO_m$, $SO_mCR^jR^k$, $NR^l$, $R^jR^kNR^l$ or $NR^lR^jR^k$;
Ar represents an aromatic residue optionally having a substituent;
B represents a halogen, an optionally branched alkyl, a cycloalkyl, a haloalkyl, an alkenyl, a haloalkenyl, an alkoxy, a haloaokoxy, an alkyl$SO_m$, a haloalkyl$SO_m$, an amino optionally substituted with 1 or 2 substituents, an alkyl$SO_m$alkyl, a cyano, a nitro or an optionally substituted phenyl;
$R^1$ represents H, a halogen, an optionally substituted and optionally branched alkyl, an optionally substituted cycloalkyl, an optionally substituted alkenyl, an optionally substituted alkynyl, an optionally substituted alkoxy, an

optionally substituted alkyl$SO_m$, an amino optionally substituted with 1 or 2 substituents, a cyano, a nitro, a formyl, a hydroxy, an optionally substituted acyl or an optionally substituted alkoxycarbonyl;
$R^2$ and $R^3$ are the same or different and represent H or an alkyl, respectively, or may form a ring together with a carbon atom which they bind to;
$R^a$ and $R^e$ are the same or different and represent H, a halogen, an optionally substituted and optionally branched alkyl, an optionally substituted cycloalkyl, an optionally substituted alkoxy or an optionally substituted alkyl$SO_m$, or an amino optionally substituted with 1 or 2 substituents, respectively, provided that they are not H at the same time;
$R^b$ and $R^d$ are the same or different and represent H, a halogen, an optionally substituted and optionally branched alkyl, an optionally substituted cycloalkyl, an optionally substituted alkenyl, an optionally substituted alkynyl, an optionally substituted alkoxy, an optionally substituted alkyl$SO_m$, an amino optionally substituted with 1 or 2 substituents, an optionally substituted acyl, an optionally substituted alkoxycarbonyl, a cyano, a nitro, a formyl or a hydroxy, respectively;
$R^c$ represents H, a halogen, an optionally substituted and optionally branched alkyl, an optionally substituted cycloalkyl, an optionally substituted alkenyl, an optionally substituted alkynyl, an optionally substituted alkoxy, an optionally substituted alkyl$SO_m$, an amino optionally substituted with 1 or 2 substituents, an optionally substituted acyl, an optionally substituted alkoxycarbonyl, a nitro, a formyl or a hydroxy;
$R^f$ and $R^h$ are the same or different and represent H, a halogen, an optionally substituted and optionally branched alkyl, an optionally substituted cycloalkyl, an optionally substituted alkenyl, an optionally substituted alkynyl, an optionally substituted acyl, an optionally substituted alkoxycarbonyl, a cyano, a nitro, a formyl or a hydroxy, respectively;
$R^g$ and $R^i$ are the same or different and represent H, a halogen, an optionally substituted and optionally branched alkyl, an optionally substituted cycloalkyl, an optionally substituted alkenyl, an optionally substituted alkynyl, an optionally substituted alkoxy, an optionally substituted alkyl$SO_m$, an amino optionally substituted with 1 or 2 substituents, an optionally substituted acyl, an optionally substituted alkoxycarbonyl, a cyano, a nitro, a formyl or a hydroxy, respectively;
$R^j$ and $R^k$ are the same or different and represent H, a cyano or an alkyl, respectively, or may form a ring together with a carbon atom which they bind to;
$R^l$ represents H or an alkyl;
m represents 0, 1 or 2; n represents 0 or 1;
provided that, when $Y^1$ and $Y^2$ are N at the same time, and n is 0, and one of $X^1$ to $X^5$ is N, and $R^1$ is hydrogen, a lower alkyl, a lower alkenyl, a lower alkynyl, a haloalkyl, an alkoxyalkyl or an optionally substituted phenyl group, then Ar represents an aromatic heterocyclic residue optionally having a substituent,
when $Y^1$ and $Y^2$ are N at the same time, and n is 0, and $X^1$ to $X^5$ are not N, and A is O, S or $NR^l$, then $R^1$ represents a group other than halogen,
when $Y^1$ and $Y^2$ are N at the same time, and n is 0, and $X^1$ to $X^5$ are not N, and A is $OCR^jR^k$ or $SCR^jR^k$, then Ar represents an aromatic hydrocarbon group optionally having a substituent or an aromatic heterocyclic residue other than an oxazole ring and a thiazole ring which may be substituted and may be fused with other ring,
when $Y^1$ and $Y^2$ are N at the same time, and n is 1, then A represents O, $CR^jR^kO$ or $OCR^jR^k$ and $R^1$ represents a group other than an alkyl$SO_m$,
when $Y^1$ and $Y^2$ are N at the same time, n is 0, and A is a single bond, then $X^1$ to $X^5$ are not N, or a salt thereof.

**16.** The pest controller according to claim 15, wherein A is a single bond, O, $CH_2O$, $OCH_2$, $CH(CH_3)O$, CH(CN)O, $OCH(CH_3)$, $CH_2S$, NH or $CH_2NH$, Ar is an optionally halogenated phenyl, or pyridyl, pyridazinyl, pyrimidinyl or thiadiazolyl, each of which may be substituted with a halogen or an alkylthio, B is a halogen, an alkyl, a haloalkyl, a haloalkenyl, an alkoxy, a haloalkoxy, a cyano, a nitro or a halophenyl, $R^1$ is H, a halogen, an alkyl, a branched alkyl, a cycloalkyl, a haloalkyl, an alkenyl, an alkynyl, a hydroxyalkyl, a cyanoalkyl, an alkoxy, an alkyl$SO_m$, an amino, a dialkylamino, an alkoxyalkyl, an alkyl$SO_m$alkyl, a dialkylaminoalkyl, a formyl, an alkoxycarbonyl or a cyano, $R^a$ and $R^e$ are the same or different and are H, a halogen, an alkyl, a haloalkyl, an alkoxy, a haloalkoxy or an alkylthio, respectively (provided that, they are not hydrogen at the same time), $R^b$, $R^d$ and $R^c$ are H or a halogen, $R^f$ and $R^h$ are the same or different and are a hydrogen or a halogen, respectively, $R^g$ and $R^i$ are the same or different and are a hydrogen, a halogen or an alkoxy, respectively, 0 to 1 of $X^1$ to $X^5$ is N, and 0 to 2 of $Z^1$ to $Z^4$ are N.

**17.** The pest controller according to claim 14, which comprises 3-(2-chloro-6-fluorophenyl)-1-[4-(3,5-dichloropyridin-2-yloxymethyl)phenyl]-5-methyl-1H-1,2,4-triazole, 3-(2-chloro-6-fluorophenyl)-1-[4-(3-chloro-5-trifluoromethylpyridin-2-yloxymethyl)phenyl]-5-methyl-1*H*-1,2,4-triazole or 3-(3-chloropyridin-2-yl)-1-[4-(3-chloro-5-trifluoromethylpyridin-2-yloxymethyl)phenyl]-5-methyl-1*H*-1,2,4-triazole.

**18.** The pest controller according to any one of claim 14 to 17, which is an insecticide or an acaricide.

## INTERNATIONAL SEARCH REPORT

International application No.
PCT/JP02/04452

A. CLASSIFICATION OF SUBJECT MATTER
Int.Cl[7] C07D231/12, 233/64, 249/08, 401/06, 401/12, 401/14, 403/12, 417/12, A01N43/653, 43/824

According to International Patent Classification (IPC) or to both national classification and IPC

B. FIELDS SEARCHED
Minimum documentation searched (classification system followed by classification symbols)
Int.Cl[7] C07D231/00-417/12, A01N1/00-65/02

Documentation searched other than minimum documentation to the extent that such documents are included in the fields searched

Electronic data base consulted during the international search (name of data base and, where practicable, search terms used)
CAPLUS(STN), REGISTRY(STN), WPIL(QUESTEL)

C. DOCUMENTS CONSIDERED TO BE RELEVANT

| Category* | Citation of document, with indication, where appropriate, of the relevant passages | Relevant to claim No. |
|---|---|---|
| X | WO 98/12194 A1 (Lead Chemical Co., Ltd.), 26 March, 1998 (26.03.98), Page 13, compounds 7, 8; page 26, compound 7h & JP 10-87658 A & AU 9743183 A & CN 1074412 A & US 6232335 A & KR 2000036238 A | 1-5,8 |
| X | WO 94/03449 A1 (Institute Luso Faramaco Distalia S.P.A.), 17 February, 1994 (17.02.94), Claims; example 1; page 21, lines 3 to 4 & EP 654030 B1 & ES 2095662 T3 & US 5587390 A | 1-5,8 |
| X | DE 3631511 A (Shering AG), 24 March, 1998 (24.03.98), Claims; page 7, example 6 (Family: none) | 1-5 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 August, 2002 (09.08.02) | 27 August, 2002 (27.08.02) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/04452

C (Continuation). DOCUMENTS CONSIDERED TO BE RELEVANT

| Category* | Citation of document, with indication, where appropriate, of the relevant passages | Relevant to claim No. |
|---|---|---|
| A | JP 8-245315 A (Nippon Soda Co., Ltd.), 24 September, 1996 (24.09.96), Claims (Family: none) | 14-18 |

Form PCT/ISA/210 (continuation of second sheet) (July 1998)